(19) Europäisches Patentamt
European Patent Office
Office européen des brevets

(11) Publication number: 0 232 017
A1

(12) **EUROPEAN PATENT APPLICATION**

(21) Application number: 87300226.5

(22) Date of filing: 12.01.87

(51) Int. Cl.⁴: **C 07 D 205/08**
C 07 D 205/10,
C 07 D 205/12,
C 07 D 403/06, C 07 D 405/06
// A61K31/395

(30) Priority: 23.01.86 US 821676

(43) Date of publication of application:
12.08.87 Bulletin 87/33

(84) Designated Contracting States: AT ES GR

(71) Applicant: THE UPJOHN COMPANY
301 Henrietta Street
Kalamazoo, Michigan 49001 (US)

(72) Inventor: Brickner, Steven J.
c/o The Upjohn Company 301 Henrietta Street
Kalamazoo Michigan 49001 (US)

(74) Representative: Perry, Robert Edward et al
GILL JENNINGS & EVERY 53-64 Chancery Lane
London WC2A 1HN (GB)

(54) **Antimicrobial N-acyl-2-azetidinones.**

(57) The present invention provides novel compositions of matter and processes for their preparation. Particularly, the present invention relates to novel monocyclic β-lactams of formula I, having a carbonyl group, other than a negatively charged group, attached to the N-1 position. The compounds are useful as antimicrobial agents or as intermediates thereto. The present invention also provides a novel process for making these compounds which utilizes palladium (O)-catalyzed carbonyiation of an appropriate 2-bromo-1-propen-3-yl amine and novel intermediates prepared by this process.

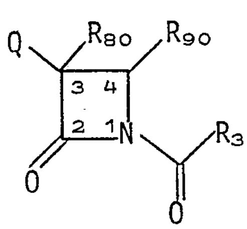

I

EP 0 232 017 A1

**Description**

ANTIMICROBIAL N-ACYL-2-AZETIDINONES

BACKGROUND OF THE INVENTION

The present invention provides novel compositions of matter and processes for their preparation. Particularly, the present invention relates to novel monocyclic β-lactams having a carbonyl group, other than a negatively charged group, attached to the N-1 position. These compounds lack a negatively charged group on the N-1 substitutent. These compounds are useful as antimicrobial agents or as intermediates thereto. The present invention also provides novel intermediates prepared by palladium (O)-catalyzed carbonylation of an appropriate 2-bromo-1-propen-3-yl amine.

The interest in monocyclic β-lactams as therapeutically useful antibacterial agents has arisen only within the past decade. This interest has increased with the discovery of the nocardicins, M. Hashimoto, T. Komari and T. Kamiya, J. Amer. Chem. Soc., 98:3023 (1976), and more recently, the monobactams, A. Imada et al., Nature, 289:590 (1981); R.B. Sykes et al., Nature 291:489 (1981): C.M. Cimarusti and R.B. Sykes, Chem. Britain, 1983:302. The early synthetic work of A.K. Bose et al., J. Med. Chem., 17:541 (1974), predated these findings in providing the first examples of synthetic monocyclic β-lactams that exhibited some antibacterial activity.

The past several years have also witnessed the advent of a number of structurally divers, "non-classical" bicyclic β-lactams, both synthetic in origin and naturally-occurring. These bear no α-amido function on the β-lactam ring, yet some exhibit high antibacterial inhibitory activity. The members include the thienamycins, G. Albers-Schonberg et al., J. Amer. Chem. Soc., 100:6491 (1978), and related carbapenems, the asparenomycins, European published applications 17246 and 30719; J. Shoji et al., J. Antibiotics, 35:15 (1982); and S. Tanabe et al., J. Antibiotics 35: 1237 (1982); northienamycins, U.S. Patent 4,247,640 (1981), and carpetimycin A, M. Nakayama et al., J. Antibiotics, 33:1388 (1980); and synthetic penicillin derivatives, J.C. Sheehan and Y.S. Lo, J. Org. Chem., 38:3227 (1973); Y.S. Lo and J.C. Sheehan, J. Amer. Chem. Soc., 94:8253 (1972).

The asparenomycins and SF-2103B (Suppl. Index of Antibiot, from Actinomycetes (1985) 85-26) are the only naturally-occurring α-alkylidene β-lactams yet reported.

One of the recently reported asparenomycin antibiotics is dihydroasparenomycin 6643-X. S. Tanabe et al., J. Antibiotics, 35:1237 (1982). Members of this class exhibit good in vitro activity against a number of Gram-positive and Gram-negative bacteria, including β-lactamase producers. S. Tanabe et al., J. Antibiotics, 35:1237 (1982); Y. Kimura et al., J, Antibiotics, 35:32 (1982). Recently, a synthetic 6-acetylmethylene penicillanic acid (RO 15-1903) has been reported to be a powerful inhibitor of various β-lactamases. M. Arisawa and R.L. Then, J. Antibiotics, 35:1578 (1982).

The literature reveals several synthetic processes for the construction of α-methylene β-lactams but which hold little or no promise for stereochemical control of olefinic geometry at the C-3 position. These include: sulphoxide, T. Minami, M. Ishida and T. Agawa, J. Chem.Soc.Chem. Comm., 12 (1978), and selenoxide eliminations, T. Agawa, M. Ishida and Ohshiro, Synthesis, 1980:933; P.J. Giddings, D.I. John and E.J. Thomas, Tetrahedron Lett., 21:395 (1980); aldol condensation of ketones with the lithium enolates of 3-trimethylsilylazetidin-2-ones, S. Kano et al., Synthesis, 1978:746; phase-transfer-catalyzed cyclization of 3-bromo-2-bromomethylpropionamides, S.R. Fletcher and I.T. Kay, J. Chem. Soc. Chem.-Comm., 1978:903; [2+2]-cycloadditions of allenes with chlorosulfonyl isocyanate, E.J. Moriconi and J.F. Kelley, J. Org.Chem., 33:3036 (1968); E.J. Moriconi and J.F. Kelley, J. Amer. Chem.Soc., 88:3657 (1966); J.D. Buynak et al., Tetrahedron Lett., 26:5501 (1985); J.D. Buynak, J. Org. Chem., 50:425 (1985); and the α-keto amide benzenesulphonylhydrazone work of R.M Adlington, A.G.M. Barrett, P. Quayle and A. Walker, Journal Chem. Soc. Chem. Comm., 1981:404.

6-Methoxymethylenepenicillanic acid is reported by D.G. Brenner, J. Org. Chem., 50:18 (1985).

INFORMATION DISCLOSURE

M. Mori et al., Tetrahedron, 41:375-385 (1985), describes a new synthesis of β-lactams. This method was applied to the synthesis of N-substituted-α-methylene-β-lactams from the corresponding 2-bromo-2-propenylamine, a secondary amine, and uses a catalytic amount of $Pd(OAc)_2$ or $Pd(acac)_2$ and $PPh_3$ under 1-4 atmospheres pressure of carbon monoxide. In K. Chiba, M. Mori and Y. Ban, Tetrahedron, 41:387-392 (1985), the method for the synthesis of α-methylene-β-lac tams was extended to the synthesis of (±)-3-aminonocardicinic acid. See also M. Okita et al., Heterocycles, 23:247 (1985); M. Mori et al., Heterocycles, 23:317 (1985).

M. Mori, K. Chiba, M. Okita and Y. Ban, J. Chem. Soc. Chem. Comm., 1979:698, describes the synthesis of α-alkylidene 2-azetidinones using palladium-catalyzed carbonylation of an allylic aminevinyl bromide, and has applied it to a synthesis of 3-aminonocardicinic acid, K. Chiba, M. Mori and Y. Ban, J. Chem. Soc. Chem. Comm. 1980:770. This process has demonstrated the stereospecificity of the carbonylation reaction in the α-benzylidene cases. All of the examples reported therein utilize allylic secondary amines, giving β-lactams with the nitrogen substituted by an alkyl or benzyl group.

2-Azetidinones substituted on the ring nitrogen atom by ((substituted-sulfonyl)amino)carbonyl are reported in Derwent Abstract 90720E of European published application (EP) 62876, which is based on U.S. application 252,672 filed 4-9-81. Other azetidinone derivatives are diclosed in Derwent Abstracts 85-100569 (EP 138,407, U.S. application serial numbers 599,841 (filed 4-13-84) and 538,719) and 85-100135 (West German published

application 3,435,998, U.S. application serial number 538,720 (filed 10-3-83)).

Derwent Abstract 85-018443 of Merck U.S. Patent 4,491,580 discloses 2-aza-bicyclo(2.1.0)pentan-3-one carboxylic acids, salts and esters which are useful as antibiotics for pharmaceutical and disinfectant purposes.

SUMMARY OF THE INVENTION

The present invention particularly provides:

A compound of the formula I wherein Q is 1) hydrogen, or
2) $-C(R_0)(R_1)(R_{70})$;
wherein $R_{70}$ and $R_{80}$ are each hydrogen or wherein $R_{70}$ and $R_{80}$ taken together are a double bond;
wherein $R_{90}$ is 1) hydrogen,
2) (C1-C12)alkyl,
3) (C2-C12)alkenyl,
4) (C2-C12)alkynyl,
5) $-CH_2$-(C2-C12)alkenyl, or
6) phenyl;
or wherein $R_{80}$ and $R_{90}$ taken together, with the carbon atoms to which they are attached, are 1) (C4-C8)cycloalkyl,
2) (C4-C8)cycloalkenyl, or
3) cyclobutyl or cyclobutenyl substituted by (C1-C8) alkyl;
4) cyclopentyl or cyclopentenyl substituted by
(i) -CHO,
(ii) $-CH_2Br$,
(iii) $-CH_2OC(O)R_5$, or
(iv) $-CH_2OC(O)R_3$;
provided that when $R_{80}$ and $R_{90}$ are taken together, Q is hydrogen;
provided that $R_{80}$, and $R_{90}$ and Q cannot all be hydrogen;
wherein $R_0$ and $R_1$ are the same or different and are: 1) hydrogen,
2) (C1-C12) alkyl substituted by zero, one or two $R_2$,
3) (C1-C12) alkyl substituted on the carbon atom of attachment by $R_{15}NH$-, $R_5CO-N(R_{19})$- or $R_{15}N(CO-R_5)$-,
4) (C2-C12) alkenyl,
5) (C2-C12) alkynyl,
6) (C3-C12) cycloalkyl substituted by zero, one or two $R_2$,
7) (C3-C12) cycloalkyl substituted on the carbon atom of attachment by $R_{15}NH$-, $R_5CO-N(R_{19})$- or $(R_{15}N(CO-R_5)$-,
8) (C3-C12) cycloalkyl-(C1-C6) alkyl substituted by zero, one or two $R_2$,
9) (C3-C12) cycloalkyl-(C1-C6) alkyl substituted on the carbon atom of attachment by $R_{15}NH$-, $R_5CO-N(R_{19})$- or $R_{15}N(CO-R_5)$-,
10) $R_{11}$,
11) $R_{11}$-(C1-C6) alkyl,
12) $R_{11}$-(C2-C6) alkenyl,
13) $R_{11}$-(C2-C6) alkynyl,
14) $R_{13}$,
15) $R_{13}$-(C1-C6) alkyl,
16) $R_{13}$-(C2-C6) alkenyl, or
17) $R_{13}$-(C2-C6) alkynyl;
or wherein $R_0$ and $R_1$ are taken together with the carbon atom to which they are bonded to form: 1) (C3-C12) cycloalkyl substituted by zero, one or two $R_{32}$, or
2) (C4-C12) cycloalkenyl;
provided that $R_0$ and/or $R_1$ contains an alkynyl group only when $R_{70}$ and $R_{80}$ taken together are a double bond; and
provided that the carbon atom of attachment of alkenyl or alkynyl to oxygen of alkenyl-0- or alkynyl-0- groups is not also part of a carbon-carbon double or triple bond;
wherein $R_2$ is: 1) $R_{16}O$-,
2) $R_{10}O$-,
3) $R_{10}(CH_2)_mS(CH_2)_n$-,
4) $R_5CO-O$-,
5) $N_3$,
6) 4,5-dihydro-4-($R_6$)-5-($R_7$)-1H-1,2,3-triazol-1-yl,
7) 4-($R_6$)-5-($R_7$)-1H-1,2,3-triazol-1-yl,
8) $R_8CO-NH$-,
9) $R_5CO-NH$-,
10) fluorine,
11) chlorine, or
12) bromine:

provided that $R_2$ is $N_3$ or 4,5-dihydro-4-($R_6$)-5-($R_7$)-1H-1,2,3-triazol-1-yl only when $R_{70}$ and $R_{80}$ taken together are a double bond; and provided that $R_2$ is hydroxy (i.e., $R_2$ is $R_{10}O$ wherein $R_{10}$ is hydrogen) only when $R_{70}$ and $R_{80}$ taken together are a double bond; and $R_2$ is a substituent on $R_1$; and
provided that $R_{32}$ is hydroxy (i.e., $R_{32}$ is $R_{10}O$ wherein $R_{10}$ is hydrogen) only when $R_{70}$ and $R_{80}$ taken together are a double bond;
wherein m is an integer zero, one, two, three or four;
wherein n is an integer zero, one, two, three or four;
wherein $R_3$ is: 1) hydrogen,
2) (C1-C12) alkyl substituted by zero, one or two $R_{22}$,
3) (C2-C12) alkenyl,
4) (C2-C12) alkynyl,
5) (C3-C12) cycloalkyl substituted by zero, one or two $R_{22}$,
6) (C3-C12) cycloalkyl-(C1-C6) alkyl substituted by zero, one or two $R_{22}$,
7) $R_{11}$,
8) $R_{11}$-(C1-C6) alkyl,
9) $R_{11}$-(C2-C6) alkenyl,
10) $R_{11}$-(C2-C6) alkynyl,
11) $R_{13}$,
12) $R_{13}$-(C1-C6) alkyl,
13) $R_{13}$-(C2-C6) alkenyl,
14) $R_{13}$-(C2-C6) alkynyl,
15) $R_{14}O$-,
16) $R_4NH$-,
17) $R_4N(CH_3)$-,
18) an α-amino acid moiety ($N^{\alpha}$-$R_{34}$)-$R_{35}$- wherein $R_{34}$ is (C1-C12) alkanoyl and $R_{35}$ is defined so that $R_{35}$-CO- is an α-amino acid acyl group selecting from the group consisting of glycyl, alanyl, valyl, leucyl, isoleucyl, phenylalanyl, O-$R_{34}$-seryl, O-$R_{34}$-threonyl, $N^{\varepsilon}$-$R_{34}$-lysyl, asparagyl, glutamyl, S-$R_{34}$-cysteinyl, methionyl, O-$R_{34}$-tyrosyl, $N^{in}$-$R_{34}$-tryptophyl, and $N^{im}$-$R_{34}$-histidyl; or
19) an α-amino acid moiety $R_{36}$- wherein $R_{36}$ is defined so that $R_{36}$-CO- is prolyl or 4-$R_{34}$ O-prolyl and $R_{34}$ is as defined above;
wherein $R_4$ is: 1) (C1-C4) alkyl,
2) phenyl, or
3) (C1-C4) alkyl-$NHSO_2$-;
wherein $R_5$ is: 1) hydrogen,
2) (C1-C12) alkyl substituted by zero, one or two $R_{22}$,
3) (C2-C12) alkenyl,
4) (C2-C12) alkynyl,
5) (C3-C12) cycloalkyl substituted by zero, one or two $R_{22}$,
6) (C3-C12) cycloalkyl-(C1-C6) alkyl substituted by zero, one or two $R_{22}$,
7) $R_{11}$,
8) $R_{11}$-(C1-C6) alkyl,
9) $R_{11}$-(C2-C6) alkenyl,
10) $R_{11}$-(C2-C6) alkynyl,
11) $R_{13}$,
12) $R_{13}$-(C1-C6) alkyl,
13) $R_{13}$-(C2-C6) alkenyl,
14) $R_{13}$-(C2-C6) alkynyl,
15) $R_{14}O$-,
16) $R_4NH$-,
17) $R_4N(CH_3)$-,
18) an α-amino acid moiety ($N^{\alpha}$-$R_{34}$)-$R_{35}$- wherein $R_{34}$ is (C1-C12) alkanoyl and $R_{35}$ is defined so that $R_{35}$-CO- is an α-amino acid acyl group selected from the group consisting of glycyl, alanyl, valyl, leucyl, isoleucyl, phenylalanyl, O-$R_{34}$-seryl, O-$R_{34}$-threonyl, $N^{\varepsilon}$-$R_{34}$-lysyl, asparagyl, glutamyl, S-$R_{34}$-cysteinyl, methionyl, O-$R_{34}$-tyrosyl, $N^{in}$-$R_{34}$-tryptophyl, and $N^{im}$-$R_{34}$-histidyl; or
19) an α-amino acid moiety $R_{36}$- wherein $R_{36}$ is defined so that $R_{36}$-CO- is prolyl or 4-$R_{34}$ O-prolyl and $R_{34}$ is as defined above;
wherein $R_6$ and $R_7$ are the same or different and are: 1) hydrogen,
2) (C1-C4) alkyl,
3) (C1-C4) alkyloxy-C(=O)-,
4) $(R_{29})_2NC(=O)$-, or
5) $(R_{24})(R_{25})(R_{26})Si$-;
wherein $R_8$ is: 1) hydrogen,
2) (C1-C12) alkyl substituted by zero, one or two $R_{20}$,
3) (C3-C12) cycloalkyl substituted by zero, one or two $R_{20}$,
4) (C3-C12) cycloalkyl-(C1-C6) alkyl substituted by zero, one or two $R_{20}$,

5) $R_{11}$,
6) $R_{11}$-(C1-C6) alkyl,
7) $R_{13}$,
8) $R_{13}$-(C1-C6) alkyl, or
9) $R_{17}O$-;

wherein $R_9$ is: 1) (C1-C12) alkyl,
2) (C3-C12) cycloalkyl,
3) (C3-C12) cycloalkyl-(C1-C6) alkyl,
4) $R_{11}$, or
5) $R_{11}$-(C1-C6) alkyl;

wherein $R_{10}$ is: 1) hydrogen,
2) (C1-C12) alkyl,
3) (C2-C12) alkenyl,
4) (C3-C12) cycloalkyl,
5) (C3-C12) cycloalkyl-(C1-C6) alkyl,
6) $R_{11}$,
7) $R_{11}$-(C1-C6) alkyl,
8) $R_{11}$-(C2-C6) alkenyl,
9) $R_{13}$,
10) $R_{13}$-(C1-C6) alkyl, or
11) $R_{13}$-(C2-C6) alkenyl;

wherein $R_{11}$ is phenyl or 1- or 2-naphthyl, substituted by zero, one or two (C1-C4) alkyl, F, Cl, Br or (C1-C4) alkyloxy;

wherein $R_{13}$ is: 1) furanyl, substituted by zero, one or two $R_9$,
2) thienyl, substituted by zero, one or two $R_9$,
3) N-((C1-C4) alkyl)-pyrrolyl, substituted by zero, one or two $R_9$,
4) N-((C1-C4) alkyl)-indolyl, substituted by zero, one or two $R_9$, or
5) benzofuranyl, substituted by zero, one or two $R_9$;

wherein $R_{14}$ is: 1) (C1-C12) alkyl,
2) (C2-C12) alkenyl,
3) (C2-C12) alkynyl,
4) (C3-C12) cycloalkyl,
5) (C3-C12) cycloalkyl-(C1-C6) alkyl,
6) $R_{11}$,
7) $R_{11}$-(C1-C6) alkyl,
8) $R_{11}$-(C2-C6) alkenyl,
9) $R_{11}$-(C2-C6) alkynyl,
10) $R_{13}$,
11) $R_{13}$-(C1-C6) alkyl,
12) $R_{13}$-(C2-C6) alkenyl, or
13) $R_{13}$-(C2-C6) alkynyl;

wherein $R_{15}$ is phenyl, substituted by zero, one, two or three F, Cl, Br, (C1-C3) alkyloxy or by zero, one or two $NO_2$ or $NH_2$;

provided that phenyl is substituted by $NO_2$ only when $R_{70}$ and $R_{80}$ taken together are a double bond, and that phenyl is substituted by $NH_2$ only when $R_{70}$ and $R_{80}$ are each hydrogen;

wherein $R_{16}$ is $(R_{24})(R_{25})(R_{26})Si$, benzyl, tetrahydropyran-2-yl or other oxygen protecting group;

wherein $R_{17}$ is: 1) (C1-C12) alkyl,
2) (C3-C12) cycloalkyl,
3) (C3-C12) cycloalkyl-(C1-C6) alkyl,
4) $R_{11}$,
5) $R_{11}$-(C1-C6) alkyl,
6) $R_{13}$, or
7) $R_{13}$-(C1-C6) alkyl;

wherein $R_{18}$ is: 1) (C1-C12) alkyl,
2) (C2-C12) alkenyl,
3) (C3-C12) cycloalkyl,
4) (C3-C12) cycloalkyl-(C1-C6) alkyl,
5) $R_{11}$,
6) $R_{11}$-(C1-C6) alkyl,
7) $R_{11}$-(C2-C6) alkenyl,
8) $R_{13}$,
9) $R_{13}$-(C1-C6) alkyl, or
10) $R_{13}$-(C2-C6) alkenyl;

wherein $R_{19}$ is hydrogen or (C1-C12) alkyl;

wherein $R_{20}$ is: 1) (C1-C12) alkyl,

2) (C3-C12) cycloalkyl,
3) (C3-C12) cycloalkyl-(C1-C6) alkyl,
4) phenyl,
5) phenyl-(C1-C6) alkyl,
6) naphthyl, or
7) naphthyl-(C1-C6) alkyl;

wherein $R_{22}$ is: 1) $R_{16}O$-,
2) $R_{10}O$-,
3) $R_{10}(CH_2)_mS(CH_2)_n$-,
4) F,
5) Cl,
6) Br,
7) I,
8) $R_{23}NH$-,
9) NC-,
10) $NO_2$,
11) NHCHO,
12) (C1-C4) alkyloxy-C(=O)-,
13) phenyl-$CH_2OC(=O)$-,
14) (C1-C4) alkyl-C(=O)-$OCH_2$-,
15) (C1-C4) alkyloxy-N=,
16) CN-,
17) SCN-,
18) (C1-C4) alkyl-C(=O)-,
19) (C1-C4) alkyl-$SO_2$-,
20) phenyl-$SO_2$-,
21) ((C1-C4) alkyl)-phenyl-$SO_2$-,
22) $(R_{33})_3N(+)$ X(-), or
23) $R_{27}$, $R_{28}$-substituted-1-pyridinium(+) X(-);

wherein X(-) is a pharmacologically acceptable anion;
wherein $R_{27}$ and $R_{28}$ are the same or different and are: 1) hydrogen, or
2) (C1-C4) alkyl;

or wherein $R_{27}$ and $R_{28}$ are bonded to adjacent carbon atoms of the pyridine ring in which they occur and taken together are (C3-C5) methylene to form a (C5-C7)-membered carbocyclic ring fused to the pyridine ring;
wherein $R_{23}$ is: 1) hydrogen,
2) t-butyloxycarbonyl,
3) trityl,
4) benzyloxycarbonyl, or
5) benzyl;

wherein $R_{24}$, $R_{25}$ and $R_{26}$ are the same or different and are: 1) (C1-C4) alkyl, or
2) phenyl;

wherein $R_{29}$ is (C1-C4) alkyl;
wherein $R_{32}$ is: 1) $R_{16}O$-,
2) $R_{10}O$-,
3) $R_{10}(CH_2)_mS(CH_2)_n$-,
4) (C1-C4) alkyl, or
5) phenyl substituted by zero, one or two $R_{16}O$-, $R_{10}O$-, $R_{10}$-$(CH_2)_mS(CH_2)_n$-, (C1-C4) alkyl, F, Cl, or Br;

wherein $R_{33}$ is:
1) (C1-C4) alkyl, or
2) benzyl.

A compound of the formula II wherein $R_{60}$ and $R_{61}$ are the same or different and are: 1) hydrogen,
2) (C1-C12) alkyl substituted by zero, one or two $R_{12}$,
3) (C2-C12) alkenyl,
4) (C2-C12) alkynyl,
5) (C3-C12) cycloalkyl substituted by zero, one or two $R_{12}$,
6) (C3-C12) cycloalkyl-(C1-C6) alkyl substituted by zero, one or two $R_{12}$,
7) $R_{11}$,
8) $R_{11}$-(C1-C6) alkyl,
9) $R_{11}$-(C2-C6) alkenyl,
10) $R_{11}$-(C2-C6) alkynyl,
11) $R_{13}$,
12) $R_{13}$-(C1-C6) alkyl,
13) $R_{13}$-(C2-C6) alkenyl, or
14) $R_{13}$-(C2-C6) alkynyl;

or wherein $R_{60}$ and $R_{61}$ are taken together with the carbon atom to which they are bonded to form: 1) (C3-C12) cycloalkyl substituted by zero, one or two $R_{12}$, or
2) (C4-C12) cycloalkenyl;
provided that $R_{60}$ or $R_{61}$ contains an alkynyl group only when $\underline{---}$ is a double bond; and
provided that the carbon atom of attachment of alkenyl or alkynyl to oxygen of alkenyl-O- or alkynyl-O- groups is not also part of a carbon-carbon double or triple bond;
wherein $R_{11}$ is phenyl or 1- or 2-naphthyl, substituted by zero, one or two (C1-C4) alkyl, F, Cl, Br or (C1-C4) alkyloxy:
wherein $R_{12}$ is: 1) $(R_{24})(R_{25})(R_{26})SiO-$,
2) $R_{18}O-$,
3) $R_{18}(CH_2)_mS(CH_2)_n-$, or
4) $R_{40}(CH_2)_mS(CH_2)_n-$;
wherein m is an integer zero, one, two, three or four;
wherein n is an integer zero, one, two, three or four;
wherein $R_{13}$ is: 1) furanyl, substituted by zero, one or two $R_9$,
2) thienyl, substituted by zero, one or two $R_9$,
3) N-((C1-C4) alkyl)-pyrrolyl, substituted by zero, one or two $R_9$,
4) N-((C1-C4) alkyl)-indolyl, substituted by zero, one or two $R_9$, or
5) benzofuranyl, substituted by zero, one or two $R_9$;
wherein $R_9$ is: 1) (C1-C12) alkyl,
2) (C3-C12) cycloalkyl,
3) (C3-C12) cycloalkyl-(C1-C6) alkyl,
4) $R_{11}$, or
5) $R_{11}$-(C1-C6) alkyl;
wherein $R_{18}$ is: 1) (C1-C12) alkyl,
2) (C2-C12) alkenyl,
3) (C3-C12) cycloalkyl,
4) (C3-C12) cycloalkyl-(C1-C6) alkyl,
5) $R_{11}$,
6) $R_{11}$-(C1-C6) alkyl,
7) $R_{11}$-(C2-C6) alkenyl,
8) $R_{13}$,
9) $R_{13}$-(C1-C6) alkyl, or
10) $R_{13}$-(C2-C6) alkenyl;
wherein $R_{24}$, $R_{25}$ and $R_{26}$ are the same or different and are: 1) (C1-C4) alkyl, or
2) phenyl;
wherein $R_{40}$ is: 1) a sulfur-protecting group, or
2) hydrogen;
provided that $R_{40}$ is a sulfur-protecting group when m is zero and that $R_{40}$ is hydrogen when m is other than zero which comprises treating a compound of the formula III with a catalytic amount of palladium(O) tetrakis (triphenylphosphine) in the presence of triphenylphosphine and a base selected from the group consisting of triethylamine, diisopropylethylamine and tri-n-butylamine at 80 to 135°C.

The carbon atom content of various hydrocarbon-containing moieties is indicated by a prefix designating the minimum and maximum number of carbon atoms in the moiety, i.e., the prefix (Ci-Cj) indicates a moiety of the integer "i" to the integer "j" carbon atoms, inclusive. Thus, for example, (C1-C3) alkyl refers to alkyl of one to three carbon atoms, inclusive, including both straight and branched chain, i.e., methyl, ethyl, propyl and isopropyl.

Examples of alkyl to one to twelve carbon atoms, inclusive, are methyl, ethyl, propyl, butyl, pentyl, hexyl, heptyl, octyl, nonyl, decyl, undecyl, dodecyl and isomeric forms thereof. Alkenyl as used herein refers to compounds having one carbon-carbon double bond. Alkynyl as used herein referse to compounds having one carbon-carbon triple bond.

($N^{\alpha}$-$R_{34}$) refers to substitution by $R_{34}$ on the α carbon. $N^{\varepsilon}$-$R_{34}$-lysyl refers to lysyl substituted on the ε carbon by $R_{34}$; $N^{in}$-$R_{34}$-tryptophyl refers to tryptophyl substituted on the indolyl nitrogen by $R_{34}$; $N^{im}$-$R_{34}$-histidyl refers to histidyl substituted on an imidazole nitrogen by $R_{34}$.

It is readily apparent to one skilled in the art that the compounds of this invention of formula Ia, when $R_{70}$ and $R_{80}$ taken together are a double bond and $R_0$ and $R_1$ are different, exist as E-or Z-isomers. See J.E. Blackwood et al., J. Amer. Chem. Soc., 90:509-510 (1968) for a description of the terms E and Z. Both E and Z isomers are included within the scope of this invention. For example, if $R_0$ is fluoromethyl and $R_1$ is methyl, a formula Ia compound is a Z-isomer. If $R_0$ is methyl and $R_1$ is fluoromethyl, a formula Ia compound is an E-isomer.

The compounds of this invention of formula Ia, when $R_{70}$ and $R_{80}$ are each hydrogen is a single bond and $R_0$, $R_1$ and $R_3$ are as defined above contain an asymmetric azetidine ring carbon (denoted C3). Furthermore, if $R_0$ and $R_1$ are different, the carbon atom bearing $R_0$ and $R_1$ (i.e., the carbon atom alpha to the ring) is likewise asymmetric. Additional asymmetric carbon atoms are possible when $R_0$, $R_1$ and/or $R_3$ contain substituent groups. As is well known in the art, such asymmetric carbon atoms give rise to enantiomers and diastereomers, and all such stereoisomers, either in pure form or mixtures thereof, are included within the

scope of this invention. See, for example, J.B. Henderickson, D.J. Cram, and G.S. Hammond, Organic Chemistry, Third Edition, McGraw-Hill Book Company, New York, N.Y., 1970, pages 198-230, particularly pages 207, 208, 213, 215.

When $R_0$ and/or $R_1$ of the formula Ia compound contains alkenyl unsaturation, such unsaturation must be protected during exposure to conditions for hydrogenation which would undesirably saturate the unsaturation. Subsequent to exposure to such conditions, the protected form is converted to the desired carbon-carbon unsaturation. Such protection and deprotection are known in the art: see, for example, M.F. Semmelhack et al., Tetrahedron Letters. 2667 (1973); T.L Nagabhushan, Can. J. Chem. 48:383 (1970); S. Hanessian et al., Tetrahedron Letters, 737 (1978); N.C. Barua et al., Tetrahedron Letters, 23:1365 (1982).

Protection and deprotection of reactive groups are well known to those skilled in that art. Protection and deprotection of hydroxy groups and also of nitrogen groups are well known in the art: see, for example, T.W. Greene, Protecting Groups in Organic Synthesis, Wiley, New York (1981); J.F.W. McOmie, Ed., Protective Groups in Organic Chemistry, Plenum Press (1973); and J. Fuhrhop and G. Benzlin, Organic Synthesis, Verlag Chemie (1983).

Examples of pharmacologically acceptable anions include: acetate, adipate, aspartate, benzoate, benzenesulfonate, bisulfate, butyrate, citrate, camphorate, camphorsulfonate, digluconate, dodecylsulfate, ethanesulfonate, fumarate, glucoheptanoate, glycerophosphate, hemisulfate, heptanoate, hexanoate, chloride, bromide, iodide, 2-hydroxyethanesulfonate, lactate, maleate, methanesulfonate, 2-naphthalenesulfonate, nicotinate, oxalate, palmoate, pectinate, persulfate, 3-phenylpropionate, picrate, pivalate, propionate, succinate, tartrate, methanesulfonate, tosylate, undecanoate, and the like.

The processes of the present invention are more completely understood by reference to the charts below. The compounds of formula I of this invention are prepared according to Charts A-M and the descriptions thereof. Briefly, Chart A shows the formation of the β-lactam ring. Chart B shows hydrogenation of the α-alkylidene double bond and acylation of the azetidinone nitrogen. Chart C shows the preparation of O,N-bisacyl compounds. Chart D shows the prepara tion of α-alkylidene O,N-bisacyl compounds. Charts E, F and G show the preparation of compound bearing other substitution on $R_0$ or $R_1$. Charts H and I show the preparation of amine- and amide-substituted $R_0$ or $R_1$ groups. Chart J shows the preparation of N-acyl cycloalkyl-and cycloalkenyl-fused azetidinones. Chart K shows the preparation of N-acyl substituted cycloalkyl- and cycloalkenyl-fused azetidinones. Chart L shows the preparation of N-acyl C-4 substituted azetidinones. Chart M shows the preparation of N-acyl, C-4 alkyl, C-3 alkyl- and alkylidene-azetidinones.

In Charts A-M, the variables are as defined above. Additional variables are defined below:

wherein $R_{60}$ and $R_{61}$ are the same or different and are: 1) hydrogen,

2) (C1-C12) alkyl substituted by zero, one or two $R_{12}$,
3) (C2-C12) alkenyl,
4) (C2-C12) alkynyl,
5) (C3-C12) cycloalkyl substituted by zero, one or two $R_{12}$,
6) (C3-C12) cycloalkyl-(C1-C6) alkyl substituted by zero, one or two $R_{12}$,
7) $R_{11}$,
8) $R_{11}$-(C1-C6) alkyl,
9) $R_{11}$-(C2-C6) alkenyl,
10) $R_{11}$-(C2-C6) alkynyl,
11) $R_{13}$,
12) $R_{13}$-(C1-C6) alkyl,
13) $R_{13}$-(C2-C6) alkenyl, or
14) $R_{13}$-(C2-C6) alkynyl;

or wherein $R_{60}$ and $R_{61}$ are taken together with the carbon atom to which they are bonded to form: 1) (C3-C12) cycloalkyl substituted by zero, one or two $R_{12}$, or

2) (C4-C12) cycloalkenyl:

provided that $R_{60}$ or $R_{61}$ contains an alkynyl group only when --- is a double bond; and provided that the carbon atom of attachment of alkenyl or alkynyl to oxygen of alkenyl-O-or alkynyl-O- groups is not also part of a carbon-carbon double or triple bond;

wherein $R_{12}$ is: 1) $(R_{24})(R_{25})(R_{26})SiO-$,

2) $R_{18}O-$,
3) $R_{18}(CH_2)_mS(CH_2)_n-$, or
4) $R_{40}(CH_2)_mS(CH_2)_n-$;

wherein $R_{40}$ is: 1) a sulfur-protecting group, or

2) hydrogen;

provided that $R_{40}$ is a sulfur-protecting group when m is zero and that $R_{40}$ is H when m is other than zero.

Use of a wavy line bond in the charts herein to depict the bonding of a group to oe carbon atom of a double bond of a particular structure, as, e.g., in structure A-3 in Chart A, represents (1) a compound with one or the other possible orientation of the groups bonded to that carbon atom, or (2) a mixture of the compounds with either possible orientation of the groups bonded to that carbon atom.

The carbon atom of attachment of a group is the carbon atom by means of which that group is attached to another atom. For example, for a formula Ia compound, when $R_0$ is (C1-C12) alkyl, the carbon atom of attachment is the carbon atom joining the (C1-C12) alkyl group to the α-carbon of the formula Ia compound.

Throughout this document, $R_{30}$ is defined so that the moiety $R_{30}CH(R_2)$-is as defined for $R_{60}$ when substituted by $R_2$ on the carbon atom of attachment of $R_{60}$. This representation is chosen so that the carbon atom of attachment of $R_{60}$ and the functional group $R_2$ attached to that carbon atom are explicitly shown. Thus, it is possible to show chemical transformations of this functional group $R_2$. Similarly, $R_{31}$ is defined so that the moiety $R_{31}CH(R_2)$- is as defined for $R_{61}$ when substituted by $R_2$ on the carbon atom of attachment of $R_{61}$.

The starting materials for the processes of this invention are either known or are prepared by known methods. The products of these processes are isolated by known methods such as extraction, distillation, crystallization, chromatographic separation and the like.

Charts A and F illustrate the preparation of intermediate 2-azetidin-1-one compounds. Subsequently, Charts B-E and G-M illustrate the preparation of compounds of formula I.

Using the methods disclosed herein, all of the compounds of formula I of this invention are prepared.

CHART A

Chart A illustrates the synthesis of intermediates containing the 2-azetidinone ring. Referring to Chart A, treatment of an aldehyde or ketone of fomula A-1, with a brominated phosphonate carbanion, e.g., $(CH_3O)_2P(=O)C^-(Br)(CO_2CH_3)Na^+$, gives an E and Z mixture of the bromoacrylate of formula A-2. The A-2 isomers are separated, if desired at this stage, by chromotography on silica gel. Bromoacrylate A-2 is treated with diisobutylaluminum hydride in a solvent such as tetrahydrofuran, diethyl ether, hexane or methylene chloride at -78° to 0° C to give the compound of formula A-3. Alternatively and preferably, the E and Z mixture of formula A-2 is treated as indicated above to give the E and Z isomers of the compound of formula A-3 which are separated at this stage.

In an identical manner for either isomer (or less preferably for a mixture of the isomers) treatment of the compound of formula A-3 with mesyl chloride or tosyl chloride and triethylamine or any suitable hindered trialkylamine in a solvent such as methylene chloride, tetrahydrofuran or diethyl ether ar -15° to 0° C provides the sulfonate ester of the formula A-4, wherein $R_{40}$ is methyl or p-methylphenyl. The mesylate, or tosylate of the formula A-4 is displaced by sodium azide treatment in aqueous tetrahydrofuran or dimethylformamide at 0° to 5° C followed by a quick aqueous extractive workup, to avoid a [3,3]-sigmatropic rearrangement, to produce the azide of formula A-5. The axide of formula A-5 is immediately subjected to reduction with lithium aluminum hydride, diisobutylaluminum hydride or hydrogen sulfide in diethyl ether or tetrahydrofuran to give the allylic amine of formula A-6. Treatment of the amine of formula A-6 with a catalytic amount of palladium (O) tetrakis (triphenylphosphine) (1-10 mole % or more) in the presence of triphenylphosphine and a base such as triethylamine, diisopropylethylamine or tri-n-butylamine at 80° to 135° C in dimethylformamide under an atmosphere of carbon monoxide gas gives the α-alkylidene azetidinone of formula A-7.

CHART B

The processes of Chart B illustrate hydrogenation and acylation processes used to prepare compounds of this invention. Referring to CHart B, the formula A-7 compound of Chart A is employed as the formula B-1 starting material. The formula B-1 compound is hydrogen ated to produce the formula B-2 azetidinone which is acylated to produce the N-acylated azetidinone of formula B-3.

An alternative process for some compounds of this invention is to reverse the order to these two steps: the α-alkylidene azetidinone of formula B-1 is first acylated to produce the N-acylated α-alkylidene azetidinone of formula B-4 which is hydrogenated to produce the N-acylated azetidinone of formula B-3, recognizing that alkenyl and alkynyl unsaturation in the compound of formula B-4 is also saturated in the process, and that certain protecting groups (e.g., benzyl and benzyloxycarbonyl) are cleaved or altered under these conditions. Thus, this B-1 to B-4 to B-3 sequence is used only to produce compounds of formula B-3 wherein $R_0$, $R_1$ and $R_3$ do not contain (a) alkenyl or alkynyl carbon-carbon unsaturation which would be undesirably saturated, or (b) a protecting group which would be undesirably cleaved or altered under the hydrogenation conditions.

Protecting groups contained in formula B-3 or B-4 compounds are optionally removed by known methods to produce compounds of formula Ia of this invention.

For the processes of this invention, as described above for Chart B, it is generally possible to hydrogenate the α-alkylidene double bond either before or after acylating the ring nitrogen or other sites, recognizing the effects of such hydrogenation conditions on other functionality as described above.

Typical hydrogenation conditions include treatment with 1 to 3 atmospheres of hydrogen gas in the presence of 10% palladium on carbon or palladium black catalyst in ethyl acetate, methylene chloride, ethanol or tetrahydrofuran solvent at 15 to 30° C. Alternatively, this hydrogenation is accomplished using a chiral catalyst by known methods, i.e., using [rhodium(1,5-cyclooctadiene)-(1,2-ethanediylbis[(o-methoxyphenyl)phenylphosphine]](+)$BF_4$(-) according to the method of B.D. Vineyard et al., J. Amer.Chem. Soc., 99:5946 (1977) to produce a compound of formula B-2 or B-3 which is predominantly or exclusively one enantiomer. See also J.P. Collman et al., Principles and applications of Organotransition Metal Chemistry, University Science Books, Mill Valley, California, pages 341-349 (1980); and W.S. Knowles et al., Chem. Comm., 1445 (1968).

Hydrogenation of the α-alkylidene double bond of compounds such as the compounds of formula B-1 and B-4 proceeds via cis addition of hydrogen to the double bond without prior isomerization of the double bond in the starting material. Thus, for example, a formula B-1 compound wherein ---- is a double bond and wherein

$R_{60}$ is $CH_3CO$-O-$CH_2$-and $R_{61}$ is methyl is termed a Z-isomer. Achiral hydrogenation of such a Z-isomer produces a 1:1 mixture of the RR and SS diastereomers and none of the Rs or Sr diastereomers, where R and S are the usual designation of absolute configuration, and where RR refers to the R-configuration at each of the two asymmetric centers (i.e., at carbon atom position 3 of the ring and at the $R_{60}$-CH($R_{61}$)- carbon atom bonded to the carbon atom position 3 of the ring, respectively) and SS refers to the S-configuration at each of the two asymmetric centers. Chiral hydrogenation of such a Z-isoner produces a mixture of RR and S isomers which is not 1:1, i.e., either the RR or SS isomer predominates.

On the other hand, achiral hydrogenation of an E-isomer (e.g., a compound of formula B-1 wherein $R_{60}$ is methyl and $R_{61}$ is $CH_3CO$-O-$CH_2$-) produces a 1:1 mixture of the Rs and Sr diastereomers and none of the RR or SS diastereomers. Chiral hydrogenation of such an E-isomer produces a mixture of RS and SR isomers which is not 1:1, i.e., either the RS or SR isomer predominates.

The acylation of compounds of formula B-1 or B-2 is achieved as follows: Formula B-3 or B-4 compounds wherein $R_3$ is hydrogen are prepared by reacting a formula B-1 or B-2 compound with ethyl formate according to knwon methods. To prepare formula B-3 or B-4 compounds wherein a carbon atom of $R_3$ is directly bonded to the carbonyl carbon, acylation of the formula B-1 or B-2 compound is performed by one of three methods: 1) acylation with an acid anhydride in pyridine in the presence of 4-(N,N-dimethylamino)pyridine at 0° to 20° C; 2) treatment with an acid chloride in the presence of a hindered trialkylamine base such as triethylamine in methylene chloride, diethyl ether or tetrahydrofuran at -78° to 20° C; or 3) treatment with a carboxylic acid in the presence of dicyclohexylcarbodiimide and either 4-(N,N-dimethylamino)pyridine or 1-hydroxybenzotriazole in diethyl ether, methylene chloride or tetrahydrofuran at 0° to 20° C. Other known acylation methods are also operable.

When $R_3$ is $R_{14}O$- and thus has an oxygen atom directly bonded to the carbonyl group, acylation of the formula B-1 or B-2 compound is performed by treatment with a chloroformate of the formula Cl-CO-$OR_{14}$ in the presence of triethylamine or other trialkylamine in diethyl ether, methylene chloride or tetrahydrofuran at 0° to 20° C. An excess of the chloroformate reagent may be added to effect complete conversion.

When $R_3$ is $R_4NH$ and thus has a nitrogen atom directly bonded to the N-acyl group, acylation of the formula B-1 or B-2 compound is performed by treatment with: (1) an alkyl isocyanate or phenyl isocyanate at -78° to 20° C in acetonitrile, methylene chloride, chloroform, tetrahydrofuran or diethyl ether to give the formula B-3 or B-4 compound wherein $R_3$ is $R_4NH$- and $R_4$ is (C1-C4) alkyl or phenyl, or (2) with chlorosulfonyl isocyanate in methylene chloride, chloroform, tetrahydrofuran, acetonitrile or diethyl ether, followed by reaction with an alkyl amine at room temperature for 1 to 5 hours to give the formula B-3 or B-4 compound wherein $R_3$ is $R_4NH$- and $R_4$ is (C1-C4) alkyl-$NHSO_2$-. Such compounds wherein $R_3$ is $R_4NH$- are converted to compounds wherein $R_3$ is $R_4N(CH_3)$- by reaction with a base such as sodium hydride in a suitable solvent such as tetrahydrofuran at about 0° C, followed by reaction with methyl iodide.

CHART C

Chart C illustrates the synthesis of formula Ia compounds of this invention wherein $R_0$ bears a hydroxy group or a hydroxy group substituted witn an $R_3$-CO- group or wherein both the hydroxy of $R_0$ and the azetidine nitrogen atom bear the same of different $R_3$-CO-group. The silyl ether of formula C-1 is prepared by the method of Chart A, starting with a compound of the formula $R_{61}$CO-CH($R_{30}$)(OSi-($R_{24}$)($R_{25}$)($R_{26}$)), which is prepared via silylation of the corresponding α-hydroxy ketone, which either is commercially available or is prepared by known methods. Referring to Chart C, the silyl ether of formula C-1 is deprotected with either tetra-(n-butyl)ammonium fluoride or triethylammonium fluoride in tetrahydrofuran, diethyl ether or methylene chloride at 0° to 20°C to give the alcohol of formula C-2. The alcohol of formula C-2 is hydrogenated as described above for Chart B to produce the alcohol of formula C-3.

In order to introduce the same acyl group twice, (i.e., $R_5$ is the same as $R_3$), the alcohol of formula C-3 is acylated as described above for Chart B, e.g., the formula C-3 alcohol is reacted with an anhydride $(R_3CO)_2O$ in pyridine in the presence of the acylation atalyst 4-(dimethylamino)pyridine to produce the bisacylated compound of formula C-4.

In order to introduce two acyl groups, which can be the same or different, in a stepwise manner, the alcohol of formula C-3 is first acylated selectively on oxygen, using the anhydride $(R_5CO)_2)$ in pyridine without the acylation catalyst to give the compound of formula C-5. Acylation of the azetidine of formula C-5 with the same or different $(R_3CO)_2O$ anhydride in the presence of 4-(dimethylamino)pyridine gives the bisacylated compound of formula C-4, wherein $R_3$ and $R_5$ are the same or different.

The processes of Chart C are similarly carried out starting with the protected E-isomer alcohol of formula C-6 to produce additional E-isomer compounds of formula Ia of this invention.

The processes of Chart C illustrate the functionality of $R_{60}$ (which is $R_{30}$CH($R_2$)- wherein $R_2$ is hydroxy or $R_3$CO-O-) at the carbon atom of attachment of $R_{60}$. The processes of Chart C are likewise used to prepare compounds of this invention wherein the functionality of $R_{60}$ or $R_{61}$ occurs exclusively or additionally at a carbon atom or atoms other than the carbon atom of attachment of $R_{60}$ or $R_{61}$.

CHART D

Chart D illustrates essentially the reversal of the order of the hydrogenation and acylation steps of Chart C, and this sequence is applicable to the preparation of compounds of formula D-2 and D-3 and also compounds of formula D-4 and D-5 wherein $R_3$ does not contain alkenyl or alkynyl unsaturation or protective groups which

would be cleaved or undesirably altered under the conditions of the hydrogenation. The formula C-2 compound of Chart C is employed as the formula D-1 starting material. Using the methods of Chart C above, the alcohol of formula D-1 is either bisacylated to produce the compound of formula D-2 of sequentially acylated with the same or different acyl groups to produce first the compound of formula D-3 and then the compound of formula D-2 wherein $R_3$ and $R_5$ are the same or different. Also using the methods of Chart C above, the compound of formula D-2 is then hydrogenated ro produce the azetidinone of formula D-5, or the compound of formula D-3 is hydrogenated to produce the compound of formula D-4 which is acylated to produce the compound of formula D-5.

The processes of Chart D are likewise employed using in place of the formula D-1 compound: (1) the E-isomer alcohol obtained by O-deprotecting the compound of formula C-6, or (2) a different O-protected form of this E-isomer alcohol, e.g., the tetrahydropyranyl ether, thereby producing additional E-isomer compounds of formula Ia of this invention, including those wherein $R_1$ is substituted by hydroxy and $R_{70}$ and $R_{80}$ taken together are a double bond and those wherein $R_1$ is substituted by $R_5CO$-O-. In particular, in preparing formula Ia compounds wherein $R_1$ is substituted by hydroxy and $R_{70}$ and $R_{80}$ taken together are a double bond, it may be necessary to use a protecting group other than $(R_{24})(R_{25})(R_{26})Si$, e.g., tetrahydropyranyl, since following $R_3CO$- acylation of the azetidine ring nitrogen it was not possible in one instance to remove the $(R_{24})(R_{25})(R_{26})Si$ protecting group and leave the molecule otherwise intact. I.e., several attempts to O-deprotect (E)-3-[1-methyl-2-(tert-butyldiphenylsiloxy)ethylidene]-1-(1-oxobutyl)-2-azetidinone did not produce the desired O-deprotected product. However O-deprotection of 3-(E)-[1-methyl--2-(2-tetrahydropyranyloxy)ethylidene]-1-(1-oxohexyl)-2-azetidinone produces the desired O-deprotected 3-(E)-[1-methyl-2-(hydroxy)-ethylidene]-1-(1-oxohexyl)-2-azetidinone.

The processes of Chart D illustrate the functionality of $R_{60}$ (which is $R_{30}CH(R_2)$- wherein $R_2$ is hydroxy or $R_3CO$-O-) at the carbon atom of attachment of $R_{60}$. The processes of Chart D are likewise used to prepare compounds of this invention wherein the functionality of $R_{60}$ or $R_{61}$ occurs exclusively or additionally at a carbon atom or atoms other than the carbon atom of attachment of $R_{60}$ or $R_{61}$.

CHART E

Chart E illustrates the introduction of certain N-containing groups of formula Ia compounds. Referring to Chart E, the formula C-2 alcohol of Chart C is employed as the formula E-1 startingmaterial. Treatment of the formula E-1 alcohol with a sulfonyl chloride of formula $R_{40}SO_2Cl$ wherein $R_{40}$ is methyl or p-methylphenyl, in the presence of a trialkylamine such as triethylamine in diethyl ether, methylene chloride, or tetrahydrofuran gives the allylic sulfonate ester of formula E-2, e.g., the mesylate or tosylate ester. Treatment of the formula E-2 sulfonate ester with sodium azide gives the formula E-3 compound. N-acylation of the azetidinone ring nitrogen atom, as described in Chart B above, gives the allylic azide of formula E-4. Reductive acylation of the formula E-4 azide using 10% palladium on carbon, 1 to 3 atmospheres of hydrogen gas and an acid anhydride $(R_8CO)_2O$ as solvent, gives the amido-substituted analog of formula E-5. In the absence of the anhydride solvent, a major by-product is formed arising from intramolecular nucleophilic β-lactam ring opening by the free primary amine.

Additionally, the allylic azide of formula E-4 is reacted (a) with a suitable dipolaraphile of formula $R_6C \equiv CR_7$ to produce the adduct of formula E-6 wherein the bond θ is a double bond, or (b) with a suitably activated olefin of formula $R_6CH = CHR_7$ to produce the adduct of formula E-6 wherein the bond θ is a single bond. Catalytic hydrogenation of the α-alkylidene azetidinone of formula E-6 wherein θ is a double bond, as described above for Chart B, produces the azetidinone of formula E-7.

To prepare a compound of formula E-6 (wherein one or both of $R_6$) and $R_7$ are hydrogen), an ethylene or acetylene precursor is used which contains an $(R_{24})(R_{25})(R_{26})Si$ group at that $R_6$ and/or $R_7$ position. After the cycloaddition reaction, the silyl group is removed by methods described herein or known in the art, leaving hydrogen in its place.

The processes of Chart E are likewise employed using in place of the formula E-1 compound the E-isomer alcohol obtained by O-deprotection of the compound of formula C-6 of Chart C to produce additional E-isomer compounds of this invention.

The processes of Chart E illustrate the functionality of $R_{60}$ (which is $R_{30}CH(R_2)$- wherein $R_2$ is azide and other groups illustrated in Chart E) at the carbon atom of attachment of $R_{60}$. The processes of Chart E are likewise used to prepare compounds of this invention wherein the functionality of $R_{60}$ or $R_{61}$ occurs exclusively or additionally at a carbon atom or atoms other than the carbon atom of attachment of $R_{60}$ or $R_{61}$.

CHART F

The processes of Chart F are used to prepare intermediate primary amine compounds of formula F-3. The azide of formula E-3 is used as the formula F-1 starting material. The azide of formula F-1 is reduced to the amine of formula F-3 by a Staudinger Reaction (see Merck Index, Tenth Edition, page ONR-85) using triphenylphosphine in an aprotic solvent such as methylene chloride to produce the intermediate compound of formula F-2 which is hydrolyzed with water to produce the amine of formula F-3.

The processes of Chart F are similarly carried out starting with the E-isomer of formula F-4 which is prepared according to the method of Chart E to produce additional E-isomer compounds of this invention.

The processes of Chart F illustrate the functionality of $R_{60}$ (which is $R_{30}CH(R_2)$- wherein $R_2$ is azido, and other groups illustrated in Chart F) at the carbon atom of attachment of $R_{60}$. The processes of Chart F are

likewise used to prepare compounds of this invention wherein the functionality of $R_{60}$ or $R_{61}$ occurs exclusively or additionally at a carbon atom or atoms other than the carbon atom of attachment of $R_{60}$ or $R_{61}$.

CHART G

The processes of Chart G are used according to methods known in the art to introduce $R_{70}$ which is fluorine, chorine or bromine. The alcohol of formula C-2 is used as the formula G-1 starting material. Reaction of the alcohol of formula G-1 with diethylaminosulfurtrifluoride in methylene chloride produces the compound of formula G-2 wherein $R_{70}$ is fluorine. Reaction of the alcohol of formula G-1 with phosphorous pentachloride produces the compound of formula G-2 wherein $R_{70}$ is chlorine. Reaction of the alcohol of formula G-1 with phosphorous tribromide produces the compound of formula G-2 wherein $R_{70}$ is bromine. According to methods described above for Chart B, the azetidinone of formula G-2 is hydrogenated to produce the compound of formula G-3 which is acylated to produce the compound of formula G-4. Alternatively, the compound of formula G-2 is first acylated to produce the compound of formula G-5 which is hydrogenated to produce the compound of formula G-4.

The processes of Chart G are similarly carried out using in place of the formula G-1 starting material the E-isomer alcohol obtained by deprotection of the compound of formula C-6 of Chart C to produce additional E-isomer compounds of formula Ia of this invention.

The processes of Chart G illustrate the functionality of $R_{60}$ (which is $R_{30}CH(R_2)$- wherein $R_2$ is fluoro, chloro or bromo) at the carbon atom of attachment of $R_{60}$. The processes of Chart G are likewise used to prepare compounds of this invention wherein the functionality of $R_{60}$ or $R_{61}$ occurs exclusively or additionally at a carbon atom or atoms other than the carbon atom of attachment of $R_{60}$ or $R_{61}$.

CHART H

Chart H illustrates the preparation of compounds of formula Ia wherein $R_0$ is substituted on the carbon atom of attachment by $R_5CO-N(R_{52})$- wherein $R_{52}$ is hydrogen, (C1-C12) alkyl or $R_{15}$. The sulfonate ester of formula E-2 of Chart E is used as the formula H-1 starting material. Sulfonate ester of formula H-1 is reacted with an amine of formula $R_{55}NH_2$ wherein $R_{55}$ is (C1-C12) alkyl or $R_{15}$ by methods known in the art to produce the amine of formula H-2.

The amine of formula H-3 comprises the amine of formula H-2 and additionally the amine of formula F-3 of Chart F. The amine of formula H-3 is hydrogenated as described above for Chart B to produce the amine of formula H-4. Monoacylation of the amine of formula H-4 with an anhydride of formula $(R_5CO)_2O$ in pyridine without an acylation catalyst produces the compound of formula H-5, which is acylated with the same or different anhydride of formula $(R_3CO)_2)O$ in pyridine to produce the bisacylated compound of formula H-6 with the same or different $R_3$ groups.

Alternatively, the amine of formula H-3 is monoacylated as above to produce the compound of formula H-7 which is acylated as above to produce the bisacylated compound of formula H-8 wherein $R_3$ and $R_5$ are the same or different. The compound of formula H-8 is hydrogenated as described above for Chart B to produce the compound of formula H-6, recognizing that, as described above, certain other functionality of the formula H-8 compound may also be saturated or altered under the hydrogenation conditions.

The processes of Chart H are similarly carried out starting with the E-isomer compounds of formula H-9 and H-10, analogous to the formula H-1 and H-3 compounds, respectively, to produce additional E-isomer compounds of this invention.

CHART I

Chart I illustrates the preparation of compounds of formula Ia wherein $R_0$ is substituted on the carbon atom of attachment by $R_{15}NH$-. The formula I-1 starting material is included within the scope of the formula H-2 amines prepared in Chart H. The amine of formula I-1 is protected on the amine group by methods known in the art to produce the compound of formula I-2 wherein $R_{53}$ is a nitrogen protecting group, e.g., t-butyloxycarbonyl (t-BOC), triphenylmethyl (trityl), benzyl, benzyloxycarbonyl, and the like. When $R_{53}$ is a nitrogen protecting group (e.g., t-BOC or trityl) which is not removed by hydrogenation conditions as described above for Chart B, such hydrogenation of the compound of formula I-2 produces the compound of formula I-3. Acylation of the compound of formula I-3 with the anhydride of formula $(R_3CO)_2O$ in the presence of 4-(dimethylamino)pyridine produces the compound of formula I-4. Deprotection of the formula I-4 compound by methods known in the art produces the compound of formula I-5.

Alternatively, when $R_{53}$ is a nitrogen protecting group which is removed by hydrogenation conditions (e.g., benzyl or benzyloxycarbonyl) or is not removed by hydrogenation conditions (e.g., t-BOC or trityl) as described above for Chart B, the compound of formula I-2 is acylated as above to produce the compound of formula I-6. Hydrogenation of the compound of formula I-6 as above, followed by N-deprotection, if the protecting group is not removed by the hydrogenation conditions, produces the compound of formula I-5, recognizing that, as described above, certain functional groups in other parts of the formula I-6 compound will also be saturated or altered under the hydrogenation conditions.

The processes of Chart I are similarly carried out starting with the E-isomer amine of formula I-7, which is included within the scope of the formula H-2 amines prepared in Chart H, to produce additional E-isomer compounds of this invention.

Chart J

Chart J illustrates the preparation of N-acyl (C5-C8) cycloalkyl- and (C6, C8) cycloalkenyl- fused azetidinones of formula Ib, wherein $R_{80}$ and $R_{90}$ taken together, with the carbon atoms to which they are attached, from (C5-C8) cycloalkyl- or (C6, C8) cycloalkenyl.

In Scheme I, the compound of formula J-1, wherein n is 3 to 6, is treated sequentially with chlorosulfonyl isocyanate and then with aqueous sodium bisulfate to yield the compound of formula J-2. H. Bestian et al., Liebigs Ann. Chem., 718:94-100 (1968). The compound of formula J-2 is acylated, as described in Chart B, to yield the compound of formula J-3.

In Scheme II, the N-acyl (C6, C8) cycloalkenyl- fused azetidinones are prepared by the same known procedures used Scheme I. The 1,4-cyclohexadiene and 1,5-cyclooctadiene of formula J-4, wherein k is 1 or 2, are readily available. L.A. Paquette et al., J. Am. Chem. Soc., 93:152 (1971).

Chart K

Chart K illustrates the preparation of N-acyl substituted (C4 and C5) cycloalkenyl- and (C4 and C5) cycloalkyl- fused azetidinones of formula Ib, wherein $R_{80}$ and $R_{90}$ taken together, with the carbon atoms to which they are attached, form substituted (C4 or C5) cycloalkenyl- or substituted (C4 or C5) cycloalkyl.

In Scheme I, the compound of formula K-1 is converted to the compound of formula K-2, wherein X is -CHO, $-CH_2Br$ or $-CH_2OH$, by procedures known in the literature. N. Tamura et al., Tet. Letters, 27:3749 (1986). The compound of formula K-2, wherein X is -CHO or$-CH_2Br$, is acylated, as described in Chart B, to yield the compound of formula K-3, wherein X is -CHO or $-CH_2Br$. Using the methods of Chart D, the compound of formula K-2, wherein X is $-CH_2OH$, is either bisacylated to produce the compound of formula K-3, wherein X is $CH_2OC(O)R_3$, or sequentially acylated with the same or different acyl groups to produce the compound of formula K-3, wherein X is $-CH_2OC$-$(O)R_5$ and $R_3$ and $R_5$ are the same or different. Using the methods of Chart B, the compound of formula K-3 is hydrogenated to yield the compound of formula K-4.

In Scheme II of Chart K, the compound of formula K-7, wherein $R_{100}$ is (C1-C8) alkyl, is converted to the compound of formula K-8 by procedures known in the literature. J. Brennan et al., Tet. Letters, 877 (1984). The compound of formula K-8 is acylated, as described in Chart B, to yield the compound of formula K-9. The compound of formula K-9 is hydrogenated, as described in Chart B, to yield the compound of formula K-10.

Alternatively, as described in Chart B, the compound of formula K-8 is hydrogenated first, to yield the compound of formula K-11, and then acylated to yield the compound of formula K-10.

Chart L

Chart L illustrates the preparation of N-acyl C-4 substituted azetidinones of formula Ic, wherein $R_{90}$ is (C1-C12) alkyl, (C2-C12) alkenyl, (C2-C12) alkynyl, $-CH_2$-(C2-C12) alkenyl or phenyl.

In Chart L, $R_{101}$ is defined as (C1-C12) alkyl, (C2-C12) alkenyl, (C2-C12) alkynyl, $-CH_2$-(C2-C12) alkenyl or phenyl and $R_{102}$ is defined as (C1-C8) alkyl. The compound of formula L-2 is reacted sequentially with chlorosulfonyl isocyanate and then with aqueous sodium bisulfite to yield the compound of formula L-3. H. Bestian et al., Liebigs Ann. Chem., 718:94-100 (1968). Alternatively, the compound of formula L-1 is reacted with a lithium alkyl cuprate of the formula $(R_{101})_2CuLi$ to yield the compound of formula L-3. D.H. Hva and A. Verna, Tet. Letters, 547 (1985). The compound of formula L-3 is acylated, as described in Chart B, to yield the compound of formula L-4.

Chart M

Chart M illustrates the preparation of N-acyl, C-4 alkyl, C-3 alkylidene and C-3 alkyl azetidinones of formula Id. wherein $R_0$ and $R_1$ are each hydrogen or (C1-C8) alkyl, and $R_{90}$ is (C1-C8) alkyl.

In Chart M, $R_{105}$ and $R_{106}$ are each defined as hydrogen or (C1-C8) alkyl and $R_{107}$ is defined as (C1-C8) alkyl.

In Scheme I, the compound of formula M-1 is reacted sequentially with chlorosulfonyl isocyanate and then with aqueous sodium bisulfite to yield the compound of formula M-2. H. Bestian et al., Liebigs Ann. Chem. 718:94-100 (1968). The compound of formula M-2 is acylated, as described in Chart B, to yield the compound of formula M-3.

In Scheme II, using the methods of Chart B, the compound of formula M-4, J.D. Buynak and M.N Roa, J. Org. Chem., 51:1571 (1986), is converted to the compound of formula M-6. The compound of formula M-4 is hydrogenated to yield the compound of formula M-5. The compound of formula M-5 is acylated to yield the compound of formula M-6. Alternatively, the compound of formula M-4 is first acylated, to yield the compound of formula M-7, which is hydrogenated to yield the compound of formula M-6, where the substituents of $R_3$ are not susceptible to reductive decomposition.

Except as noted herein, the compounds of formula I are useful as antimicrobial agents. The exceptions described below are generally more useful as synthetic intermediates than as antimicrobial agents.

Compounds of formula Ia wherein $R_2$ is $R_{16}O$- (i.e., a protected oxygen group) are generally of relatively low activity or are not active as antibacterial agents, but are useful as intermediates, being protected forms of the corresponding hydroxysubstituted compounds which are antibacterially active.

The compounds of formula I that are active as antimicrobial agents have exhibited a gram positive and anaerobe spectrum of antimicrobial activity. The antimicrobial activity of the compounds was determined by measuring their zones of inhibition in the dipped-disc Biospectrum assay, a standard test well known in the art.

All compounds were tested at the 1.0 mg/ml concentration level, on 12.7 mm or 6.35 mm Schleicher and Schuell analytical paper discs. Those compounds producing very large zones of inhibition were also tested at 0.1 mg/ml, those zone sizes being given in parentheses.

Among the anaerobic microorganisms against which these compounds were tested are the following: Bacteroides fragilis, Clostridium perfringens and Bacteroides theatiotaomicron. Among the gram-positive aerobes against which these compounds were tested are the following: multiply-antibiotic resistant Staphylococcus aureus UC 6685 strain, UC3665 strain, and UC 6675 strain, S. epidermidis, S. pyogenes, S. pneumoniae and S. lutea. Among the yeasts against which these compounds were tested are the following: C. albicans and penicillium oxalicum.

The more preferred compounds of this invention include the following N-acyl 3-isopropyl-2-azetidinones: 2-azetidinone, 1-acetyl-3-(1-methylethyl)-, (±)-; 2-azetidinone, 3-(1-methylethyl)-1-(1-oxobutyl)-; 2-azetidinone, 3-(1-methylethyl)-1-(1-oxopentyl)-, (±)-; 2-azetidinone, 3-(-1methylethyl)-1-(1-oxohexyl)-; and 2-azetidinone, 3-(1-methlethyl)-1-(oxooctyl)-, (±)-; and N-hexanoyl-cis-3,4-tetramethylene-2-azetidinone. The most preferred compound of this invention is 2-azetidinone, 3-(1-methylethyl)-1-(1-oxohexyl)-. These novel compounds exhibit high activity vs. anaerobes and Gram positive aerobes. The results of antimicrobial testing of these compounds in the dipped-disc Biospectrum assay at 1 mg/ml are given in Tables XIII, XIV and XV below.

The formula I compounds of the present invention are formulated for administration to humans and animals in unit dosage forms, such as tablets, capsules, pills, powders, lyophilized powders, granules, sterile parenteral solutions or suspension, oral solutions or suspensions, rectal suppositories, and oil-in-water or water-in-oil emulsions. Dosage ranges are about 1 to about 100mg/kg for a 70 kg human administered one to four times per day. The compounds of this invention are administered orally or parenterally (e.g., intramuscularly, intravenously and the like). Parenteral administration is preferred.

DESCRIPTION OF THE PREFERRED EMBODIMENTS

Without further elaboration, it is believed that one skilled in the art can, using preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

The following abbreviations are defined for use in this document: deuteriochloroform ($CDCl_3$), methanol-$d_4$ ($CD_3OD$), fast atom bombardment (FAB), diisobutylaluminum hydride (DIBAL-H).

Preparation 1 Methyl-2-bromo-3-methyl-2-butenoate (Formula A-2: $R_{61}$ is $CH_3$; $R_{60}$is $Ch_3$) Refer to Chart A.

To a mechanically stirred slurry of 14.96 g of 50% sodium hydride in oil in 1-5 L of freshly distilled tetrahydrofuran under nitrogen at room temperature is added 49 ml of trimethyl phosphonoacetate slowly to control hydrogen evolution. Ether (500 g) is added to facilitate stirring. The resulting thick white suspension is stirred for 20 minutes, then bromine is added dropwise until a light yellow coloration in the reaction mixture persists (approximately 20 ml). The resultant slurry is cooled to 0° C and 14.96 g of 50% sodium hydride in oil is cautiously added in 10 portions. After addition is complete, the mixture is allowed to warm to room temperature, then 44 ml of acetone is added over 30 minutes. The mixture is stirred at room temperature for 16.5 hr, then a majority of the solvent is distilled off under reduced pressure. The remaining solution (approximately 700 ml) is poured into 1500 ml of water and extracted three times with methylene chloride (1000 ml, 500 ml then 250 ml), and the combined organic layers washed twice with 500 ml of water, and 450 ml of saturated aqueous sodium chloride, dried with magnesium sulfate, and concentrated in vacuo to give 80 g of a light yellow liquid. The crude oil is purified via vacuum distillation (0.1 Torr, bath temperature 60-70° C), to give 50.7 g of the title product as a colorless liquid.

Physical characteristics are as follows:

$^1H$ NMR (δ, $CDCl_3$): 3.80, 2.16, 2.07.
IR ($cm^{-1}$, Chloroform): 2945, 1707, 1610, 1433, 1366, 1092.
Mass spectrum (m/e): 194, 192, 179, 177, 163, 161, 53.
Exact mass found: 191.9793.
TLC (1:1 ethyl acetate/hexane, UV): Rf = 0.77.

Preparation 2 2-Bromo-3-methyl-2-buten-1-ol (formula A-3: $R_{61}$ is $CH_3$; $R_{60}$ is $CH_3$) Refer to Chart A.

To a solution of 50.7 g of methyl-2-bromo-3-methyl-2-butenoate in 720 ml methylene chloride at -78° C under argon is added dropwise over 1 hr 657 ml of a 1 M solution of diisobutylaluminum hydride/methylene chloride. The mixture is stirred at -78° C for an additional 2 hours. TLC analysis indicates complete conversion of starting material. The reaction is quenched with water (10 ml over 5 minutes, stirred an additional 10 minutes, until hydrogen gas evolution ceases), allowed to warm to room temperature, and the methylene chloride taken off under reduced pressure. To the resulting white solid is added 1400 ml ether and 60 ml water; a thick white precipitate forms. The mixture is dried with magnesium sulfate, filtered, and the filtrate concentrated in vacuo to give 38.43 g of the title product as a clear oil.

Physical characteristics are as follows:

$^1H$ NMR (δ, $CDCl_3$): 4.36, 2.89, 1.93, 1.90.
IR ($cm^{-1}$, Chloroform): 3401, 1652, 1389, 1370, 1132, 1101.
Mass spectrum (m/e): 166, 164, 151, 149, 146, 67.
Exact mass found: 163.9840.
TLC (1:1 ethyl acetate/hexane, p-anisaldehyde): Rf = 0.63, black.

Preparation3 2-Bromo-1-methanesulfonyloxy-3-methyl-2-butene (Formula A-4: $R_{61}$ is $CH_3$; $R_{60}$ is $CH_3$; $R_{40}$ is $CH_3$) and 1-Azido-2-bromo-3-methyl-2-butene (Formula A-5: $R_{61}$ is $CH_3$; $R_{60}$ is $CH_3$) Refer to Chart A.

To a solution of 38.4 g of 2-bromo-3-methyl-2-buten-1-ol and 48.7 ml of triethylamine in 1 L of tetrahydrofuran at -5°C is added dropwise over 20 minutes 27.0 ml of methanesulfonyl chloride . The mixture is stirred at -5°C for 1 hr to produce 2-bromo-1-methansulfonyloxy-3-methyl-2-butene. The reaction mixture is filtered (cake washed twice with 300 ml ether and 150 ml tetrahydrofuran) and 75 g of sodium azide in approximately 50 ml of water is added to the filtrate. The mixture is stirred at 0°C for 45 hr at which time TLC indicates complete conversion. The reaction mixture is poured into 2 L of water, extracted with methylene chloride first with 600 ml, then three times with 300 ml, the combined organic layers are washed with 300 ml of saturated aqueous sodium chloride, dried with magnesium sulfate, filtered and concentrated in vacuo to give 42.1 g of 1-azido-2-bromo-3-methyl-2-butene as a yellow liquid.

Physical characteristics are as follows:
2-Bromo-1-methanesulfonlyloxy-3-methyl-2-butene: TLC (1:1 ethyl acetate/hexane, p-anisaldehyde): Rf = 0.62, purple.
1-Azido-2-bromo-3-methyl-2-butene: $^1H$ NMR (δ, $CDCl_3$): 4.19, 1.97, 1.88. TLC (1:1 ethyl acetate/hexane, p-anisaldehyde): Rf = 0.67, magenta.

Preparation 4 1-Amino-2-bromo-3-methyl-2-butene (Formula A-6: $R_{61}$ is $CH_3$; $R_{60}$ is $CH_3$) Refer to Chart A.

To a suspension of 17.6 g of lithium aluminum hydride in 1.0 L of ether is added dropwise over 1 hr., maintaining a temperature of 5-10°C, a solution of 42 g of 1-azido-2-bromo-3-methyl-2-butene in 78 ml of ether. This is stirred an additional 0.5 hr, then quenched by the sequential addition of 18.3 ml of water (dropwise over 0.5 hr.), 18.3 ml 15% aqueous potassium hydroxide, and 55 ml of water. Another 300 ml of ether is added and the mixture dried with magnesium sulfate, filtered (solids washed three times with 400 ml ether), and concentrated under reduced pressure to give 35.9 g of a yellow liquid. This is purified via vacuum distillation (0.1 Torr, bath temperature 60°C) to give 33.44 g of the title product as a colorless liquid.

Physical characteristics are as follows:
$^1H$ NMR (δ, $CDCl_3$): 3.54, 1.89, 1.84, 1.89-1.84.
TLC (1:1 ethyl acetate/hexane, p-anisaldehyde): Rf = 0.10, black.
IR ($cm^{-1}$, Chloroform): 3375, 2917, 1649, 1591, 1443, 1368.

Preparation 5 3-(1-Methylethylidene)-2-azetidinone (Formula A-7: $R_{61}$ is $CH_3$; $R_{60}$ is $CH_3$) Refer to Chart A.

A mixture of 4.71 g of 1-amino-2-bromo-3-methyl-2-butene, 1.68 g of tetrakis(triphenylphosphine)palladium (O), 1.53 g of triphenylphosphine, and 7.62 ml of tributylamine in 470 ml of dimethylformamide is heated at 125-130°C under an atmosphere of carbon monoxide (balloon) for 1.5 hr. The reaction is allowed to cool to room temperature overnight, then the dimethylformamide is taken off in vacuo. To the crude residue is added approximately 40 ml of ethyl acetate. The mother liquor is decanted (filter stick), and the remaining solid washed two times with 15 ml of 25% ethyl acetate/pentane to give 1.32 g of a light yellow solid. A second crop is obtained by cooling the combined washings and mother liquor to -78°C and filtering to give 196 mg of a light yellow solid. The mother liquid is concentrated in vacuo, dissolved in a minimum amount of ethyl acetate, cooled to -78°C, and the mother liquor decanted to give a brown, oily solid. This is purified by sublimation to give 193 mg of a white solid (m.p. = 156-158°C). Total weight = 1.71 g. The analytical sample is obtained after triple sublimation (0.01 Torr, bath temperature = 55-60°C).

Physical characteristics are as follows:
$^1H$ NMR (δ, $CDCl_3$): 6.67, 3.70, 2.06, 1.73.
IR ($cm^{-1}$, Chloroform): 3439, 1739, 1440, 1371, 1131.
Mass spectrum (m/e): 111, 96, 82, 68, 67.
Exact mass found: 111.0680.
TLC (1:1 ethyl acetate/hexane, p-anisaldehyde): Rf = 0.23, baby blue.
Anal. found: C, 64.08; H, 8.11; N, 12.21

Example 1 1-Acetyl-3-(1-methylethylidene)-2-azetidinone (Formula B-4: $R_{61}$ is methyl, $R_{60}$ is methyl and $R_3$ is methyl) Refer to Chart B.

A mixture of 0.030 g of 3-(1-methylethylidene)-2-azetidinone, 0.020 g of 4-N,N-dimethylaminopyridine, and 0.029 ml of acetic anhydride in 1 ml of pyridine is stirred at 20°C for 21.5 hours. The mixture is concentrated in vacuo. and the residue purified by preparative TLC (20 cm x 20 cm 1000 microns silica gel, 1:1 ethyl acetate/hexane) to give 0.024 g of the title product as a white solid.

Physical characteristics are as follows:
$^1H$ NMR (δ. $CDCl_3$): 3.97. 2.40, 2.14, 1.74.
IR ($cm^{-1}$, Chloroform): 1775, 1716, 1684.

TLC (1:1 ethyl acetate/hexane, p-anisaldehyde): Rf = 0.47, light blue.

Example 2 3-(1-Methylethylidene)-1-(methylthioacetyl)-2-azetidinone (Formula B-4: $R_{61}$ is methyl, $R_{60}$ is methyl and $R_3$ is methylthiomethyl) Refer to Chart B.

Preparation of methylthioacetyl chloride: A solution of potassium hydroxide (31.36 g) in 250 ml methanol is added dropwise to a solution of ethyl (methylthio) acetate (25 g, 98% Aldrich) in 150 ml methanol at 0° C. Upon concentrating in vacuo a white solid forms. The solid is dissolved in 100 ml water and extracted once with 100 ml of diethyl ether. The aqueous layer is adjusted to pH 3 with 3N aqueous hydrochloric acid and extracted twice with 150 ml of ethyl acetate. The combined organic layers are washed once with 150 ml of brine, dried with magnesium sulfate. and concentrated in vacuo to give 18.3 g of the acid as a colorless liquid which solidifies on standing at 0° C. A mixture of methylthioacetic acid (3.45 g) and 10 ml of thionyl chloride is refluxed until no more gas evolves. The solution turns gold in color. Excess thionyl chloride is removed by distillation at 50° C at approximately 30 Torrr vacuum followed by distillation of the acid chloride at approximately 0.1 Torr at 20° C to give 2.53 g of methylthioacetyl chloride as a colorless liquid: $^1H$ NMR (δ, $CDCl_3$): 3.66, 2.25.

To a solution of 3-(1-methylethylidene)-2-azetidinone (25 mg) and triethylamine (91 mg) in 2 ml of methylene chloride under argon at 0° C is added a solution of (methylthio)acetyl chloride (95 mg) in 1 ml of methylene chloride. The mixture is allowed to warm to 20° C overnight, then concentrated in vacuo. The residue is purified by chromatography on a Uniplate silica gel GF taper plate (eluted two times in 20% ethyl acetate/hexane) to give 19.5 mg of the title product as a gold solid.

Physical characteristics are as follows:

$^1H$ NMR (δ, $CDCl_3$): 4.0, 3.6-3.56, 2.21, 2.13, 1.82.

IR ($cm^{-1}$, chloroform): 3507, 2916, 1767, 1718, 1679, 1346, 1289, 1121.

Example 3 1-(Cyanoacetyl)-3-(1-methylethylidene)-2-azetidinone (Formula B-4: $R_{61}$ is methyl, $R_{60}$ is methyl and $R_3$ is cyanomethyl) Refer to Chart B.

A mixture of 0.020 g of 3-(1-methylethylidene)-2-azetidinone, 0.017 g of 4,4-dimethylaminopyridine, 0.038 g of cyanoacetic acid, and 0.076 g of dicyclohexylcarbodiimide in 5ml of freshly distilled tetrahydrofuran is stirred at 20° C under argon for 46 hrs. The mixture is filtered, the solid washed with a small amount of ether, and the filtrate concentrated in vacuo. The residue is purified by preparative TLC (20 cm x 20 cm, 1000 microns silica gel, 1:1 ethyl acetate/hexane) to give 0.010 g of approximately 90% pure title product as a white solid.

Physical characteristics are as follows:

$^1H$ NMR (δ, $CDCl_3$): 4.10, 3.96, 2.17, 1.87.

TLC (1:1 ethyl acetate/hexane, p-anisaldehyde): Rf = 0.51, green-brown.

Examples 4-10 (Refer to Chart B).

In Table I below, the N-acyl 3-isopropylidene 2-azetidinones of formula B-4 are prepared from 3-(1-methylethylidene)-2-azetidinone of formula B-1 (A-7) (Preparation 5) by one of the three acylation methods exemplified in Examples, 1, 2 and 3 above: A) anhydride/-4-N,N-dimethylaminopyridine, B) acid chloride/triethylamine, or C) carboxylic acid/dicyclohexylcarbodiimide, respectively.

All of the N-acyl 3-isopropylidene 2-azetidinones of Table I below are of the formula B-4, wherein $R_{61}$ is methyl, $R_{60}$ is methyl and $R_3$ is as defined. The preferred method of preparation and the appropriate reactant are also specified.

TABLE I

| Example | Method | Reactant | $R_3$ | Physical Data[a] |
|---|---|---|---|---|
| 4 | A | butyric anhydride | propyl | Exact mass:181.1106 |
| 5 | A | valeric anhydride | butyl | Rf = 0.60 |
| 6 | A | hexanoic anhydride | pentyl | $^1$H NMR ($\delta$,$CDCl_3$):3.94, 2.71, 2.13, 1.85, 1.85-1.5, 1.47-1.15, 0.90. |
| 7 | B | octanoyl chloride | heptyl | Rf = 0.61 |
| 8 | B | undecenoyl chloride | 9-decen-1-yl | Rf = 0.58 |
| 9 | C | 4-pentynoic acid | 3-butyn-1-yl | Rf = 0.63 |
| 10 | B | 6-bromo-hexanoyl chloride | 5-bromo-pentyl | Exact mass:287.0503 |

[a]Thin layer chromatography (TLC) spots are visualized herein most often by spraying the TLC plate with p-anisaldehyde spray and heating the plate; visualization by UV light or using phosphomolybdic acid spray is also used as appropriate; other known methods could also be used. TLC Rf values are dtermined in 1:1 ethyl acetate:hexane unless otherwise indicated by a footnote as follows: [b]10% ethyl acetate in hexane vol/vol); [c]30% ethyl acetate in hexane (vol/vol).

---

Examples 11 - 19 (Refer to Chart B).

General procedure for hydrogenation reactions: A mixture of the isopropylidene (e.g., 25 mg) of formula B-4 and 5 mg of 10o/o palladium/carbon or palladium black in 5 ml of ethyl acetate is alternately evacuated and filled with hydrogen gas from a balloon four times. The mixture is then stirred at 20° C for 2-24 hrs, until the absence of starting material is indicated by TLC. The mixture is then filtered through a short plug of Celite, the pad washed with 1 ml of ethyl acetate, and the filtrate concentrated in vacuo to produce the 3-isopropyl analogs of formula B-3, as listed in Table II below. The product is homogenous by TLC and $^1$H NMR, and does not require subsequent purification.

All of the N-acyl 3-isopropyl 2-azetidinones of Table II below are of the formula B-3, wherein $R_{61}$ is methyl, $R_{60}$ is methyl and $R_3$ is as defined. The example in which the isopropylidene precursor is prepared is also specified.

TABLE II

| Example | Precursor Example | $R_3$ | Physical Data[a] |
|---|---|---|---|
| 11 | 1 | methyl | $^1$H NMR ($\delta$,$CDCl_3$): 3.60, 3.29, 3.03, 2.37, 2.03, 1.1, 1.0. |
| 12 | 4 | propyl | Exact mass:183.1252 |
| 13 | 5 | butyl | $^1$H NMR ($\delta$,$CDCl_3$): 3.57, 3.28, 3.01, 2.67, 2.02, 1.85-1.2, 1.1, 1.0, 0.9. |
| 14 | 6 | pentyl | Exact mass: 211.1569 |
| 15 | 7 | heptyl | $^1$H NMR ($\delta$,$CDCl_3$): 3.55, 3.26, 3.00, 2.64, 1.99, 1.7-1.4, 1.27, 1.1-0.8. |
| 16 | 10 | 5-bromo-1-pentyl | Exact mass: 289.0664 |
| 17 | 2 | methyl-thio-methyl | $^1$H NMR ($\delta$,$CDCl_3$): 3.8-3.5, 3.53, 3.36, 3.12, 2.20, 2.1, 1.1, 1.0. |
| 18 | 3 | cyano-methyl | Rf = 0.60 |
| 19 | 8 | decyl | Exact mass: 239.1861 |

[a]Refer to Footnote a of Table I.

Preparation 6 1-(Tert-butyldimethylsiloxy)-2-propanone (Formula A-1: $R_{60}$ is methyl, $R_{61}$ is tert-butyldimethylsiloxy-methyl) Refer to Chart A.

A mixture of 24.14 g of tert-butyldimethylsilyl chloride, 27.0 g of imidazole, and 15.0 ml of acetol in 100 ml of dry dimethylformamide is stirred at 35-40° C for 2 hr. The mixture is poured into 500 ml of water and extracted four times with 100 ml of hexane. The combined organic layers are washed with 20 ml of water, then 200 ml of saturated aqueous sodium chloride, dried with magnesium sulfate, and concentrated by rotary evaporation to give 31.28 g of the title product as a colorless liquid, homogeneous by TLC (Rf = 0.57, 20% ethyl acetate/hexane).

Physical characteristics are as follows:

$^1$H NMR ($\delta$, $CDCl_3$): 4.08, 2.10, 0.87, 0.4.

Preparation 7 Methyl 2-bromo-4-tert-butyldimethylsiloxy-3-methyl[E,Z]-2-butenoate (Formula A-2: $R_{61}$ is tert-butyldimethylsiloxy-methyl, $R_{60}$ is methyl or vice versa) Refer to Chart A.

To a slurry of 2.90 g of 50% sodium hydride dispersion in oil in 225 ml of freshly distilled tetrahydrofuran under nitrogen at room temperature is added over 10 min 9.4 ml (10.6 g) of trimethyl phosphono-acetate. (Caution: hydrogen evolution). The resulting thick, white suspension is stirred for 15 min, then 3.0 ml of bromine is added dropwise over 10 min, followed by an additional 0.5 ml of bromine to maintain a light yellow coloration in the reaction mixture. The resultant slurry is cooled to 5°C and 2.8 g of 50% sodium hydride dispersion in oil is added with care in five portions. (Caution: hydrogen evolution). After the addition is complete, the mixture is allowed to warm to room temperature with stirring for 12 min, then 10.33 g or 1-tert-butyldimethylsiloxypropanone in 10 ml of dry tetrahydrofuran is added over 5 min. The mixture is stirred at room temperature for 21 hr, then poured into 300 ml of water and 300 ml of ethyl acetate. The aqueous layer is extracted two times with 150 ml of ethyl acetate, one time with 200 ml of ethyl acetate, and the combined organic layers washed two times with 500 ml of saturated aqueous sodium chloride, dried with magnesium

sulfate, and concentrated by rotary evaporation to give 18.82 g of a dark amber oil. The residue is purified by medium pressure liquid chromatography on a 5.5 x 35 cm 40-63 microns silica gel column. The column is eluted consecutively with 300 ml hexane, 1 L 1% ethyl acetate/hexane, 1 L 1.5% ethyl acetate/hexane, and finally 2.0 % ethyl acetate/hexane, and 20 ml fractions are collected. Fractions 71-95 yield 5.85 g of a colorless oil, consisting by $^1$H NMR of 5:1 E and Z isomers of the title product. Fractions 96-100 yield 2.75 g of a 0.8:1 E and Z isomers of the title product. Fractions 101-105 yield 1.98 g of the title product as a colorless oil (0.4:1 E and Z isomers). An additional 1.18 g of the Z isomer of the title product is obtained in fractions 106-114, contaminated with uncharacterized impurities.

Physical characteristics are as follows:

E isomer: 1H NMR (δ, $CDCl_3$): 4.51, 3.78, 2.08, 0.89, 0.06.

Z isomer: $^1$H NMR (δ, $CDCl_3$): 4.38, 3.76, 2.13, 0.90, 0.09.

E and Z isomers: mass spectrum (m/e): 324, 322, 309, 307, 89; exact mass found: 307.0350; IR ($cm^{-1}$, $CDCl_3$): 1712.

Preparation 8 2-Bromo-4-tert-butyldimethylsiloxy-3-methyl-[E,Z]-2-buten-1-ol (Formula A-3: $R_{61}$ is tert-butyldimethylsiloxy-methyl, $R_{60}$ is methyl or vice versa) Refer to Chart A.

To a solution of 10.26 g of methyl 2-bromo-4-tert-butyldimethylsiloxy-3-methyl-[E,Z]-2-butenoate in 230 ml of freshly distilled tetrahydrofuran at -78° C under nitrogen is added dropwise over 10 min 50 ml (10.9 g) of a 25 wt% solution of DIBAL-H/toluene. The mixture is stirred at -78° C for 6.5 hr. TLC analysis indicates some starting material still present. The mixture is then warmed to -15° C (ice-salt bath) and stirred for 20 min, then quenched with 3.3 ml of acetone. The mixture is allowed to warm to room temperature, then 4.5 ml of dilute aqueous potassium hydroxide solution (prepared by adding 0.3 ml 15% aqueous potassium hydroxide to 9.5 ml of water) is added, and the mixture stirred 18 hr. A thixk gelatinous precipitate is formed. Ether (500 ml) is added, and the mixture dried with magnesium sulfate. The mixture is filtered and the cake washed well two times with 100 ml of ether. The filtrate is concentrated in vacuo to give 8.34 g of a light yellow oil. The residue is purified by medium pressure liquid chromatography on a 5.5 x 35 cm 40-63 microns silica gel column. The column is eluted sequentially with 500 ml of 5% ethyl acetate/hexane, then 1 L each of 7.5%, 10%, 12.5%, and finally 15% ethyl acetate/hexane, taking 20 ml fractions after one initial 400 ml fraction. Fractions 9-34 yield 1.74 g of unreacted starting ester. Visualization of the TLC'd fractions is by UV and p-anisaldehyde stain. Fraction 55-79 yield 2.63 g of a colorless oil, the E isomer of the title product. Fraction 80-96 yield 2.03 g of a E and Z mixture of the title product as a colorless oil. Fractions 97-124 yield 0.90 g of pure Z isomer of the title product as a colorless oil.

Physical characteristics are as follows:

E isomer: $^1$H NMR (δ, $CDCl_3$): 4.39, 4.20, 2.2, 1.90, 0.87, 0.07.

Z isomer: $^1$H NMR (δ, $CDCl_3$): 4.35, 4.30, 1.86, 0.90, 0.09; IR ($cm^{-1}$, film): 3338, 1648; mass spectrum (m/e): 281, 279, 75; mass found: C, 45.11; H, 7.88; Br. 27.02.

Preparation 9 2-Bromo-4-tert-butyldimethylsiloxy-1-methanesulfonyloxy-3-methyl-[Z]-2-butene (Formula A-4: $R_{61}$ is tert-butyldimethylsiloxy, $R_{60}$ is methyl, $R_{40}$ is methyl);
1-Tert-butyldimethylsiloxy-2-methyl-3-bromo-4-azido-[Z]-2-butene (Formula A-5: $R_{61}$ is tert-butyldimethylsiloxy-methyl, $R_{60}$ is methyl) Refer to Chart A.

To a solution of 23.2 g of 2-bromo-4-tert-butyldimethylsiloxy-3-methyl-[Z]-2-buten-1-ol and 15.3 ml of triethylamine in 500 ml of freshly distilled tetrahydrofuran at -5° C (salt-ice bath) under argon is added dropwise over 10 min 7.3 ml of methanesulfonyl chloride to produce 2-bromo-4-tert-butyldimethylsiloxy-1-methanesulfonyloxy-3-methyl-[Z]-2-butene. After 3.0 hr the reaction mixture is filtered, and the triethylamine hydrochloride cake washed two times with 100 ml of tetrahydrofuran. The filtrate is combined with 45 ml dimethylformamide, cooled to 0° C, and combined with 20.9 g of sodium azide. TLC analysis after stirring at 3° C (cold room) for 64 hr shows complete conversion of starting material. The reaction mixture is poured into 600 ml of water, and extracted four times with 440 ml of ethyl acetate. The combined organic layers are washed with 800 ml saturated, aqueous sodium chloride, dried with potassium carbonate, filtered and concentrated in vacuo to give 22,4 g of 1-Tert-butyldimethylsiloxy-2-methyl-3-bromo-4-azido-[Z]-2-butene as a light tan oil which is 90% pure by NMR.

Physical characteristics are as follows:

2-bromo-4-tert-butyldimethylsiloxy-1-methanesulfonyloxy-3-methyl-[Z]-2-butene: TLC (0.5% isopropyl alcohol/methylene chloride, p-anisaldehyde): Rf =0.54, green-blue.

1-Tert-butyldimethylsiloxy-2-methyl-3-bromo-4-azido-[Z]-2-butene: $^1$H NMR (δ, $CDCl_3$): 4.33, 4.15, 1.89, 0.94, 0.12. TLC (1:1 ethyl acetate/hexane, p-anisaldehyde): Rf= 0.72, (2% ethyl acetate/-hexane), Rf = 0.38, green.

Likewise. the above procedure is used to obtain the E isomers of the title products.

Preparation 10 1-Tert-butyldimethylsiloxy-2-methyl-3-bromo-4-amino-[Z]-2-butene (Formula A-6: $R_{61}$ is methyl. $R_{60}$ is tert-butyldimethylsiloxy methyl) and
1-Tert-butyldimethylsiloxy-2-methyl-3-bromo-4-amino-[E]-2-butene (Formula A-6: $R_{61}$ is tert-butyldimethylsiloxy-methyl, $R_{60}$ is methyl) Refer to Chart A.

To a slurry of 5.55 g of 95% lithium aluminum hydride in 530 ml ether cooled to 0° C, is added dropwise over

50 min a solution of 22.3 g of the azide, 1-tert-butyldimethylsiloxy-2-methyl-3-bromo-4-azido-[Z]-2-butene, in 175 ml ether, maintaining a reaction temperature of 2-5°C. TLC analysis after 5 min shows complete conversion of starting material. The reaction is quenched with the cautious addition of 5.5 ml of water, followed by 5.5 ml of 15% aqueous potassium hydroxide, then 16.5 ml of water. The mixture is dried with potassium carbonate, filtered, and the filtrate concentrated in vacuo to give 18.44 g of a light yellow liquid. A 251 mg aliquot is purified by preparative TLC (20 x 20 cm, 2000 microns silica gel, 1:1 ethyl acetate/hexane) to give 220 mg of pure 1-tert-butyldimethylsiloxy-2-methyl-3-bromo-4-amino-[Z]-2-butene. The bulk of the allylic amine is utilized without further purification.

Physical characteristics of the Z isomer are as follows:
$^1H$ NMR (δ, $CDCl_3$): 4.28, 3.54, 1.83, 1.62, 0.91, 0.09.
IR ($cm^{-1}$, chloroform): 3382, 1646.
TLC (1:1 ethylacetate/hexane, p-anisaldehyde): Rf = 0.16, yellow.
Mass spectrum (m/e, FAB): 294, 277, 162, 115, 73.
GC (3 ft. SP 2250, 150°C): Retention time = 3.92 min.

To a solution of 6.227 g of 1-tert-butyldimethylsiloxy-2-methyl-3-bromo-4-azido-[E]-2-butene in 140 ml of ether at 0°C is added 1.488 g of lithium aluminum hydride in several portions. The mixture is stirred at 0°C for 10 min, then 6.3 ml of water is cautiously added, followed by 6.3 ml of 15% aqueous potassium hydroxide, then 18.8 ml of water. The resulting mixture is dried with potassium carbonate, and concentrated. The residue is purified by medium pressure liquid chromatography (4.5 x 26 cm, 55% ethyl acetate/hexane) to give 3.11 g of 1-tert-butyldimethylsiloxy-2-methyl-3-bromo-4-amino-[E]-2-butene.

Physical characteristics of the E isomer are as follows:
$^1H$ NMR (δ, $CDCl_3$): 4.1, 3.6, 1.95, 1.9, 0.9, 0.05.

Preparation 11 3-[Z]-(1-(tert-butyldimethylsiloxymethyl)-ethylidene)-2-azetidinone (Formula C-1: $R_{61}$ is methyl, $R_{30}$ is hydrogen, $(R_{24})(R_{25})(R_{26})$ is tert-butyldimethyl) and 3-[E]-(1-(tert-butyldimethylsiloxymethyl)ethylidene-2-azetidinone (Formula C-6: $R_{60}$ is methyl, $R_{31}$ is hydrogen, $(R_{24})(R_{25})(R_{26})$ is tert-butyldimethyl) Refer to Chart C.

A mixture of 1.07 g of 1-tert-butyldimethylsiloxy-2-methyl-3-bromo-4-amino-[Z]-2-butene, 0.042 g of tetrakis(triphenylphosphine) palladium (O), 0.019 g of triphenylphosphine, and 0.56 ml of triethylamine in 80 ml dimethylformamide is heated at 95-100°C under an atmosphere of carbon monoxide for 21.5 hr. GC analysis indicates complete conversion of starting material. The mixture is poured into 140 ml of water, extracted once with 70 ml of ether and three times with 33 ml of ether, and the combined organic layers washed with 33 ml of water, dried with magnesium sulfate, filtered and concentrated in vacuo to give 0.99 g of a yellow crystalline solid. This solid is purified by medium pressure liquid chromatography on a 4.5 x 19.5 cm 40-63 microns silica gel column eluted with 40% ethyl acetate/hexane. Taking 20 ml fractions, fraction 17-29 yield 0.626 g of 3-[Z]-(1-tert-butyldimethylsiloxymethylethylidene)-2-azetidinone as a white crystalline solid, mp = 89.5 92.0°C.

Physical characteristics of the Z isomer are as follows:
$^1H$ NMR (δ, $CDCl_3$): 6.52, 4.50, 3.69, 1.78, 0.92, 0.12.
IR ($cm^{-1}$, mineral oil mull): 3414, 1748, 1724.
TLC (1:1 ethyl acetate/hexane, p-anisaldehyde): Rf = 0.43, bright yellow.
Mass spectrum (m/e): 241, 226, 184,; exact mass found: 241.1492.
Anal. found: C, 59.46; H, 9.47; N, 5.74.
GC (3 ft. SP 2250, 150°C): Retention time = 13.3 min.

A mixture of 1.256 g of 1-tert-butyldimethylsiloxy-2-methyl-3-bromo-4-amino-[E]-2-butene, 0.136 g of tetrakis(triphenylphosphine) palladium (O), 0.065 g of triphenylphosphine, and 0.47 ml of triethylamine in 60 ml of dimethylformamide all under an argon atmosphere, is alternatively evacuated and filled four times with carbon monoxide via a balloon fitted with a needle, and stirred at 100°C for 4 hr. TLC analysis at this time shows only partial conversion. TLC analysis after an additional 3.5 hr shows complete conversion of starting material. The reaction mixture is cooled to room temperature then poured into 200 ml of water and extracted once with 85 ml of ether, three times with 40 ml of ether. The combined organic layers are washed with 40 ml of water, dried with magnesium sulfate, and concentrated in vacuo to give a brown oil with some yellow solid. The residue is purified by medium pressure liquid chromatography on a 4.0 x 17 cm 40-63 microns silica gel column eluted with 35% ethyl acetate/hexane. Collecting approximately 20 ml fractions, 20-32 yield 0.579 of 3-[E]-)tert-butyldimethylsiloxymethylethylidene)azetidin-2-one as a light yellow solid (m.p. = 87°C). In addition, fractions 33-50 give 0.075 g of 50% pure 3-[E]-)tert-butyldimethylsiloxymethylethylidene)azetidin-2-one as tan crystals.

Physical characteristics of the E isomer are as follows:
$^1H$ NMR (δ, $CDCl_3$): 5.75, 4.12, 3.84, 1.95, 0.90, 0.06.
IR ($cm^{-1}$, $CDCl_3$): 3533, 1736.
Mass spectrum (m/e, FAB): 242, 73.
Exact mass found: 280.1119.

Preparation 12 3-[Z]-(1-(Hydroxymethyl)ethylidene)-2-azetidinone (Formula C-2 (D-1): $R_{30}$ is hydrogen, $R_{61}$ is methyl), and 3-[E]-(1-(hydroxymethyl)-ethylidene)-2-azetidinone Refer to Chart C.

To a solution of 2.162 g of silyl ether 3-[Z]-)1-tert-butyldimethylsiloxymethylethylidene)-2-azetidinone, in 50 ml of dry tetrahydrofuran at 20°C under argon atmosphere, is added at once 27 ml of 1 M tetrabutylammonium fluoride/tetrahydrofuran. The mixture is stirred for 1 hr. then concentrated by rotary evaporation; the residue is immediately chromatographed on a 4 cm x 22 cm column 40-63 microns silica gel (gradient elution with 25%, 35% and finally 65% acetone/methylene chloride) to give 1.006 g of 3-[Z]-(1-hydroxymethylethylidene)-2-azetidinone as a light yellow solid.

Physical characteristics of the Z isomer are as follows:
TLC (1:1 ethyl acetate/hexane): Rf = 0.06; (1:1 methylene chloride/acetone, p-anisaldehyde): Rf = 0.49, yellow.
$^1$H NMR (δ, $CD_3OD$): 4.39, 3.72, 1.82, 1.57.
IR (cm$^{-1}$, Nujol): 3290, 1724, 1461.
Mass spectrum (m/e, FAB): KI added gives 166.
Mass found: 166.0266.

To a solution of 1.31 g of silyl ether 3-[E]-(1-tert-butyldimethylsiloxymethylethylidene)azetidin-2-one, in 30 ml of tetrahydrofuran at 20°C is added 16 ml of 1 M tetrabutylammonium fluoride/ tetrahydrofuran. The mixture is stirred for 1 hr, then concentrated in vacuo and the residue immediately chromatographed on a 5.5 cm x 24 cm 63-200 microns silica gel column, (20%, 30%, 50%, and finally 75% acetone/methylene chloride eluant) to give 0.634 g of 3-[E]-(1-hydroxymethylethylidene)-2-azetidinone as a white crystalline solid.

Physical characteristics of the E isomer are as follows:
TLC (100% ethyl acetate/hexane, p-anisaldehyde): Rf = 0.14, yellow.
IR (cm$^{-1}$, Nujol): 3202, 1720.
$^1$H NMR (δ, $CD_3OD$): 4.06, 3.83, 1.97.
Mass spectrum (m/e, FAB): 128 observed.

Preparation 13 3-[Z]-(1-(acetoxymethyl)ethylidene)-2-azetidinone; (Formula D-3: $R_{61}$ is methyl, $R_{30}$ is hydrogen, $R_5$ is methyl) Refer to Chart D.

A solution of 0.34 g of alcohol, 3-[Z]-(1-hydroxymethylethylidene)-2-azetidinone, in 3 ml of dry pyridine and 1 ml of acetic anhydride is stirred at 20°C for 18 hr. The mixture is poured into 25 ml of water and extracted three times with 10 ml of methylene chloride. The combined organic layers are washed with 10 ml of saturated aqueous sodium chloride, dried with sodium sulfate, and concentrated in vacuo to give 0.258 g of a yellow oil. On standing at -10°C overnight, the oil partially crystallizes. The mixture is triturated with 1 ml of hexane and 5 ml of ether, which serves to dissolve the oil. The ether-hexane layer is decanted from the solid, and the white crystalline solid washed two times with 1 ml of ether. These washes are combined with the mother liquor to yield 0.170 g of a yellow oil upon evaporation of the solvent. The slightly off-white crystals are collected to give 0.093 g (m.p. = 93-97°C) of 3-[Z]-(1-acetoxymethylethylidene)-2-azetidinone. The mother liquor is purified by preparative TLC (1000 microns, 20 x 20 cm silica gel, 1:1 ethyl acetate/hexane) to give an additional 0.053 g of 3-[Z]-(1-acetoxymethylethylidene)-2-azetidinone as a white crystalline solid.

Physical characteristics are as follows:
$^1$H NMR (δ, $CDCl_3$): 6.8-6.5, 4.95, 3.74, 2.10, 1.77.
TLC (1:1 ethyl acetate/hexane): Rf = 0.15.

Likewise, the above procedure is used to obtain the E isomer of the title product.

Preparation 14 (Z)-3-[1-Methyl-2-(1-oxobutoxy)ethylidene]-2-azetidinone (Formula D-3: $R_{61}$ is methyl, $R_{30}$ is hydrogen, $R_5$ is n-propyl) Refer to Chart D.

A mixture of 0.092 g of 3-[Z]-(1-hydroxymethylethylidene)-2-azetidinone and 1.2 ml of butyric anhydride in 5.0 ml of pyridine is stirred at 20°C for 20 hr. TLC analysis shows complete conversion of starting material. After the standard workup, 0.373 g of a tan and white solid is obtained, which is purified by preparative TLC (2000 microns, 20 x 20 cm silica gel, 1:1 ethyl acetate/hexane) to give 0.105 g of the title product as a white crystalline solid, m.p. = 81.5-86.0°C.

Physical characteristics are as follows:
$^1$H NMR (δ, $CDCl_3$): 6.95, 4.98, 3.76, 2.32, 1.78, 1.8-1.4, 0.98.
IR (cm$^{-1}$, mineral oil mull): 3225, 1735, 1715.
Mass spectrum (m/e, FAB): 198, 182, 169, 154, 126; exact mass found: 198.1126.

Example 20 1-Acetyl-3-[Z]-(1-(acetoxymethyl)ethylidene)-2-azetidinone (Formula D-2: $R_{61}$ is methyl, $R_{30}$ is hydrogen, $R_3$ is methyl, $R_5$ is methyl) Refer to Chart D.

A mixture of 0.032 g of 3-[Z]-(1-hydroxymethylethylidene)-2-azetidinone, 0.002 g of 4-N,N-dimethylaminopyridine, and 1.0 ml of acetic anhydride in 3.0 ml of pyridine is stirred at 20°C for 6 hr. After the standard workup procedure, 0.082 g of a light yellow oil is obtained. The residue is chromatographed on silica gel 40-63 microns (0.5 cm x 9 cm, 1:1 ethyl acetate/hexane, 0.5 ml fractions). Fractions 3-5 give 0.044 g of 1-acetyl-3-[Z]-(1-acetoxymethylethylidene)2-azetidinone as a light yellow oil. TLC analysis indicates minor impurities. A 0.028 g sample is purified by preparative TLC (1000 microns, 1:1 ethyl acetate, 20 cm x 20 cm in silica gel) to give 0.0221 g of pure 1-acetyl-3-[Z]-(1-acetoxymethylethylidene)-2-azetidinone as a colorless oil which crystallizes

on standing at -10°C to give a light yellow solid, m.p = 53-55°C.
Physical characteristics are as follows:
$^1H$ NMR (δ, $CD_3OD$): 5.00, 4.02, 2.42, 2.13, 1.87.
TLC (1:1 ethyl acetate/hexane) Rf = 0.34.
IR ($cm^{-1}$, $CDCl_3$): 1779, 1739, 1689.
Exact mass found: 212.0934.

Example 21 (Z)-3-[1-Methyl-2-(1-oxoheptyloxy)ethylidene]-1-(1-oxoheptyl)-2-azetidinone (Formula D-2: $R_{61}$ is methyl, $R_{30}$ is hydrogen, $R_5$ is n-hexyl, $R_3$ is n-hexyl) Refer to Chart D.

To a solution of 0.021 g of 3-[Z]-(1-hydroxymethylethylidene)-2-azetidinone and 0.049 ml of triethylamine in 5 ml of methylene chloride at 0°C is added 0.054 ml of heptanoyl chloride over 30 sec. The mixture is stirred at 0°C for 30 min, then at 20°C for 1.6 hr. TLC shows incomplete conversion. After an additional 2 equivalents each of triethylamine and heptanoyl chloride is added (0°C), the mixture is again stirred at 20°C for 1.7 hr. Finally 0.1 ml each of the above two reagents are added at 20°C, and after 19 hr, the mixture is concentrated in vacuo. The residue is purified by preparative TLC (1000 microns, 20 cm x 20 cm, 1:1 ethyl acetate/hexane) to give 0.035 g of the title product as an oily solid.
Physical characteristics are as follows:
$^1H$ NMR (δ, $CDCl_3$): 5.00, 4.02, 2.72, 2.35, 1.86, 1.73-0.77.
IR ($cm^{-1}$, chloroform): 1776, 1733, 1692
TLC (1:1 ethyl acetate/hexane, p-anisaldehyde): Rf = 0.65, yellow.

Example 22 [Z]-1-(4-Cyclohexyl-1-oxobutyl)-3-[1-methyl-2-(1-oxobutoxy)ethylidene]-2-azetidinone (Formula D-2: $R_{61}$ is methyl, $R_{30}$ is hydrogen, $R_5$ is n-propyl, $R_3$ is 3-cyclohexyl-n-propyl) Refer to Chart D.

A mixture of 0.012 g of (Z)-3-[1-methyl-2-(1-oxobutoxy)ethylidene]-2-azetidinone, 0.031 g of cyclohexanebutyric acid, 0.038 g of N,N-dicyclohexylcarbodiimide, and 0.011 g of N,N-dimethylaminopyridine in 1.5 ml of methylene chloride is stirred at 20°C for 22.5 hr. TLC analysis indicates complete conversion of starting material. The reaction mixture is immediately purified by preparative TLC (1000 microns, 20 x 20 cm, silica gel, 1% acetone/chloroform) to give 0.022 g of the title product as a white solid.
Physical characteristics are as follows:
$^1H$ NMR (δ, $CDCl_3$): 4.99, 3.98, 2.68, 2.33, 1.83, 1.9-1.45, 1.37-0.68, 0.95.
IR ($cm^{-1}$, chloroform): 1775, 1735, 1692.
TLC (1:1 ethyl acetate/hexane, p-anisaldehyde): Rf = 0.68; (1% acetone/methylene chloride): Rf = 0.31, yellow.
Mass spectrum (m/e, FAB) 350, 280, 252, 225, 128; upon addition of potassium iodide a peak at m/e = 388.
Exact mass found: 388.1901.

Examples 23 - 45 (Refer to Chart D)

In Table III below, the O,N-bisacyl E and Z alkylidene-2-azetidinones are prepared by one of the three acylation methods exemplified in Examples 20, 21 and 22 above: A) anhydride/4-N,N-dimethylaminopyridine, B) acid chloride/triethylamine or C) carboxylic acid/dicyclohexylcarbodiimide, respectively. The bisacyl analogs wherein $R_5$ and $R_3$ are different are prepared from the formula D-3 precursor of Preparation 13 or 14, as indicated. The bisacyl analogs wherein $R_5$ and $R_3$ are the same are prepared from the formula D-1 (C-2) precursor of Preparation 12, as specified.

Standard workup for acylations: To the stirring reaction mixture (in 2 ml of pyridine, e.g.) is added 4 ml each of methylene chloride and saturated aqueous ammonium chloride and the mixture briefly stirred (or shaken). The layers are separated, and the aqueous layer is extracted twice with 4 ml of methylene chloride. The combined organic layers are dried with magnesium sulfate, concentrated by rotary evaporation, and the residual pyridine removed in vacuo (approximately 0.05 Torr). The residue is then purified by the appropriate chromatographic method.

In Examples 31 and 42 below, using the acid chloride method, acid-catalyzed olefin isomerization occurs. Acylation of the Z isomer of Preparation 12 results in complete isomerization of the double bond to give the E isomer of Example 42. Acylation of the E isomer of Preparation 12 results in partial olefinic isomerization to give a mixture of the E and Z isomers of Example 31.

All of the O,N-bisacyl E and Z alkylidene-2-azetidinones of Table III below are of the formula D-2, wherein $R_{61}$ is methyl, $R_{30}$ is hydrogen and $R_3$ and $R_5$ are as defined. The preferred method of preparation and the appropriate reactant are also specified.

In Table IV below, physical data are given for the O,N-biscacyl E and Z alkylidene-2-azetidinones prepared in Table III.

TABLE III

| Example | Method | Precursor | Reactant | $R_5$ | $R_3$ |
|---|---|---|---|---|---|
| 23 | A | Prep. 13 | butyric anhydride | $CH_3$ | $(CH_2)_2CH_3$ |
| 24 | A | Prep. 13 | hexanoic anhydride | $CH_3$ | $(CH_2)_4CH_3$ |
| 25 | A | Prep. 12 (Z isomer) | chloroacetic anhydride | $CH_2Cl$ | $CH_2Cl$ |
| 26 | A | Prep. 12 (Z isomer) | hexanoic anhydride | $(CH_2)_4CH_3$ | $(CH_2)_4CH_3$ |
| 27 | A | Prep. 14 | acetic anhydride | $(CH_2)_2CH_3$ | $CH_3$ |
| 28 | A | Prep. 12 (Z isomer) | butyric anhydride | $(CH_2)_2CH_3$ | $(CH_2)_2CH_3$ |
| 29 | B | Prep. 14 | isobutyryl chloride | $(CH_2)_2CH_3$ | $CH(CH_3)_2$ |
| 30 | A | Prep. 14 | hexanoic anhydride | $(CH_2)_2CH_3$ | $(CH_2)_4CH_3$ |
| 31 | B | Prep. 14 | octanoyl chloride | $(CH_2)_2CH_3$ | $(CH_2)_6CH_3$ |
| 32 | B | Prep. 14 | 10-undecenoyl chloride | $(CH_2)_2CH_3$ | $(CH_2)_8$-$CH{=}CH_2$ |
| 33 | B | Prep. 14 | 3-phenyl-propionyl-chloride | $(CH_2)_2CH_3$ | $CH_2CH_2$-phenyl |
| 34 | C | Prep. 14 | succinic acid mono benzyl ester | $(CH_2)_2CH_3$ | $(CH_2)_2$-$CO_2CH_2$-phenyl |
| 35 | B | Prep. 14 | benzoyl chloride | $(CH_2)_2CH_3$ | phenyl |
| 36 | C | Prep. 14 | 4-(2-thienyl) butyric acid | $(CH_2)_2CH_3$ | $(CH_2)_3$-2-thienyl |
| 37 | B | Prep. 14 | 3-chloro-propionyl chloride | $(CH_2)_2CH_3$ | $CH{=}CH_2$[1] |
| 38 | C | Prep. 14 | N-(carbo-benzyl-oxy)-L-proline | $(CH_2)_2CH_3$ | N-(carbo-ben-zyloxy)-L-prolyl[2] |

| Example | Method | Precursor | Reactant | $R_5$ | $R_3$ |
|---|---|---|---|---|---|
| 39 | A | Prep. 12 (E isomer) | acetic anhydride | $CH_3$ | $CH_3$ |
| 40 | A | Prep. 13 (E isomer) | butyric anhydride | $CH_3$ | $(CH_2)_2CH_3$ |
| 41 | A | Prep. 12 (E isomer) | butyric anhydride | $(CH_2)_2CH_3$ | $(CH_2)_2CH_3$ |
| 42 | B | Prep. 12 (Z isomer) | isobutyryl chloride | $CH(CH_3)_2$ | $CH(CH_3)_2$ |
| 43 | C | Prep. 12 (E isomer) | 4-cyclo-hexylbuty-ric acid | $(CH_2)_3$-cyclohexyl | $(CH_2)_3$-cyclo-hexyl |
| 44 | C | Prep. 12 (E isomer) | trans-2-hexenoic acid | $CH{=}CH{-}(CH_2)_2CH_3$ | $CH{=}CH{-}(CH_2)_2CH_3$ |
| 45 | C | Prep. 12 (E isomer) | trans-3-hexenoic acid | $CH_2CH{=}CHCH_2CH_3$ | $CH_2CH{=}CHCH_2CH_3$ |

[1]Under the conditions of the acylation, HCl is eliminated producing this carbon-carbon double bond.

[2]This name is for the fragment $R_3CO$-.

TABLE IV

| Example | Physical Data[a] |
|---|---|
| 23 | Rf = 0.50 |
| 24 | Rf = 0.53 |
| 25 | Rf = 0.50 |
| 26 | Exact mass: 362.1749 |
| 27 | Rf = 0.58 |
| 28 | Exact mass: 268.1533 |
| 29 | Rf = 0.57 |
| 30 | Rf = 0.57 |
| 31 | Rf = 0.67 (E & Z isomer) |
| 32 | Rf = 0.61 |
| 33 | Rf = 0.59 |
| 34 | Rf = 0.48 |
| 35 | Rf = 0.11[b] |
| 36 | Exact mass: 350.1408 |
| 37 | Rf = 0.54 |
| 38 | Rf = 0.37 |
| 39 | Rf = 0.44 |
| 40 | Rf = 0.54 |
| 41 | Rf = 0.7, Rf = 0.10[b] |
| 42 | Rf = 0.37 (E isomer) |
| 43 | Rf = 0.71 |
| 44 | Rf = 0.50[c] |
| 45 | Rf = 0.47 |

[a]Refer to Footnote a in Table I.

Example 46 (Z)-1-Acetyl-3-[2-(chloroacetyloxy)-1-methylethylidene]-2-azetidinone (Formula D-2: $R_{61}$ is methyl, $R_{30}$ is hydrogen, $R_3$ is methyl, $R_5$ is methylchloro) Refer to Chart D.

Preparation of (Z)-3-[2-chloroacetyloxy)-1-methylethylidene]-2-azetidinone: A mixture of 0.0091 g of 3-[Z]-(1-hydroxymethylethylidene)-2-azetidinone and 0.243 g of chloroacetic anhydride in 1.4 ml of pyridine is stirred at 20° C for 3.5 hr, then 5 ml each of methylene chloride and water are added and the mixture stirred to 30 min. The layers are separated and the aqueous layer extracted two times with 4 ml of methylene chloride. The combined organic layers are washed two times with 5 ml of saturated aqueous ammonium chloride, dried with magnesium sulfate, and concentrated in vacuo to give 0.015 g of a yellow oil. The residue is purified by chromatography (1:1 ethyl acetate/hexane, 6 cm x 0.5 cm silica gel 40-63 microns, 1 ml fractions) to give in fractions 3 and 4, 1.2 mg of (Z)-1-chloroacetyl-3-[2-(chloroacetyloxy)-1-methylethylidene]-2-azetidinone as a yellow oil; and fractions 7-13, inclusive, affords 0.0033 g of the mono-O-chloroacetyl compound as a colorless oil, which crystallizes on standing at 0° C: TLC (1:1 ethyl acetate/hexane, UV): Rf = 0.22.

A solution of 0.0033 g of (Z)-3-[2-(chloroacetyloxy)-1-methylethylidene]-2-azetidinone, 0.001 g of 4-dimethylaminopyridine, and 0.3 ml (200 fold excess) of acetic anhydride in 1.0 ml of pyridine is stirred at 20° C for 17 hr. To the orange mixture is added 5 ml each of methylene chloride and saturated aqueous ammonium chloride and the aqueous layer extracted with 1 ml of methylene chloride. The combined organic layers are dried with magnesium sulfate and concentrated in vacuo to give 0.013 g of an orange residue. The residue is chromatographed (1:1 ethyl acetate/hexane, 5 cm x 0.5 cm silica gel 40-63 microns, 1 ml fractions) to give in fraction 4, 0.0010 g of the title compound as a colorless oil. (Fractions 3 and 5 yield an additional 0.0015 g of approximately 50% pure material).

Physical characteristics are as follows:
$^1H$ NMR (δ, $CDCl_3$): 5.07, 4.10, 4.02, 2.40, 1.86.
TLC (1:1 ethyl acetate/hexane, UV): Rf = 0.40.

Example 47 [Z]-1-[6-(N-Carbobenzyloxy) amino-1-oxohexyl]-3-[1-methyl-2-(1-oxobutoxy)ethylidene]-2-azetidinone (Formula D-2: $R_{61}$ is methyl, $R_{30}$ is hydrogen, $R_5$ is n-propyl and $R_3$ is 5-(N-carbobenzyloxy)amino-pentyl) Refer to Chart D.

Preparation of 6-(N-carbobenzyloxy)aminocaproic acid: To a solution of 8.2 g of 6-aminocaproic acid in 50 ml of 2 N aqueous sodium hydroxide at 0° C is added simultaneously by slow dropwise addition over 15 min 8.9 ml benzylchloroformate and 25 ml 4 N aqueous sodium hydroxide. The mixture is stirred for an additional 20 min then extracted with 75 ml ether. The aqueous phase is acidified to pH 1, filtered, and the resulting white solid washed with cold water, taken up in chloroform, washed with saturated sodium chloride, dried with magnesium sulfate, filtered, and concentrated in vacuo to give 15.14 g of 6-(N-carbobenzyloxy)aminocaproic acid as a white crystalline solid.

A mixture of 0.232 g of (Z)-3-[1-methyl-2-(1-oxobutoxy)ethylidene]-2-azetidinone, 0.469 g of 6-(N-carbobenzyloxy)amino caproic acid, 0.486 g of N,N'-dicyclohexylcarbodiimide, and 0.216 g of N,N-dimethylaminopyridine in 3 ml methylene chloride is stirred at 20° C for 17.5 hr. At this time the reaction solution is filtered and the filtrate concentrated in vacuo to give 1.1 g of a yellow solid. This is purified by preparative TLC (2-20 cm x 20 cm, 2000 microns silica gel plates, 1:1 ethyl acetate/hexane) to give 525 mg (quantitative yield) of the title product as a yellow solid.

Physical characteristics are as follows:
$^1H$ NMR (δ, $CDCl_3$): 7.29, 5.05, 4.98, 3.98, 3.16, 2.69, 2.31, 1.82, 1.9-1.08, 0.93.
TLC (1:1 ethyl acetate/hexane, UV): Rf = 0.46.

Example 48 (Z)-1-Acetyl-3-(2-benzylamino-1-methylethylidene)-2-azetidinone and (E)-1-Acetyl-3-(2-benzylamino-1-methylethylidene)-2-azetidinone.

To a 10 ml flask charged with 0.0045 g of triphenylphosphine and 0.0125 g of (Z)-1-acetyl-3-(1-acetyloxymethylethylidene)-2-azetidinone, previously evacuated and filled with argon is added 0.016 g of tetrakis(triphenylphosphine)palladium (O), and the flask alternately evacuated and filled four times with argon. Tetrahydrofuran (4 ml) is added via syringe, and the solution degassed with argon, then 0.012 ml of triethylamine and 0.010 ml of benzyl amine are added, and the mixture stirred at 20° C in the dark for 42 hr. The mixture is TLC'd and then concentrated in vacuo. The residue is purified by preparative TLC (300-1000 microns silica gel Taper plate, Analtech, 60% ethyl acetate/hexane) to give 0.0028 g of a colorless oil, identified as (E)-1-acetyl-3-(2-benzylamino-1-methylethylidene)-2-azetidinone (E, Rf = 0.53), and 0.0025 g of a colorless oil, assigned as (Z)-1-acetyl-3-(2-benzylamino-1-methylethylidene)-2-azetidinone (Z, Rf = 0.27).

Physical characteristics of the E isomer are as follows:
$^1H$ NMR (δ, $CDCl_3$): 7.30, 4.04, 3.72, 3.29, 2.41, 2.13.
IR ($cm^{-1}$, $CDCl_3$): 1770, 1684, 1594, 1362, 1317.
Mass spectrum (m/e, FAB): 259.
Exact mass found: 259.1427.

Physical characteristics of the Z isomer are as follows:
$^1H$ NMR (δ, $CDCl_3$): 7.28, 3.92, 3.73, 3.62, 2.34, 1.82.
IR ($cm^{-1}$, $CDCl_3$): 1773, 1689, 1600, 1320.
Mass spectrum (m/e, FAB): 259.

Examples 49-67 (Refer to Chart D).

General procedure for hydrogenation reactions: A mixture of the isopropylidene precursor (e.g. 25 mg) of formula D-2 and 5 mg of 10% palladium/carbon in 5 ml of ethyl acetate is alternately evacuated and filled with hydrogen (via a balloon) four times. The mixture is then stirred at room temperature from 2-24 hrs, until the absence of starting material is indicated by TLC. The mixture is then filtered through a short plug of Celite, the pad washed with 1 ml of ethyl acetate, and the filtrate concentrated in vacuo to produce the isopropyl analogs of formula D-5 as listed in Table V below. In most cases, TLC analysis indicates only one spot. Catalytic hydrogenation of the Z olefin provides the RR/SS racemeate and of the E olefin provides the RS/SR racemate. The N- succinoyl analog of example 60 and the N-pyrolyl congener of example 63 are derived from the corresponding benzyl ester and the N-carbobenzyloxy-protected alkylidene, respectively. Both protecting groups are removed by concomitant hydrogenolytic cleavage during olefin reduction.

All of the O,N bisacyl-E and Z isopropyl-2-azetidinones of Table V below are of the formula D-5 wherein $R_{61}$ is methyl, $R_{30}$ is hydrogen and $R_5$ and $R_3$ are as defined. The example in which the isopropylidene precursor is prepared is also specified.

In Table VI below, physical data are given for the dihydro analogs prepared in Table V.

TABLE V

| Example | Precursor Example | $R_5$ | $R_3$ |
| --- | --- | --- | --- |
| 49 | 20 | $CH_3$ | $CH_3$ |
| 50 | 23 | $CH_3$ | $(CH_2)_2CH_3$ |
| 51 | 27 | $(CH_2)_2CH_3$ | $CH_3$ |
| 52 | 28 | $(CH_2)_2CH_3$ | $(CH_2)_2CH_3$ |
| 53 | 30 | $(CH_2)_2CH_3$ | $(CH_2)_4CH_3$ |
| 54 | 31 | $(CH_2)_2CH_3$ | $(CH_2)_6CH_3$ |
| 55 | 32 | $(CH_2)_2CH_3$ | $(CH_2)_9CH_3$ |
| 56 | 29 | $(CH_2)_2CH_3$ | $CH(CH_3)_2$ |
| 57 | 22 | $(CH_2)_2CH_3$ | $(CH_2)_3$-cyclo- hexyl |
| 58 | 26 | $(CH_2)_4CH_3$ | $(CH_2)_4CH_3$ |
| 59 | 21 | $(CH_2)_5CH_3$ | $(CH_2)_5CH_3$ |
| 60 | 34 | $(CH_2)_2CH_3$ | $(CH_2)_2CO_2H$ |
| 61 | 35 | $(CH_2)_2CH_3$ | phenyl |
| 62 | 36 | $(CH_2)_2CH_3$ | $(CH_2)_3$-2-thienyl |
| 63 | 38 | $(CH_2)_2CH_3$ | L-prolyl[3] |
| 64 | 39 | $CH_3$ | $CH_3$ |
| 65 | 40 | $CH_3$ | $(CH_2)_2CH_3$ |
| 66 | 41 | $(CH_2)_2CH_3$ | $(CH_2)_2CH_3$ |
| 67 | 43 | $(CH_2)_3$-cyclohexyl | $(CH_2)_3$-cyclo- hexyl |

[3] This name is for the fragment $R_3CO$-.

TABLE VI

| Example | Physical Data[a] |
|---|---|
| 49 | Rf = 0.49 |
| 50 | 1H NMR (δ, $CDCl_3$): 4.00, 3.7-3.0, 2.62, 2.4-2.1, 2.04, 1.8-1.5, 1.1, 0.94. |
| 51 | Rf = 0.54 |
| 52 | Rf = 0.67 |
| 53 | Exact mass: 298.2021 |
| 54 | $^1H$ NMR (δ, $CDCl_3$): 4.00, 3.8-3.1, 2.66, 2.25, 1.8-1.4, 1.3, 1.1, 0.92. |
| 55 | $^1H$ NMR (δ, $CDCl_3$): 4.0, 3.82-3.02, 2.64, 2.26, 2.4-2.12, 1.76-1.48, 1.24, 1.11, 0.92, 0.86. |
| 56 | $^1H$ NMR (δ, $CDCl_3$): 4.01, 3.75-2.78, 2.4-2.2, 1.87-1.4, 1.3-1.0, 0.95. |
| 57 | Exact mass: 390.2049 |
| 58 | Exact mass: 325.2255 |
| 59 | $^1H$ NMR (δ, $CDCl_3$): 3.98, 3.84-3.00, 2.74, 2.26, 1.9-1.5, 1.3, 1.13, 0.9. |
| 60 | Exact mass: 338.0996 |
| 61 | $^1H$ NMR (δ, $CDCl_3$): 8.15-7.9, 7.6-7.3, 4.2-3.5, 3.4-3.15, 2.30, 1.8-1.4, 1.16, 0.94. |
| 62 | $^1H$ NMR (δ, $CDCl_3$): 7.09, 6.83-6.69, 4.00, 3.75-3.0, 3.0-2.64, 2.28, 2.4-2.1, 2.17-1.89, 1.88-1.48, 1.11, 0.94. |
| 63 | Rf = 0.35 |
| 64 | Exact mass: 212.0915 |
| 65 | Exact mass: 280.0926 |
| 66 | Rf = 0.64 |
| 67 | Exact mass: 433.3200 |

[a]Refer to Footnote a in Table I.

---

Example 68 [3S*(S*)]-(±)-[1-Methyl-2-(1-oxobutoxy)ethyl]-1-(1-oxopropenyl)2-azetidinone (Formula C-4: $R_{61}$ is methyl,

Example 68 [3S*(S*)]-(±)-[1-Methyl-2-(1-oxobutoxy)ethyl]-1-(1-oxopropenyl)2-azetidinone (Formula C-4: $R_{61}$ is methyl, $R_{30}$is hydrogen, $R_5$ is n-propyl and $R_3$ is ethenyl) Refer to Chart C.

Preparation of [3S*(S*)]-(±)-[1-methyl-2-(1-oxobutoxy)ethyl]-2-azetidinone: To a solution of 0.090 g of (Z)-3-[1-methyl-2-(1-oxobutoxy)ethylidene]-2-azetidinone in 1.5 ml of ethyl acetate is added 0.003 g of 10% palladium on carbon. This is stirred under hydrogen for 18 hr. TLC analysis at this time indicates only 50% conversion of starting material, so 0.003 g of 10% palladium/carbon is added and the reaction stirred under hydrogen at room temperature for 3 days. TLC analysis at this time shows complete conversion of starting material. The reaction mixture is filtered and concentrated in vacuo to give 0.045 g of a tan oil. The residue is purified by preparative TLC (1000 microns. 20 x 20 cm silica gel, 1:1 acetone, methylene chloride) to give 0.029 g of [3S*(S*)]-(±)-[1-methyl-2-(1-oxobutoxy)-ethyl]-2-azetidinone as an off-white crystalline solid.

Alternatively, a mixture of 0.012 g of (Z)-3-[1-methyl-2-(1-oxobutoxy)ethylidene]-2-azetidinone and 0.001 g of 10% palladium on carbon in 0.5 ml absolute ethanol is stirred under hydrogen at room temperature for 18.5 hr. TLC analysis indicates complete conversion of starting material. The reaction mixture is filtered and concentrated in vacuo to give 0.004 g of [3S*(S*)]-(±)-[1-methyl-2-(1-oxobutoxy)-ethyl]-2-azetidinone as a colorless oil.

To a solution of 0.003 g of [3S*(S*)]-(±)-[1-methyl-2-(1-oxobutoxy)ethyl]-2-azetidinone and 15 ml of triethylamine in 1.0 ml methylene chloride at 20°C is added 42 ml (approximately 22-fold excess) of 3-chloropropionylchloride (prepared from 3-chloropropionic acid by refluxing in thionyl chloride) over 5 sec. TLC analysis after stirring the mixture for 2,5 hr. indicates complete conversion of starting material. The mixture is concentrated in vacuo, and the residue purified by preparative TLC (1000 microns, 20 x 20 cm silica gel, 1:1 ethyl acetate/hexane) to give 4.2 mg of the title product as a yellow oil.

Physical characteristics are as follows:

$^1H$ NMR (δ, $CDCl_3$): 7.1-6.4, 5.9-5.8, 4.01, 3.7-3.0, 2.28, 2.4-2.1, 1.62, 1.15, 0.94.

TLC (1:1 ethyl acetate/hexane, UV): Rf = 0.58.

Example 69 1-(6-Amino-1-oxohexyl)-3-(1-methylethyl)-2-azetidinone

To a solution of 0.8 g [Z]-1-[6-(N-carbobenzyloxy)amino-1-oxohexyl]-3-[1-methyl-2-(1-oxobutoxy)ethylidene]-2-azetidinone in 10 ml ethyl acetate, is added 0.5 g palladium black. This is stirred under hydrogen for 18.5 hr at which time TLC indicates complete conversion of starting material. The reaction mixture is filtered, and the filtrate concentrated in vacuo to give 0.620 g of a yellow, cloudy viscous oil. $^1H$ NMR of the crude mixture indicates that hydrogenolysis of the allylic ester has predominatly taken place to give the title product.

Physical characteristics are as follows:

$^1H$ NMR (δ, $CDCl_3$): 3.58, 3.15-3.38, 2.66, 1.07, 0.98.

TLC (1:1 ethyl acetate/hexane, p-anisaldehyde): Rf = 0, white.

Example 70 (±)-3-(1-Methylethyl)-1-(1-oxo-6-(trimethylamino)hexyl)-2-azetidinone iodide

To a solution of 0.052 g of 1-(6-amino-1-oxohexyl)-3-(1-methylethyl)-2-azetidinone in 4 ml of tetrahydrofuran is added 0.033 ml of methyl iodide. The mixture is stirred at 20°C for 18 hr, the volatiles removed in vacuo. The residue is soluble in deuterium oxide. $^1H$ NMR indicates peaks corresponding to those predicted for the title product. Lyophilization gives 0.048 g of a yellow solid which is tentatively identified as the title product.

Examples 71 - 73 (Refer to Chart B).

Synthesis of the N-acyloxyalkyl analogs: Treatment of 3-(1-methylethylidene-2-azetidinone of formula B-1 (A-7) (Preparation 5) with an alkyl chloroformate in the presence of triethylamine in methylene chloride or tetrahydrofuran at 0-20°C gives the N-acyloxy-alkyl analogs shown in Table VII below. Excess triethylamine and chloroformate may be required in multiple portions in order to effect complete conversion.

Synthesis of the N-acylamino analogs: N-1 acylamino-substituted analogs of Table VII below are prepared by treatment of 3-(1-methylethylidene-2-azetidinone of formula B-1 (A-7) (Preparation 5) with an alkylisocyanate or chlorosulfonyl isocyanate.

All of the compounds of Table VII below are of the formula B-4 wherein $R_{60}$ is methyl, $R_{61}$ is methyl and $R_3$ is as defined. The appropriate reactant for the synthesis is also specified.

TABLE VII

| Example | Reactant | $R_3$ | Physical Data[a] |
|---|---|---|---|
| 71 | butyl chloroformate | $O(CH_2)_3CH_3$ | Rf = 0.67 |
| 72 | isobutyl chloroformate | $OCH_2CH(CH_3)_2$ | Exact mass: 211.1190 |
| 73 | chlorosulfonyl-isocyanate | $NH_2$ | Exact mass: 154.0731 |

[a]Refer to Footnote a in Table I.

Example 74 3-(1-Methylethylidene)-2-oxo-1-azetidine-N-(n-butyl)carboxamide (Formula B-4: $R_{61}$ is methyl, $R_{60}$ is methyl and $R_3$ is n-butyl-amino) Refer to Chart B.

To a solution of 3-(1-methylethylidene-2-azetidinone (105 mg) in 10 ml of dry toluene under argon is added n-butylisocyanate (97 mg), and the mixture heated to reflux. At 24 hr, 48 hr, and 72 hr additional n-butylisocyanate (97 mg) is added. At 96 hr the reaction mixture is concentrated in vacuo. The residue is purified by chromatography on an Analtech 2,000 micron silica gel plate (eluted with 30% ethyl acetate/hexane) to give 73 mg of 3-(1-methylethylidene)-2-oxo-1-azetidine-N-(n-butyl)carboxamide (Rf = 0.23) as a white crystalline solid.

Physical characteristics of 3-(1-methylethylidene)-2-oxo-1-azetidine-N-(n-butyl)carboxamide are as follows:

$^1H$ NMR (δ, $CDCl_3$): 6.55, 4.02, 3.30, 2.12, 1.83, 1.73-1.13, 0.93:

IR ($cm^{-1}$, chloroform): 3370, 1741, 1683, 1535, 1321, 1131; TLC (30% ethyl acetate/hexane, p-anisaldehyde): Rf = 0.23, blue; mass spectrum (m/e,): 210, 167, 138, 67; exact mass found: 210.1352.

Example 75 3-(1-Methylethylidene)-2-oxo-1-azetidine-N-methyl-N-(n-butyl)carboxamide (Formula B-4: $R_{61}$ is methyl, $R_{60}$ is methyl, $R_3$ is $CH_3(CH_2)_3N(CH_3)$-) Refer to Chart B.

To a solution of 3-(1-methylethylidene)-2-oxo-1-azetidine-N-(n-butyl)carboxamide (17.8 mg) and sodium hydride (4 mg of a 50% oil dispersion) in 5 ml of freshly distilled tetrahydrofuran at 0° C under 1 atm of argon, 6 ml of methyl iodide (13.7 mg) is added. After 2 hrs 15 ml of methyl iodide is added and the reaction mixture is allowed to stir for 19 hr. Thin layer silica gel chromatography in 30% ethyl acetate/hexane shows four zones, Rf: 0.0, 0.06, 0.24 and 0.28. After removal of the solvents in vacuo, the residue is dissolved in 10 ml of methylene chloride and washed with 10 ml of water and with 10 ml of saturated aqueous sodium chloride. The organic layer is dried with magnesium sulfate, and concentrated in vacuo to give 22 mg of the title product as a colorless grainy oil.

Examples 76 - 78 (Refer to Chart B).

General procedure for the hydrogenation of the N-acyloxyalkyl analogs: A mixture of the precursor isopropylidene (e.g., 25 mg) of formula B-4 and 5 mg of 10% palladium/carbon or palladium black in 5 ml of ethyl acetate is alternately evacuated and filled with hydrogen gas from a balloon four times. The mixture is then stirred at 20° C for 2-24 hrs, until the absence of starting material is indicated by TLC. The mixture is filtered through a short plug of Celite, the pad washed with 1 ml of ethyl acetate, and the filtrate concentrated in vacuo to produce the 3-isopropyl analogs of formula B-3, as listed in Table VIII below. The product is homogenous by TLC and $^1H$ NMR, and does not require subsequent purification.

All of the dihydro analogs of Table VIII below are of the formula B-3 wherein $R_{60}$ is methyl, $R_{61}$ is methyl and $R_3$ is as defined. The example in which the precursor isopropylidene is prepared is also specified.

TABLE VIII

| Example | Precursor Example | $R_3$ | Physical Data |
|---|---|---|---|
| 76 | 71 | $O(CH_2)_3CH_3$ | $^1H$ NMR ($\delta$, $CDCl_3$): 4.21, 3.64, 3.33, 3.02, 2.02, 1.8-1.2, 1.10, 1.00. |
| 77 | 74 | $NH(CH_2)_3CH_3$ | Exact mass: 212.1525 |
| 78 | Deleted | --- | --- |

Examples 79 - 80 (Refer to Chart C).

Synthesis of the O-acyl, N-acyloxyalkyl analogs : Treatment of (Z)-3-[1-methyl-2-(1-oxobutoxy)ethylidene]-2-azetidinone of formula D-3 (Preparation 14) with an alkyl chloroformate in the presence of triethylamine in methylene chloride or tetrahydrofuran at 0-20°C gives the O-acyl, N-acyloxyalkyl analogs shown in Table IX below. Excess triethylamine and chloroformate may be required in multiple portions in order to effect complete conversion.

All of the compounds of Table IX below are of the formula D-2 wherein $R_{61}$ is methyl, $R_{30}$ is hydrogen and $R_5$ and $R_3$ are as defined. The reactant for synthesis is also specified.

In Table X below, physical data are given for the compounds prepared in Table IX.

TABLE IX

| Example | Reactant | $R_5$ | $R_3$ |
|---|---|---|---|
| 79 | butyl chloroformate | $(CH_2)_2CH_3$ | $O(CH_2)_3CH_3$ |
| 80 | isobutyl chloroformate | $(CH_2)_2CH_3$ | $OCH_2CH(CH_3)_2$ |

TABLE X

| Example | Physical Data[a] |
|---|---|
| 79 | Rf = 0.68 |
| 80 | Exact mass: 297.1515 |

[a]Refer to Footnote a in Table I.

Examples 81 - 82 (Refer to Chart D).

General procedure for the hydrogenation of the O-acyl, N-acyloxyalkyl analogs: A mixture of the precursor isopropylidene (e.g.. 25 mg) of formula D-2 and 5 mg of 10% palladium/carbon or palladium black in 5 ml of ethyl acetate is alternately evacuated and filled with hydrogen gas from a balloon four times. The mixture is then stirred at 20°C for 2-24 hrs. until the absence of starting material is indicated by TLC. The mixture is filtered through a short plug of Celite, the pad washed with 1 ml of ethyl acetate and the filtrate concentrated in vacuo to produe the 3-isopropyl analogs of formula D-5, as listed in Table XI below. The product is

homogenous by TLC and $^1H$ NMR, and does not require subsequent purification.

All of the dihydro analogs of Table XI below are of the formula D-5 wherein $R_{61}$ is methyl, $R_{30}$ is hydrogen and $R_5$ and $R_3$ are as defined. The example in which the precursor isopropylidene is prepared is also specified.

In Table XII below, physical data are given for the compounds prepared in Table XI.

TABLE XI

| Example | Precursor Example | $R_5$ | $R_3$ |
|---|---|---|---|
| 81 | 79 | $(CH_2)_2CH_3$ | $O(CH_2)_3CH_3$ |
| 82 | 80 | $(CH_2)_2CH_3$ | $OCH_2CH(CH_3)_2$ |

TABLE XII

| Example | Physical Data[a] |
|---|---|
| 81 | $^1H$ NMR (δ, $CDCl_3$): 4.24, 4.07, 3.95-2.94, 2.22, 2.00-1.27, 1.16, 0.97. |
| 82 | Rf = 0.58 |

[a]Refer to Footnote a in Table I.

Preparation 15 3-[Z]-(1-(Methanesulfonyloxy-methyl)ethylidene)-2-azetidinone (Formula E-2: $R_{61}$ is methyl, $R_{30}$ is hydrogen, $R_{40}$ is methyl) Refer to Chart E.

To a solution of 170 mg of 3-[Z]-(1-hydroxymethylethylidene)-2-azetidinone and 176 mg of triethylamine in 14 ml of freshly distilled tetrahydrofuran at 0° C is added dropwise 184 mg of methanesulfonyl chloride. The solution turns cloudy. After 30 minutes, TLC (ethyl acetate) of the reaction mixture shows a single zone, Rf 0.4, but no starting material, Rf 0.2. To one-fourth of the reaction mixture, 22 mg of methanol is added. After 2 days, TLC shows no indication of the reaction proceeding. Solvents are removed in vacuo. NMR of the residue shows only the intermediate mesylate. The mesylate is dissolved in 2 ml of methanol. After 1 week, the mesylate is still the major zone by TLC. The reaction mixture is concentrated in vacuo and the residue purified by Prep TLC on a 1000 microns silica gel plate (eluted with ethyl acetate) to give 23 mg of the intermediate mesylate.

Physical characteristics are as follows:

$^1H$ NMR (δ, $CDCl_3$): 6.3, 5.07, 3.6, 3.07, 1.87.

Preparation 16 (Z)-3-(1-(Azidomethyl)ethylidene)-2-azetidinone (Formula E-3: $R_{61}$ is methyl, $R_{30}$ is hydrogen) Refer to Chart E.

To a solution of 0.050 g of 3-[Z]-(1-hydroxymethylethylidene)-2-azetidinone in 4 ml freshly distilled tetrahydrofuran is added 85 ml of triethylamine. The mixture is cooled to 0° C under argon atmosphere, and 36 ml of methansulfonyl chloride added dropwise over 2 min. The mixture is stirred at 0° C for 30 min. TLC analysis shows a new spot has formed (Rf = 0.35, ethyl acetate). The white solid formed is removed by filtration, and the solid washed with 1 ml of tetrahydrofuran. The filtrate is cooled to 0° C, then with stirring is added 0.050 g of sodium azide and 5 ml of water. The mixture is stirred at 20° C for 2 hr, then an additional 95 ml of water added. After 20 hrs, the white solid formed is removed by filtration, and the filtrate concentrated in vacuo to give 0.058 g of the title product as a slightly off-white crystalline solid.

Physical characteristics are as follows:

$^1H$ NMR (δ, $CDCl_3$): 6.7, 4.25, 3.82, 1.85.

IR ($cm^{-1}$, chloroform): 3436, 2105, 1744, 1439, 1343.

Example 83 (Z)-3-(1-(azidomethyl)ethylidene)-1-(1-oxohexyl)-2-azetidinone (Formula E-4: $R_{61}$ is methyl, $R_3$ is n-pentyl, $R_{30}$ is hydrogen) Refer to Chart E.

A mixture of 0.058 g of (Z)-3-(1-azidomethylethylidene)-2-azetidinone, 108 ml of hexanoic anhydride, and 0.006 g of dimethylaminopyridine in 1 ml of pyridine is stirred at 20° C for 17 hrs. The mixture is concentrated in

vacuo to give 0.159 g of a yellow oil, which is immediately purified on silica gel (1000 microns plate, 20 x 20 cm, 1:1 ethyl acetate/hexane) to give 0.096 g of a light yellow oil, which contains hexanoic acid. After approximately 4 days at 0.1 Torr, 0.060 g of pure title product is obtained.

Physical characteristics are as follows:

$^1H$ NMR (δ, $CDCl_3$): 4.23, 4.01, 2.70, 1.89, 1.8-1.5, 1.5-1.2, 0.90.

IR ($cm^{-1}$, chloroform): 2101, 1772, 1720, 1690, 1379, 1332, 1303.

TLC (ethyl acetate, p-anisaldehyde): Rf = 0.75, yellow.

Mass spectrum (m/e): 250, 222, 208, 194, 166, 124, 109, 99, 71, 43; exact mass found: 250.1421.

Example 84 [3S*(S*)-(±)-(1-Methyl-2-acetamidoethyl)-1-(1-oxohexyl)-2-azetidinone (Formula E-5: $R_{61}$ is methyl, $R_8$ is methyl, $R_{30}$ is hydrogen and $R_3$ is n-pentyl) Refer to Chart E.

A mixture of 0.011 g of (Z)-3-(1-azidomethylethylidene)-1-(1-oxohexyl)-2-azetidinone and 0.004 g of 10% palladium/carbon in 2.0 ml of acetic anhydride is stirred under 1 atm of hydrogen (balloon) for 4 hr at 20° C. The mixture is filtered through Celite, the pad washed with ethyl acetate, and the filtrate concentrated in vacuo to give 0.011 g of the title product as a colorless oil.

Physical characteristics are as follows:

$^1H$ NMR (δ, $CDCl_3$): 5.98, 3.67, 3.32, 2.67, 2.3-2.0, 2.00, 1.8-1.4, 1.35-1.15, 1.05, 0.89.

IR ($cm^{-1}$, chloroform): ~3400, 3450, 1778, ~1710, 1370, ~1230.

Mass spectrum (m/e): 268, 240, 226, 212, 182, 170, 140, 127, 99, 68, 43; exact mass found: 268.1786.

TLC (100% ethyl acetate): Rf = 0.26 white (p-anisaldehyde; contaminants UV active at 0.44 and 0.64. Material tested without further purification.

Example 85

(Z)-3-[1-[1-[4,5-bis(methoxycarbonyl)-1,2,3-triazolyl]]-methylethylidene)-1-(1-oxohexyl)-2-azetidinone (Formula E-6: $R_{61}$ is methyl, $R_3$ is n-pentyl, $R_6$ is methoxycarbonyl, $R_7$ is methoxycarbonyl) Refer to Chart E.

A mixture of 0.010 g of (Z)-3-(1-azidomethylethylidene)-1-(1-oxohexyl)-2-azetidinone and 0.0085 g of dimethylacetylene dicarboxylate in 2.0 ml of tetrahydrofuran is stirred at 20° C for 22 hrs. TLC analysis shows much starting material. Then an additional 0.1 ml (10-15 times excess) of dimethylacetylene dicarboxylate is added, and the mixture allowed to stir 7 days. (Most of the solvent evaporates). The residue is chromatographed on silica gel 20 x 20 cm plate, 1000 microns, in 1:1 ethyl acetate/hexane to give the title product (0.016 g) as a colorless oil, Rf = 0.35.

Physical characteristics are as follows:

$^1H$ NMR (δ, $CDCl_3$): 5.72, 4.08, 4.00, 2.74, 1.70, 1.4-1.2, 0.90.

IR ($cm^{-1}$, chloroform): 1773, 1735, 1696.

Example 86

[3S*(S*)]-(±)-[1-[1-[4,5-bis(methoxycarbonyl)-1,2,3-triazolyl]]methylethyl]-1-)1-oxohexyl)-2-azetidinone (Formula E-7: $R_{61}$ is methyl, $R_3$ is n-pentyl, $R_6$ is methoxycarbonyl, $R_7$ is methoxycarbonyl) Refer to Chart E.

A mixture of 0.016 g of (Z)-3-[1-[1-[4,5-bis(methoxycarbonyl)-1,2,3-triazolyl]methylethylidene]-1-(1-oxohexyl)-2-azetidinone and 0.003 g of 10% palladium/cabon in 4 ml of ethyl acetate is stirred under 1 atm of hydrogen (balloon) for 22 hr at 20° C. The mixture is filtered through Celite, the pad washed with 2 ml of ethyl acetate, and the filtrate concentrated in vacuo to give 0.013 g of the title product as a colorless oil.

Physical characteristics are as follows:

$^1H$ NMR (δ, $CDCl_3$): 4.60, 4.01, 3.98, 3.85-3.43, 3.19, 2.67, 2.0-1.2, 1.13, 0.89.

IR ($cm^{-1}$, chloroform): 1782, 1732, 1700.

Mass spectrum (m/e): 394, 363, 338, 295, 253, 237, 211, 186; exact mass found: 394.1841.

Preparation 17 Deleted.

Preparation 18 (Z)-3-(1-Fluoromethylethylidene)-2-azetidinone (Formula G-2: $R_{61}$ is methyl, $R_{70}$ is F, $R_{30}$ is hydrogen) Refer to Chart G.

A slurry of 54 mg of 3-[Z]-(1-(hydroxymethyl)ethylidene)-2-azetidinone in 2 ml methylene chloride under argon atmosphere, is cooled to -78° C and 0.061 ml of diethylaminosulfurtrifluoride is added. After 2.5 hr the reaction mixture is allowed to warm to -30° C. After an additional 3 hr at -30° C, a mixture of 1 ml saturated aqueous sodium bicarbonate and 1 ml water is added dropwise. The precipitate that forms is filtered away, the layers separated, and the aqueous layer washed with methylene chloride. The combined methylene chloride layers are dried with magnesium sulfate, filtered, and concentrated in vacuo to give a yellow brown oil. The crude residue is purified via preparative TLC (Taper plate, 1:1 ethyl acetate/hexane) to give 9 mg of the title product as a yellow crystalline solid.

Physical characteristics are as follows:

$^1H$ NMR (δ, $CDCl_3$): 6.07, 5.22, 3.78, 1.86.

IR ($cm^{-1}$, chloroform): 3681, 1755, 1602, 1132, 1101, 999.

TLC (1:1 ethyl acetate/hexane, p-anisaldehyde): Rf = 0.23, yellow.

Mass spectrum (m/e): 129, 110, 100, 86, 85, 67; exact mass found: 129.0581.

Example 87 (Z)-3-(1-(Fluoromethyl)ethylidene)-1-(1-oxohexyl)-2-azetidinone (Formula G-5: $R_{61}$ is methyl, $R_{30}$ is hydrogen, $R_{70}$ is F, $R_3$ is n-pentyl) Refer to Chart G.

To a mixture of 8 mg of (Z)-3-(1-fluoromethylethylidene)-2-azetidinone and 10 ml of triethylamine in 0.5 ml of methylene chloride is added 10.1 ml of hexanoyl chloride. After stirring at 20° C for 18.5 hr, the reaction is concentrated and purified via preparative TLC (20 by 20 cm Taper plate, 30% ethyl acetate/hexane) to give 6 mg of the title product as a clear colorless oil which crystallizes upon storage at 0° C.

Physical characteristics are as follows:

$^1H$ NMR (δ, $CDCl_3$): 5.2, 4.0, 2.70, 1.93, 1.9-1.1, 0.90.

TLC (30% ethyl acetate/hexane, UV): Rf = 0.57.

Example 88 [3S*(S*)]-(±)-(1-Methyl-2-fluoroethyl)-1-(1-oxohexyl)-2-azetidinone (Formula G-4: $R_{61}$ is methyl, $R_{30}$ is hydrogen, $R_{70}$ is F, $R_3$ is n-pentyl) Refer to Chart G.

A mixture of 0.006 g of (Z)-3-(1-fluoromethylethylidene)-1-(1-oxohexyl)-2-azetidinone and 0.010 g of 10% palladium on carbon in 0.5 ml of ethyl acetate is stirred under 1 atm of hydrogen for 20 hr (balloon).As the solvent evaporates, an additional 1.2 ml of ethyl acetate is added, the mixture stirred 3 hr at 20° C under 1 atm of hydrogen, then filtered through Celite. The filtrate is concentrated in vacuo to give 4 mg residue. The material is tested without attempted purification.

Physical characteristics are as follows:

$^1H$ NMR (δ, $CDCl_3$): 4.05, 3.8-3.0, 2.66, 2.3, 1.7-1.2, 1.2-1.0, 0.89.

Preparation 19 3-[E]-1-Methyl-2-(tetrahydropyranylhydroxy)-ethylidene]-2-azetidinone

To a mixture of 0.021 g of e-[E]-(1-hydroxymethyl)ethylidene)-2-azetidinone and 18 ml (17 mg) of dihydropyran in 1 ml of methylene chloride is added 2 mg of p-toluenesulfonic acid. After 8 min at 20° C, a TLC (1:1 ethyl acetate/hexane) shows three major new spots (Rf = 0.19, 0.28, and 0.34). After a total of 50 min 5 ml of triethylamine is added, and the mixture concentrated in vacuo to give a residue which is purified by preparative TLC (20 cm x 20 cm 300 microns - 1000 microns Taper plate, 1:1 ethyl acetate/hexane), to give the title product, Rf = 0.19 (0.014 g).

Physical characteristics are as follows:

$^1H$ NMR (δ, $CDCl_3$): 6.12, 4.62, 4.25, 3.94, 3.9-3.75, 3.55, 2.04, 2.0-1.5.

$^{13}C$ NMR ($CDCl_3$): 98.1, 68.6, 62.0, 44.4, 30.4, 25.4, 19.2, 15.1.

IR ($cm^{-1}$, $CDCl_3$): 3440, 1739, 1440, 1342, 1129, 1032.

Example 89 3-[E]-[1-Methyl-2-(tetrahydropyranylhydroxy)ethylidene]-1-(1-oxohexyl)-2-azetidinone.

A mixture of 0.013 g of 3-[E]-[1-methyl-2-(tetrahydropyranylhydroxy)ethylidene]-2-azetidinone, 21 ml of hexanoic anhydride, and 2 mg of dimethylaminopyridine in 1 ml of methylene chloride is stirred at 20° C for 16 hr. TLC shows little conversion. The solvent is evaporated under an argon stream, and 1 ml of pyridine added. After 3 days, another 21 ml of anhydride is added and an additional 5 mg of DMAP. After an additional 2 days, 4 ml each of methylene chloride and saturated aqueous ammonium chloride is added; the layers separated, and the aqueous layer extracted two times with 5 ml of methylene chloride. The combined organic layers are dried with magnesium sulfate, and concentrated in vacuo to give 0.02 g of a yellow solid. The residue is purified by chromatography on silica gel (40-63 micron, 12.5 cm x 0.5 cm, 1:1 ethyl acetate/hexane) to give 0.013 g of the title compound.

Physical characteristics are as follows:

$^1H$ NMR (δ, $CDCl_3$); 4.58, 4.30, 4.12, 3.97, 3.8-3.4, 2.70, 2.08, 1.64, 1.35, 0.88.

IR ($cm^{-1}$, $CDCl_3$): 1768, 1721, 1683.

TLC (1:1 ethyl acetate/hexane): Rf = 0.63.

Mass spectrum (m/e): 309, 253, 225, 207, 169, 85.

Example 90 3-[E]-[1-Methyl-2-(hydroxy)ethylidene]-1-[1-oxohexyl]-2-azetidinone.

To a solution of 0.009 g of 3-[E]-[1-methyl-2-(tetrahydropyranylhydroxy)ethylidene]-1-(1-oxohexyl)-2-azetidinone in 2 ml of absolute methanol is added 3 mg of p-toluenesulfonic acid. The reaction is monitored by TLC and is complete after 105 min. There is observed a clean conversion of the spot at Rf = 0.63 (starting material) to give the product spot at Rf = 0.28 (1:1 ethyl acetate/hexane).

The mixture is concentrated in vacuo, then immediately flash chromatographed (40-63 micron silica gel, on a small column 0.5 mm diameter, 1:1 ethyl acetate/hexane) to give 1.6 mg of the title compound as a white solid.

Physical characteristics are as follows:

$^1H$ NMR (δ, $CDCl_3$): 4.22, 4.13, 2.70, 2.05, 1.66, 1.23, 0.89.

Example 91 N-Hexanoyl-4-tert-butyl-2-azetidinone (Formula L-4: $R_{101}$ is tert-butyl; $R_3$ is n-pentyl) Refer to Chart L.

A mixture of 0.334 g of 4-tert-butyl azetidinone, 0.321 g of 4-dimethylaminopyridine, and 0.61 ml of hexanoic anhydride in 2.0 ml of pyridine is stirred at 20° C for 17 days. The volatiles are removed at high vacuum (0.1 Torr), and the residue purified by medium pressure liquid chromatography on silica gel 60-200μ (10% ethyl acetate/hexane) to give 0.336 g of the title product as a colorless oil.

Physical characteristics are as follows:

TLC (10% ethyl acetate/hexane) Rf=0.21 (stains white with p-anisaldehyde stain).
$^1H$ NMR (δ, 1 $CDCl_3$): 3.97, 2.8, 2.7, 1.8-1.2, 1.04, 0.90.
IR ($cm^{-1}$, film): 1788.9, 1706, 1289.
Mass spectrum (m/e, real abundance): 225, 182, 169, 142, 129, 111, 99, 43.
Exact mass found: 225.1738.

Example 92 N-Acetyl-cis-3,4-trimethylene-2-azetidinone (Formula J-3: n is 3; $R_3$ is methyl) Refer to Chart J.

A mixture of 2.575 g of cis-3,4-trimethylene-2-azetidinone, 6 ml of acetic anhydride, and 0.368 g of 4-dimethylaminopyridine in 10 ml of pyridine is stirred at 20° C for 24 hr, then the volatile components removed at 0.1 Torr. The residue is purified by medium pressure liquid chromatography on 40-63μ silica gel (20 cm x 4.5 cm column, 70% ethyl acetate/hexane) to give 2.238 g of the title product as a light yellow liquid.

Physical characteristics are as follows:
$R_f$=0.55 (100% ethyl acetate, stains white p-anisaldehyde).
$^1H$ NMR (δ, $CDCl_3$): 4.40. 3.52, 2.36, 2.3-1.3.
IR ($cm^{-1}$, film): 1778, 1706, 1377, 1340, 1317.
Mass spectrum (m/e, rel abundance): 153, 125, 86, 68.
Exact mass found: 153.0786.

Example 93 N-Hexanoyl-cis-3,4-trimethylene-2-azetidinone. (Formula J-3: n is 3; $R_3$ is n-pentyl) Refer to Chart J.

A mixture of 10.366 g of cis-3,4-trimethylene-2-azetidinone, 1.06 g of 4-dimethylaminopyridine, and 21.4 ml of hexanoic anhydride in 75 ml of pyridine is stirred at 20° C for 5 days. The volatile components are removed at 0.1 Torr, and the residue purified by medium pressure liquid chromatography on 60-200μ silica gel (5.5 cm x 31 cm column, eluting with 2L of 7.5% ethyl acetate/hexane, followed by 2L of 10% ethyl acetate/hexane, and finally 100% ethyl acetate) to give 17.07 g of the title product as a colorless oil.

Physical characteristics are as follows:
TLC (10% ethyl acetate/hexane): $R_f$=0.19, white on staining with p-anisaldehyde.
$^1H$ NMR (δ, $CDCl_3$); 4.37, 3.48, 2.64, 2.4-1.2, 0.87.
IR ($cm^{-1}$, film): 1781, 1700, 1388, 1336, 1307, 1192.
Mass spectrum (m/e, rel abundance): 209, 193, 166, 153, 142, 68.
Exact mass found: 209.1398.

Example 94 N-Hexanoyl-cis-3,4-tetramethylene-2-azetidinone (Formula J-3: n is 4; $R_3$ is n-pentyl) Refer to Chart J.

A mixture of 1.742 g of cis-3,4-tetramethylene-2-azetidinone, 0.85 g of 4-dimethylamine pyridine, and 3.2 ml of hexanoic anhydride in 10 ml of pyridine is stirred at 20° C for 5 days. The voltaile components are removed at 0.1 Torr, and the residue is purified by chromatography on silica gel 60-200μ (21 cm x 4.5 cm, 7.5-10% ethyl acetate/hexane) to give 2.493 g of the title product as a colorless liquid.

Physical characteristics are as follows:
TLC (10% ethyl acetate/hexane): $R_f$=0.2, stain white with p-anisaldehyde.
$^1H$ NMR (δ, $CDCl_3$): 4.17, 3.27, 2.69, 2.2-1.2, 0.88.
IR ($cm^{-1}$, film): 1782, 1700, 1388, 1306.
Mass spectrum (m/e, rel abundance): 223, 190, 167, 142, 127, 82.
Exact mass found: 223.1564.

TABLE XIII

Zones of Inhibition (mm) at 1 mg/ml

| Organisms | Examples 11 | 12 | 13 | 14 | 15 | 19 |
|---|---|---|---|---|---|---|
| | (12.7 mm discs) | | | | | |
| B. fragilis | >86(86)[b] | 140 | 166(126) | 154 | 148(128) | 57(45) |
| C. perfringens | 57(32) | 81RC[a] | 76RC(36RC) | 105RC | 71(54) | 23H(16) |
| B. subtilis | 36(0) | 49* | 63(24H) | 60* | 43(0) | 0 |
| B. subtilis ME | 0 | 0 | 0 | 0 | 117H(0) | 0 |
| S. aureus UC80 | 15H[d](0) | 28 | 44(17H) | 40H | 28(0) | 0 |
| S. aureus UC 3665 | 0 | 24* | 62(31) | 44* | 36(24) | 15H(0) |
| S. lutea | 43(23) | 33* | 46H(29H) | 70H | 34H(20) | 15(0) |
| S. lutea UC 3383 | 44(27) | 37* | 58H(35H) | 44 | 52(25) | 16(0) |
| S. lutea sens | 15(0) | 40* | 60(38) | 64 | 56(38) | 17(0) |
| M. avium | 0 | 0 | 0 | 29 | 33(0) | 15(0) |
| P. oxalicum | 0 | 0 | 0 | 18H | 0 | 18(0) |
| S. pyogenes | 0 | 0 | 0 | 0 | 15(0) | 21(0) |
| | (6.35 mm discs) | | | | | |
| S. aureus UC 6685 | Tr[c](0) | 30* | 70*(Tr) | 77* | 48(10) | 15(Tr) |
| S. epidermidis | 8(0) | 11 | 53(9) | 67* | 46(9) | 11(7) |
| S. pneumoniae | 0 | 13H | 0 | Tr | 8(0) | 0 |

*Averaged dimensions of an oblong zone size.
[a]RC=resistant colonies within the zones; [b]values in parentheses are at 0.1 mg/ml; [c]Tr=Trace; [d]H=hazy zone; [e]tested at 2 mg/ml.

TABLE XIV

Zones of Inhibition (mm) at 1 mg/ml

Examples

(12.7 mm discs)

| Organisms | 16 | 69 | 70[e] | 17 | 18 |
|---|---|---|---|---|---|
| B. fragilis | 61(51) | 33 | 34 | 63(42) | 40 |
| C. perfringens | 43RC(35RC) | 15 | 15 | 52RC(35RC) | 15 |
| B. subtilis | 29(15) | 15 | 17 | 26(0) | 0 |
| B. subtilis ME | 15(0) | 14 | 17 | 0 | 0 |
| S. aureus UC80 | 22(18) | 0 | 0 | 27(0) | 18H |
| S. aureus UC 3665 | 26(22) | 0 | 0 | 27(0) | 18H |
| S. lutea | 26(20) | 15H | 0 | 28H(15H) | 27 |
| S. lutea UC 3383 | 32(22) | 15H | 15H | 33(18) | 29 |
| S. lutea sens | 36(27) | 21 | 26 | 30(Tr) | 0 |
| M. avium | 22(0) | 15 | 15 | 0 | 0 |
| P. oxalicum | 18(0) | 0 | 0 | 16H(0) | 15H |
| Rhodo. sphaeroides | 0 | 0 | 15H | 17(0) | 0 |
| S. pyogenes | 0 | 16 | 18 | 0 | 0 |
| S. pastorianus | 0 | 0 | 0 | 0 | 16H |
| (6.35 mm discs) | | | | | |
| S. aureus UC 6685 | 29(21) | 0 | 0 | 22(0) | 0 |
| S. epidermidis | 26(17) | 0 | 0 | 23(0) | 13H |
| S. pneumoniae | 15(0) | Tr | 0 | 0 | 0 |

*Averaged dimensions of an oblong zone size.
[a]RC=resistant colonies within the zone; [b]values in parentheses are at 0.1 mg/ml; [c]Tr=Trace; [d]H=hazy zone; [e]tested at 2 mg/ml.

TABLE XV

Zones of Inhibition (mm) at 1 mg/ml

| Organisms | Examples 94 | 93 | 92 | 91 |
|---|---|---|---|---|
| B. fragilis | 74 | 73 | 0 | 50 |
| C. perfringens | 65 | 52 | 26H | 50H |
| S. aureus UC 6685* | 13 | 0 | 0 | 0 |
| S. epidermidis* | 0 | 13 | 0 | 0 |
| H. influenzae* | 13 | 0 | 0 | 0 |
| N. gonorrhoeae* | 11H† | 0 | 0 | 0 |
| P. oxalicum | 20 | 19 | 0 | 0 |
| S. pyogenes | 16 | 0 | 0 | 0 |
| S. lutea UC 130 | 25 | 0 | 0 | 0 |
| S. lutea UC 3383 | 22 | 0 | 0 | 0 |
| S. lutea UC 130 sens | 29 | 0 | 0 | 0 |
| S. aureus UC 3665 | 0 | 0 | 23 | 0 |

*6mm discs; all others 12.7 mm discs.
†H = Hazy zone.

FORMULA CHART

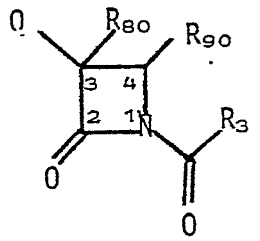

I

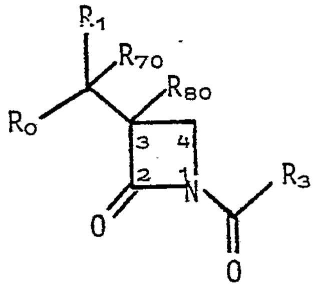

Ia

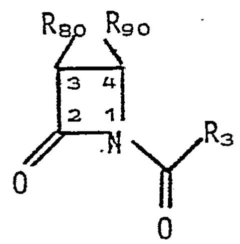

Ib

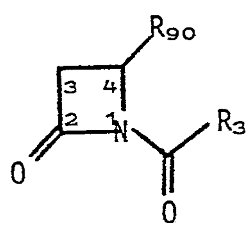

Ic

FORMULA CHART (continued)

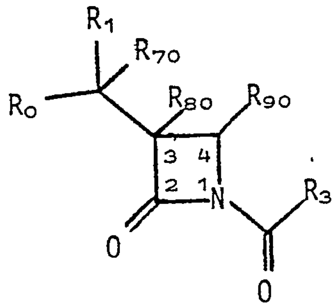

Id

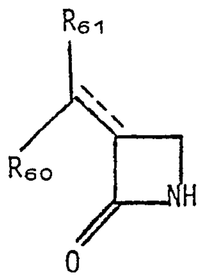

II

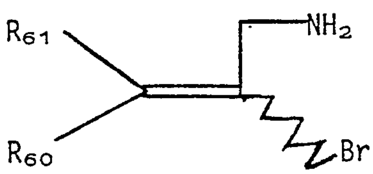

III

CHART A

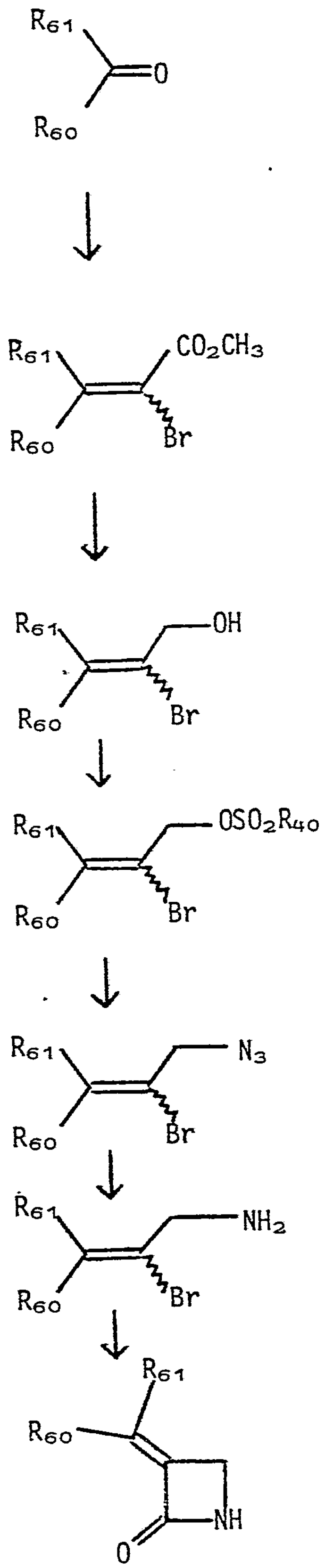

A-1

A-2

A-3

A-4

A-5

A-6

A-7

CHART B

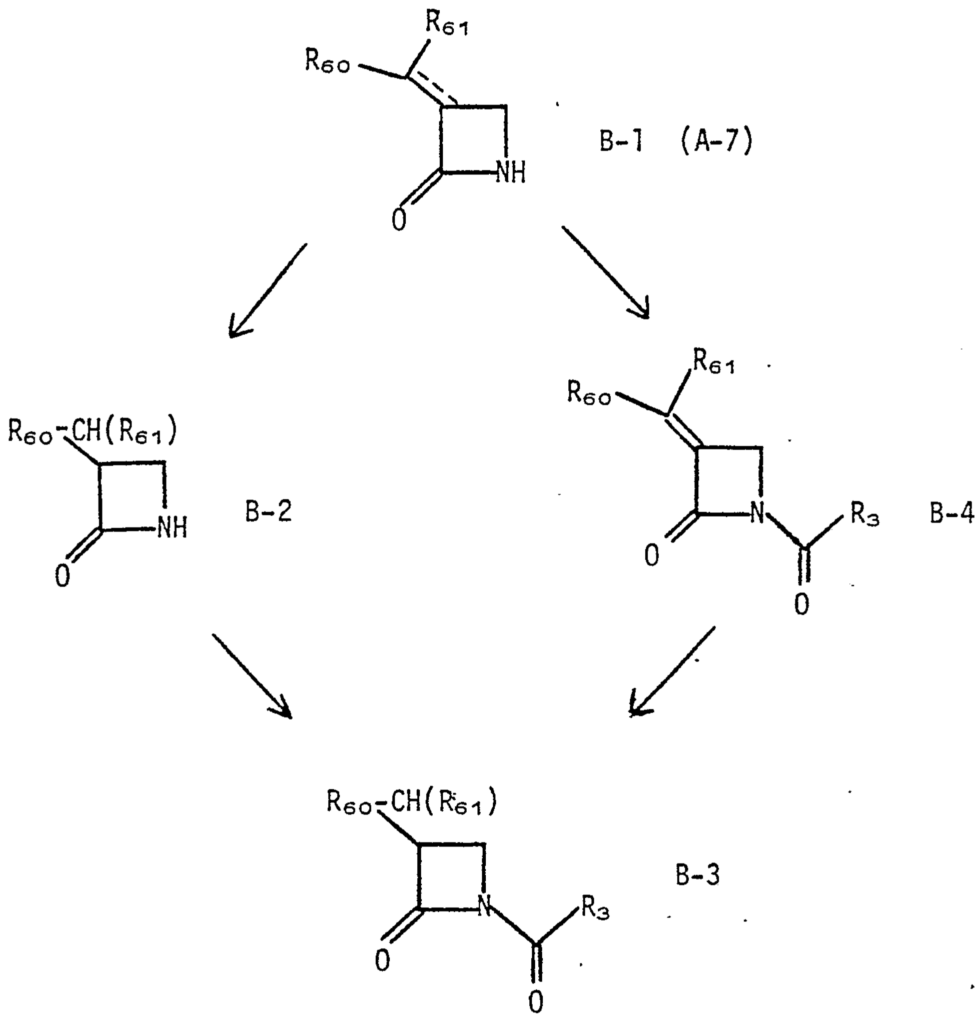

B-1 (A-7)

B-2

B-4

B-3

CHART C

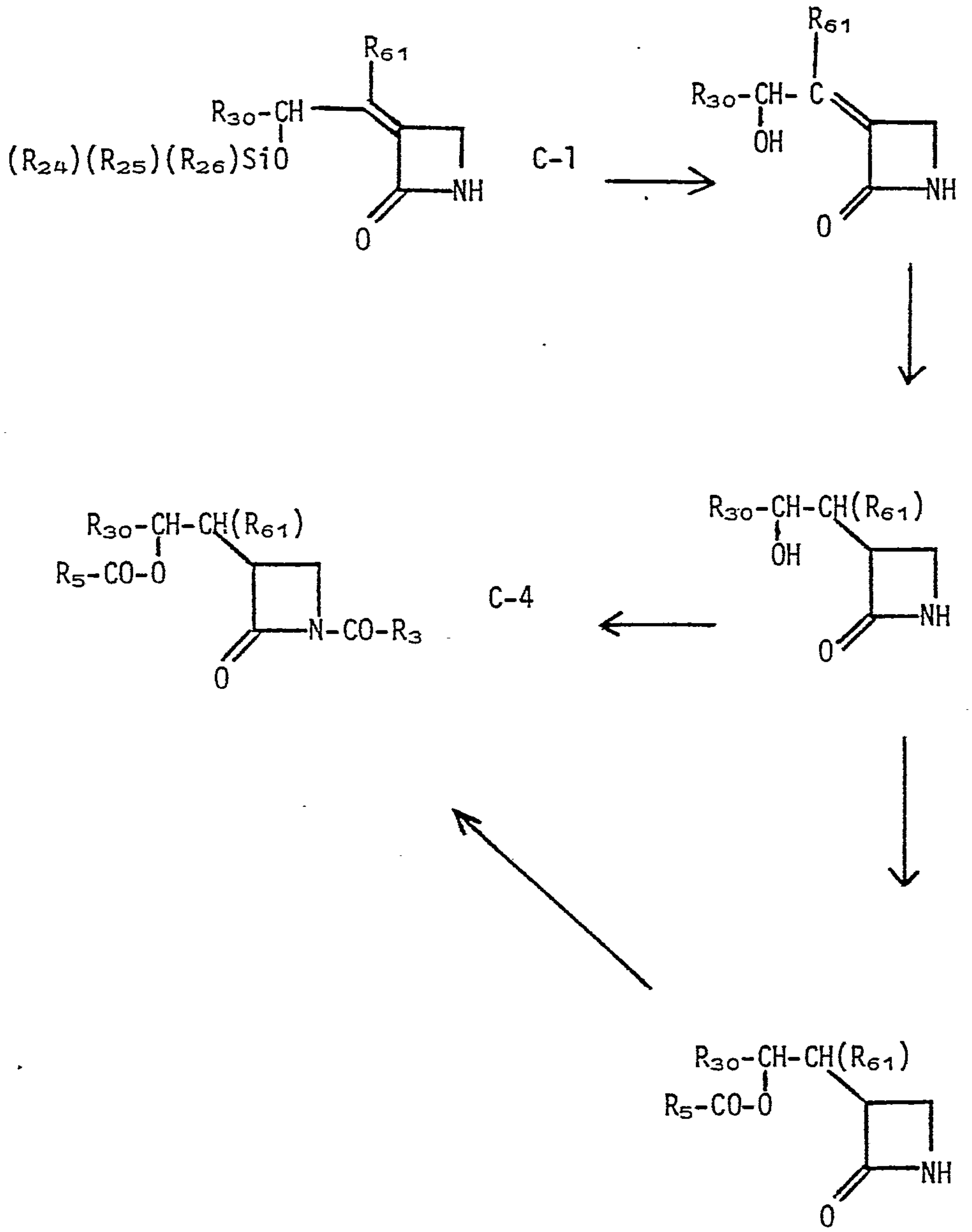

C-1

C-2

C-3

C-4

C-5

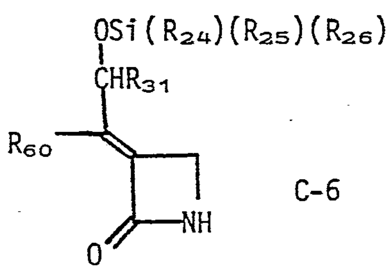

C-6

CHART D

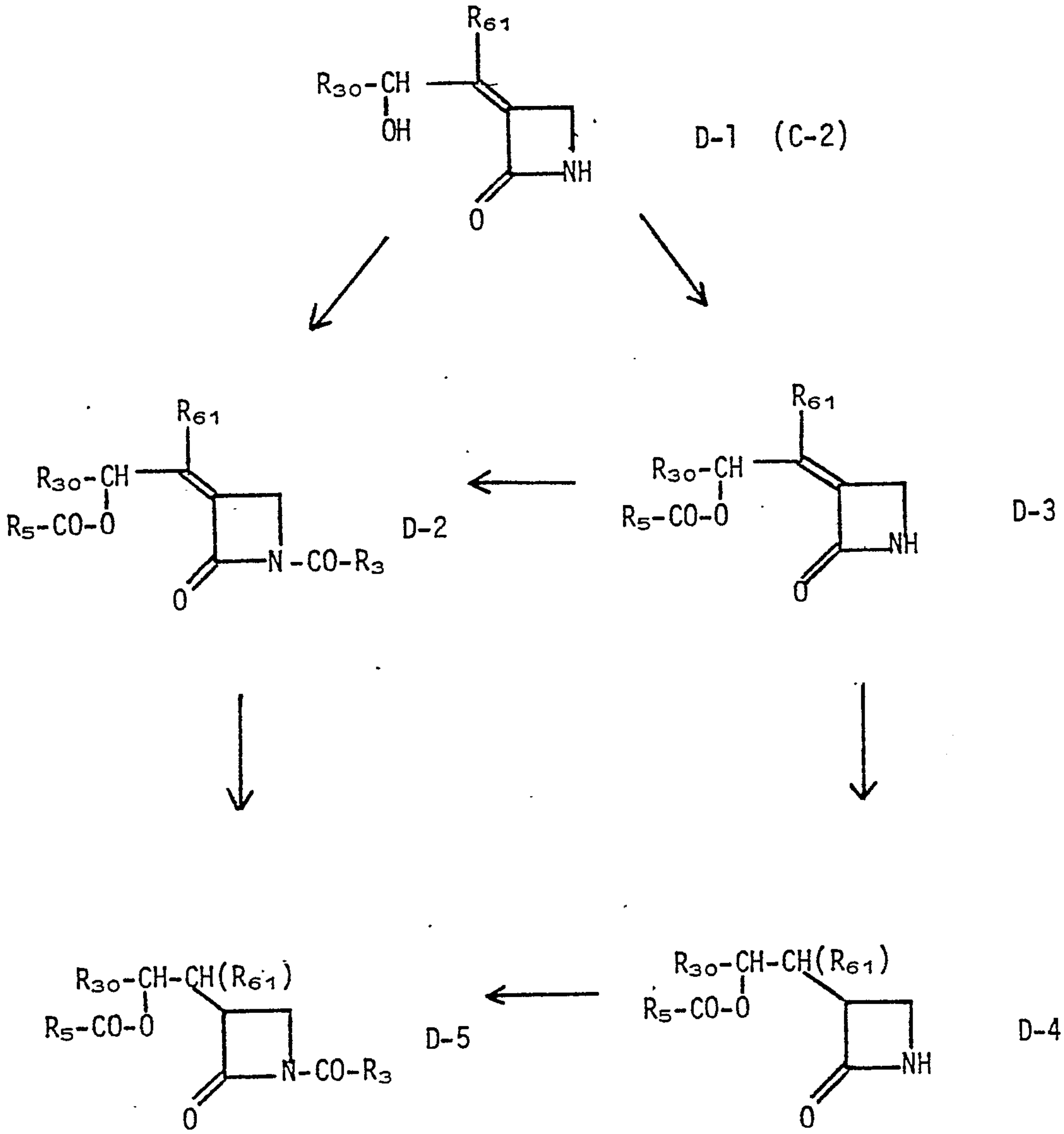

D-1 (C-2)

D-2

D-3

D-4

D-5

CHART E

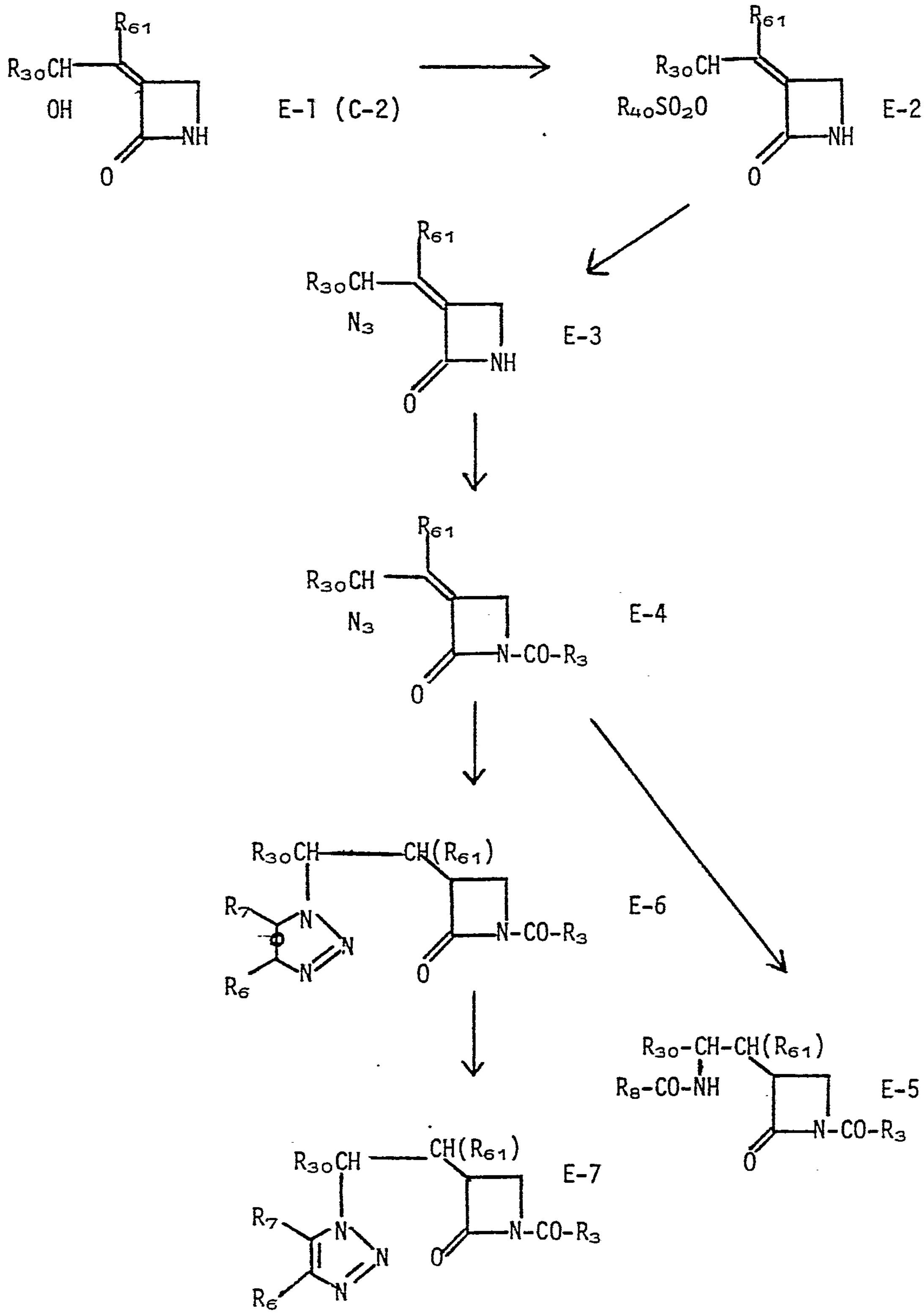

E-1 (C-2)

E-2

E-3

E-4

E-6

E-5

E-7

CHART F

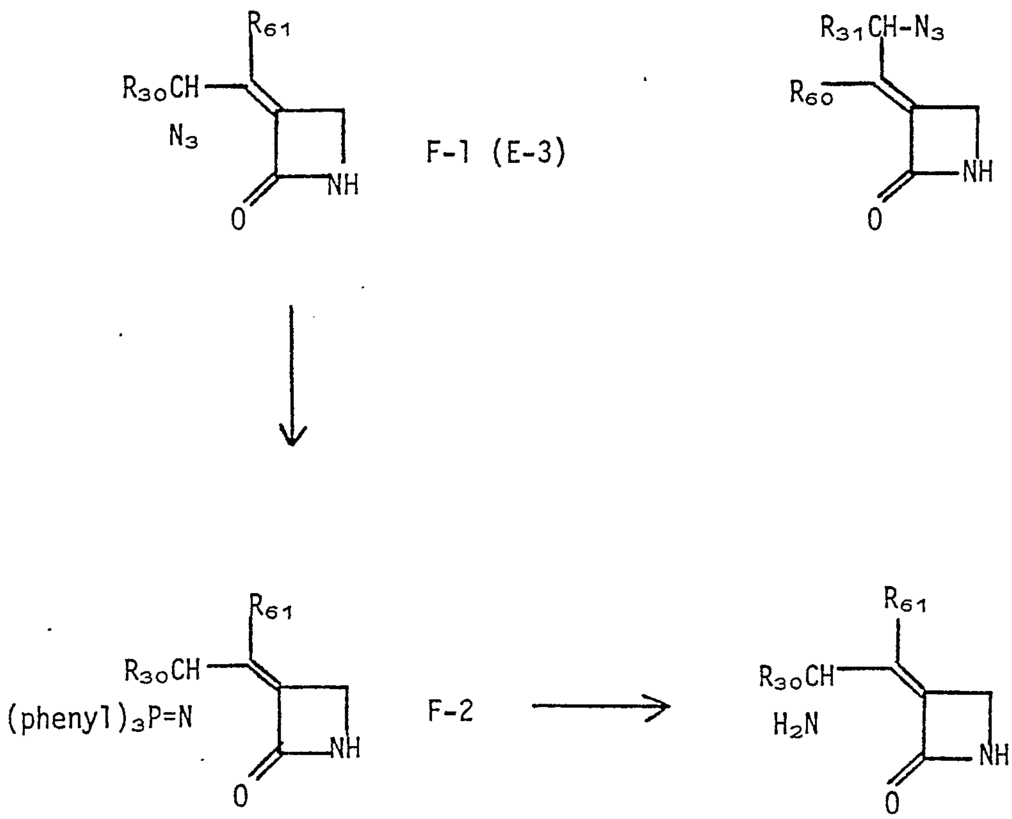

F-1 (E-3)

F-4

F-2

F-3

CHART G

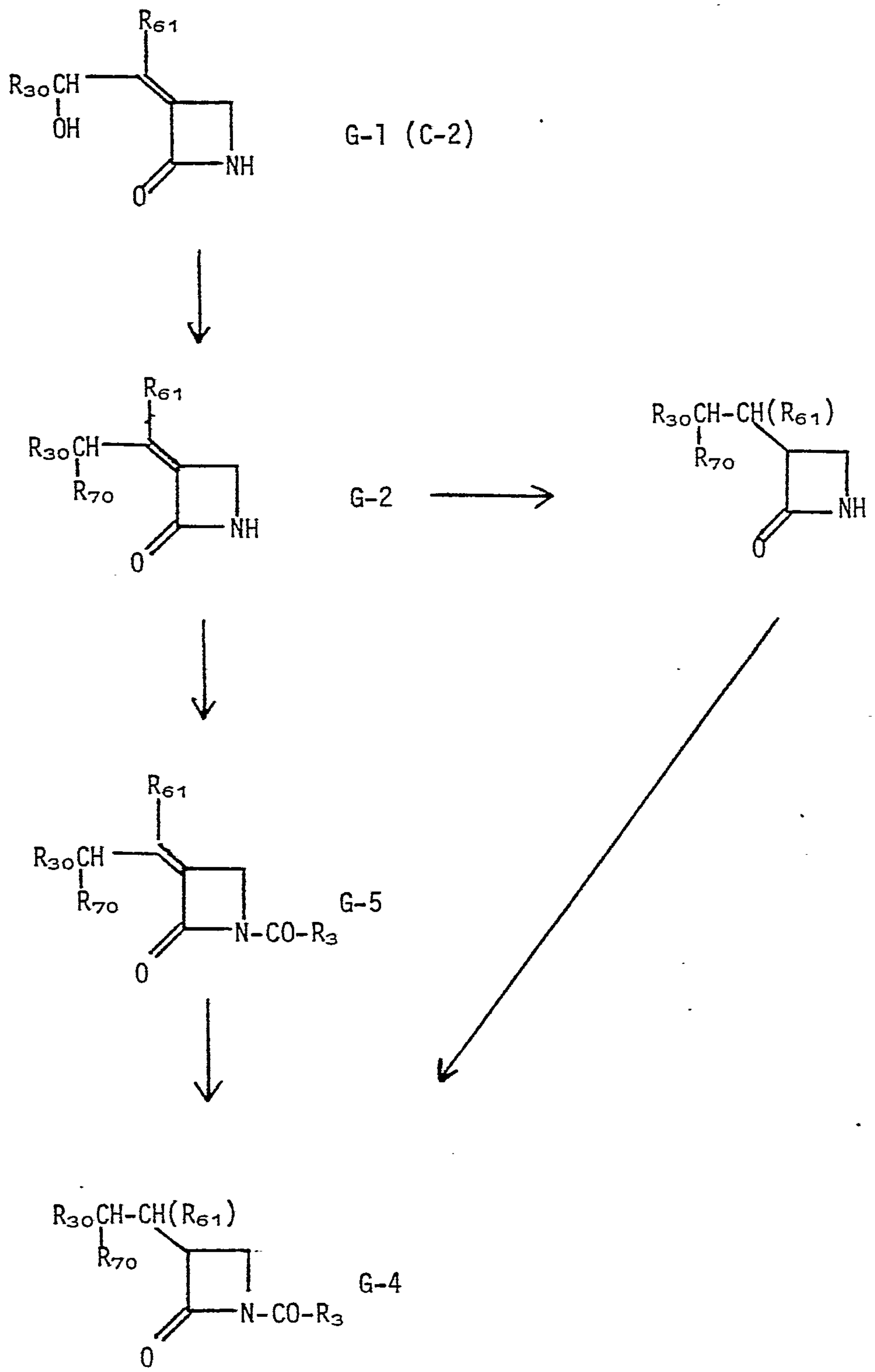

G-1 (C-2)

G-2

G-3

G-5

G-4

CHART H

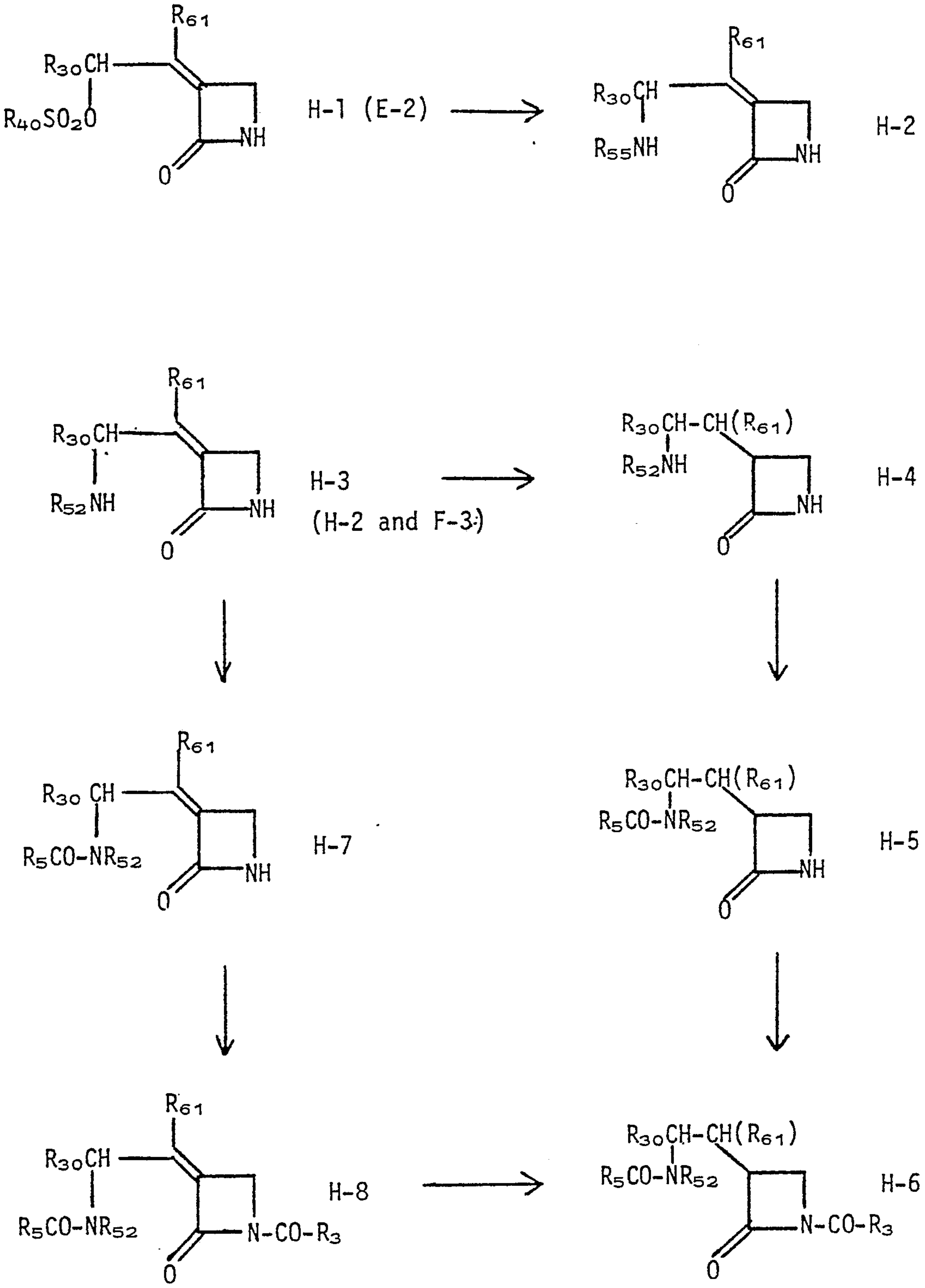

H-1 (E-2)

H-2

H-3 (H-2 and F-3)

H-4

H-7

H-5

H-8

H-6

CHART H (continued)

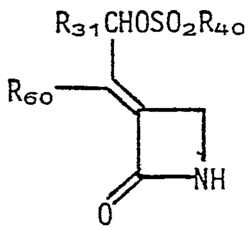

H-9

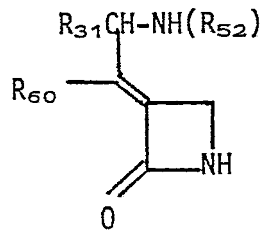

H-10

CHART I

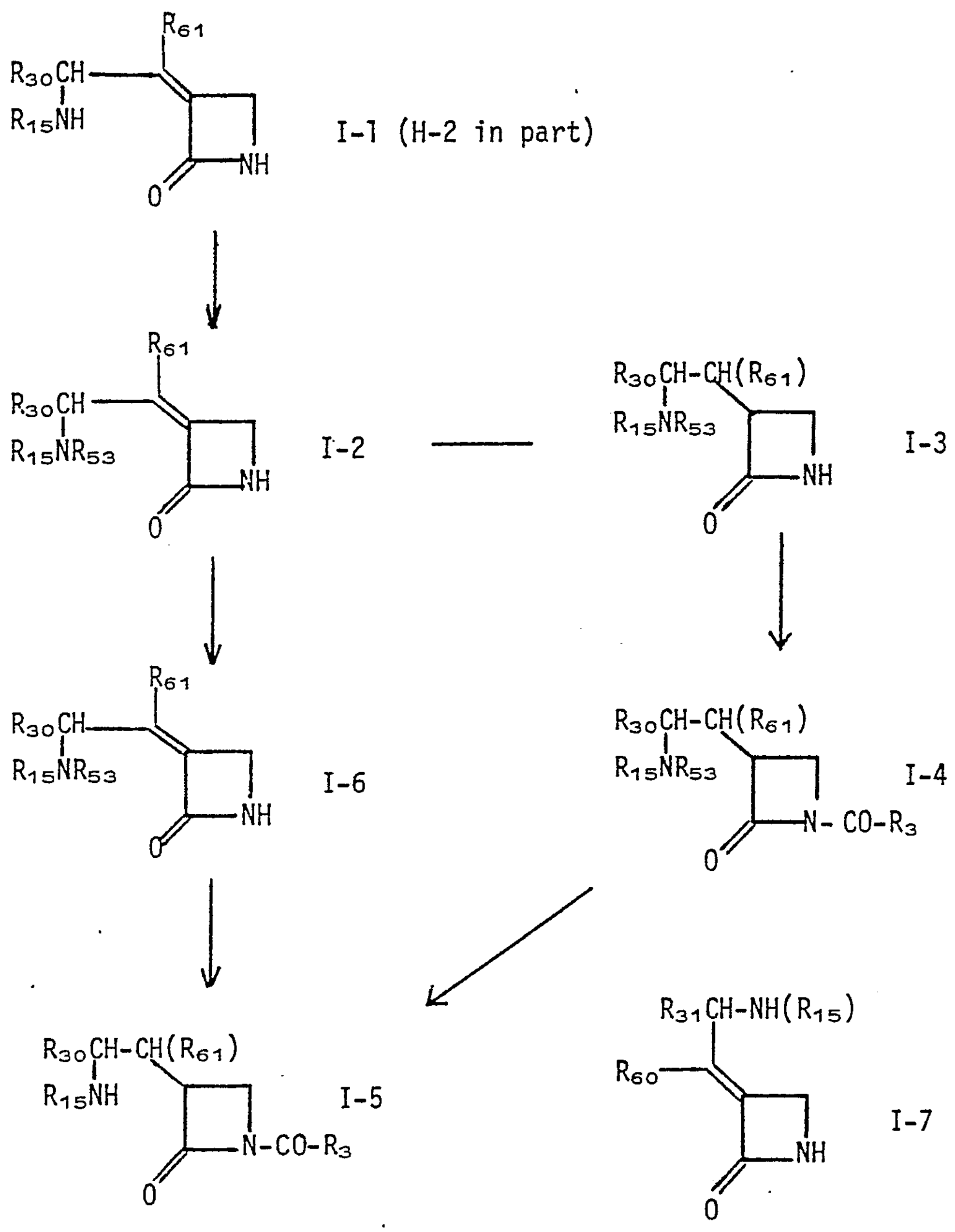

I-1 (H-2 in part)

I-2

I-3

I-6

I-4

I-5

I-7

CHART J

Scheme I

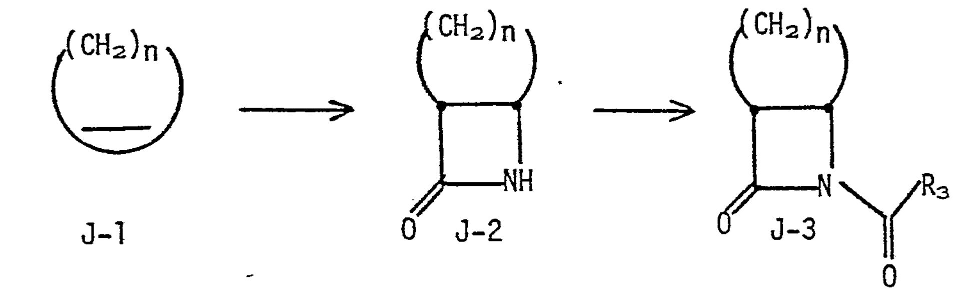

J-1 J-2 J-3

Scheme II

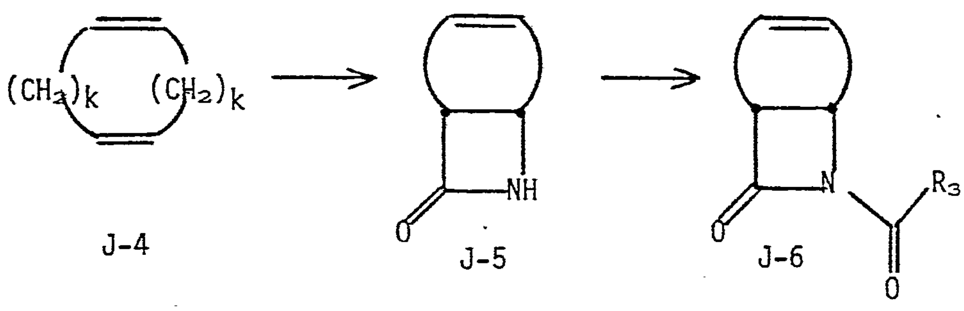

J-4 J-5 J-6

CHART K

Scheme I

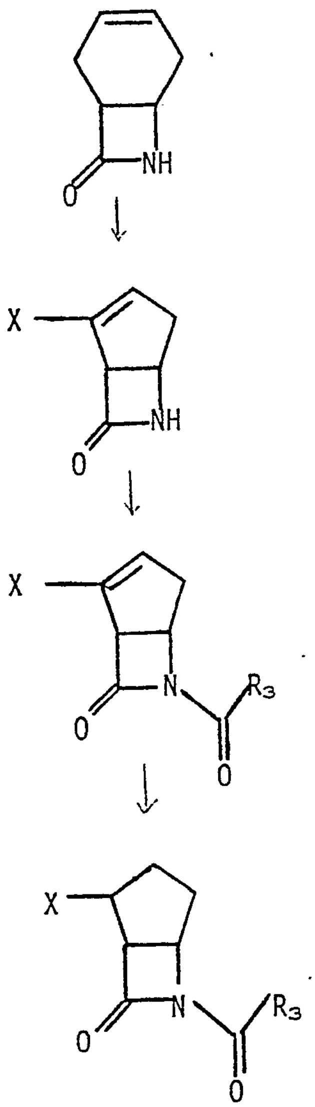

K-1

K-2

K-3

K-4

CHART K (continued)

Scheme II

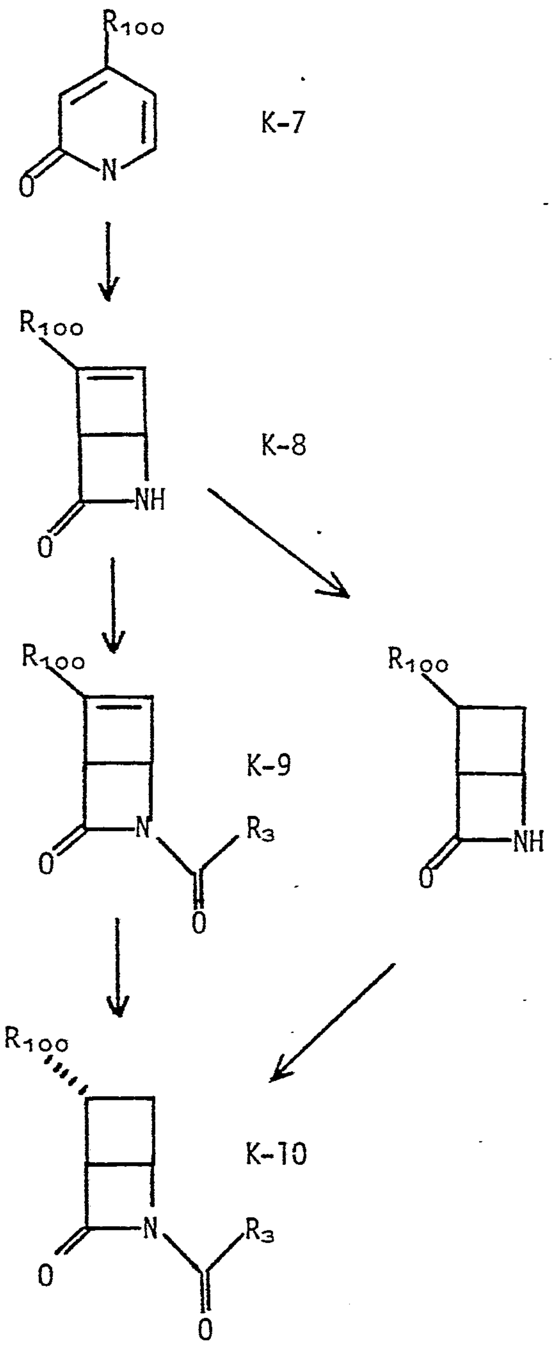

K-7

K-8

K-9

K-11

K-10

CHART L

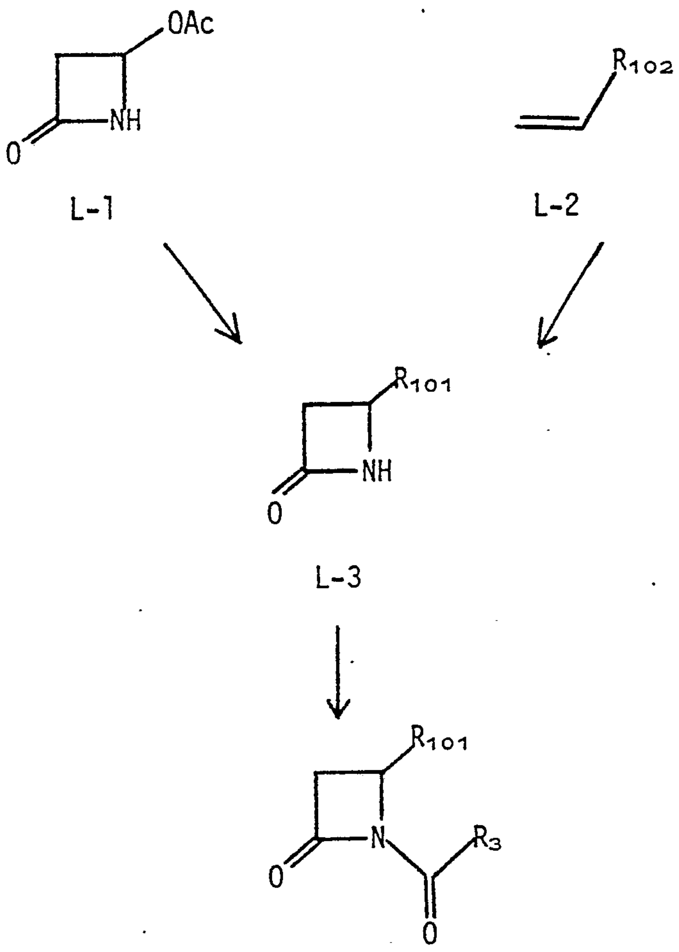

L-1

L-2

L-3

L-4

CHART M

Scheme I

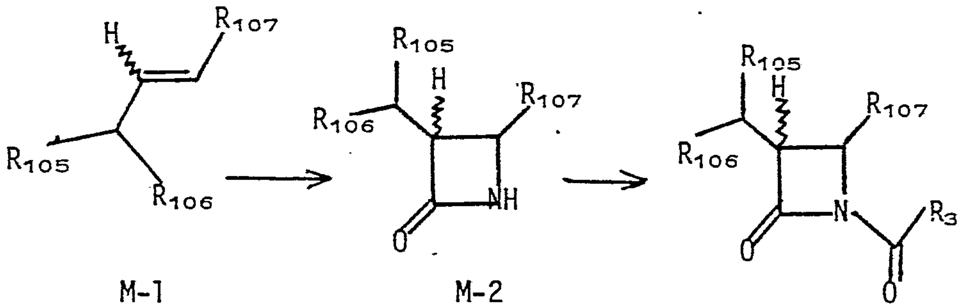

M-1 M-2 M-3

Scheme II

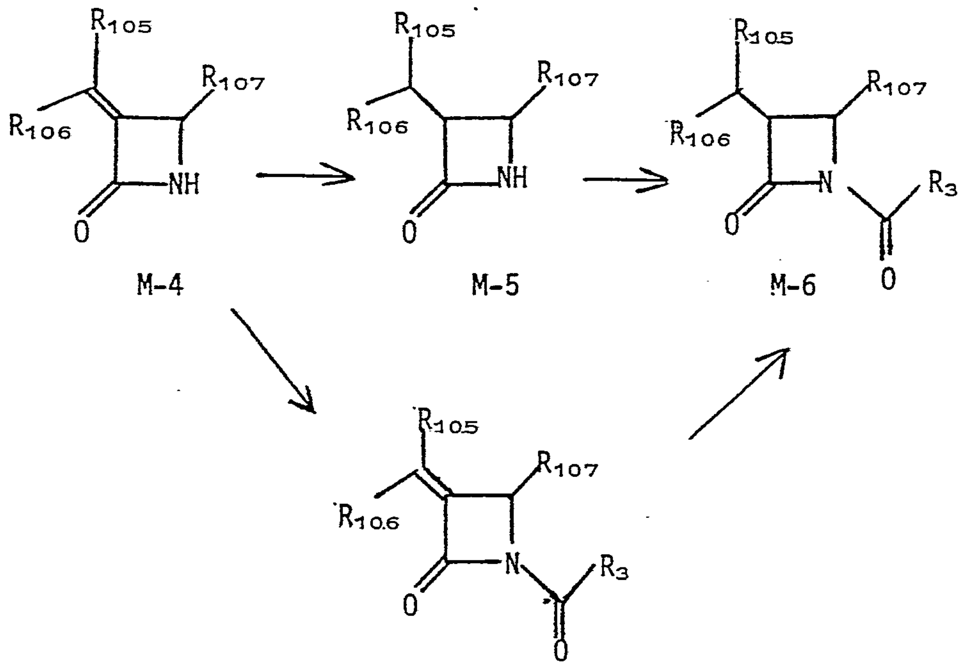

M-4 M-5 M-6

M-7

## Claims

1. A compound of the formula I

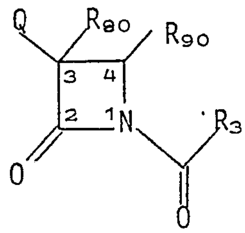

I

wherein Q is
1) hydrogen, or
2) $-C(R_0)(R_1)(R_{70})$;
wherein $R_{70}$ and $R_{80}$ are each hydrogen or wherein $R_{70}$ and $R_{80}$ taken together are a double bond;
wherein $R_{90}$ is
1) hydrogen,
2) (C1-C12)alkyl,
3) (C2-C12)alkenyl,
4) (C2-C12)alkynyl,
5) $-CH_2$-(C2-C12)alkenyl, or
6) phenyl;
or wherein $R_{80}$ and $R_{90}$ taken together, with the carbon atoms to which they are attached, are
1) (C4-C8)cycloalkyl,
2) (C4-C8)cycloalkenyl, or
3) cyclobutyl or cyclobutenyl substituted by (C1-C8) alkyl;
4) cyclopentyl or cyclopentenyl substituted by
(i) -CHO,
(ii) $-CH_2Br$,
(iii) $-CH_2OC(O)R_5$, or
(iv) $-CH_2OC(O)R_3$;
provided that when $R_{80}$ and $R_{90}$ are taken together, Q is hydrogen;
provided that $R_{80}$, $R_{90}$ and Q cannot all be hydrogen:
wherein $R_0$ and $R_1$ are the same or different and are:
1) hydrogen,
2) (C1-C12) alkyl substituted by zero, one or two $R_2$,
3) (C1-C12) alkyl substituted on the carbon atom of attachment by $R_{15}NH-$, $R_5CO-N(R_{19})-$ or $R_{15}N(CO-R_5)-$,
4) (C2-C12) alkenyl,
5) (C2-C12) alkynyl,
6) (C3-C12) cycloalkyl substituted by zero, one or two $R_2$,
7) (C3-C12) cycloalkyl substituted on the carbon atom of attachment by $R_{15}NH-$, $R_5CO-N(R_{19})-$ or $R_{15}N(CO-R_5)-$,
8) (C3-C12) cycloalkyl-(C1-C6) alkyl substituted by zero, one or two $R_2$,
9) (C3-C12) cycloalkyl-(C1-C6) alkyl substituted on the carbon atom of attachment by $R_{15}NH-$, $R_5CO-N(R_{19})-$ or $R_{15}N(CO-R_5)-$,
10) $R_{11}$,
11) $R_{11}$-(C1-C6) alkyl,
12) $R_{11}$-(C2-C6) alkenyl,
13) $R_{11}$-(C2-C6) alkynyl,
14) $R_{13}$,
15) $R_{13}$-(C1-C6) alkyl,
16) $R_{13}$-(C2-C6) alkenyl, or
17) $R_{13}$-(C2-C6) alkynyl;
or wherein $R_0$ and $R_1$ are taken together with the carbon atom to which they are bonded to form:
1) (C3-C12) cycloalkyl substituted by zero, one or two $R_{32}$, or
2) (C4-C12) cycloalkenyl;
provided that $R_0$ and/or $R_1$ contains an alkynyl group only when $R_{70}$ and $R_{80}$ taken together are a double bond; and

provided that the carbon atom of attachment of alkenyl or alkynyl to oxygen of alkenyl-0- or alkynyl-0-groups is not also part of a carbon-carbon double or triple bond;
wherein $R_2$ is:
1) $R_{16}O$-.
2) $R_{10}O$-,
3) $R_{10}(CH_2)_mS(CH_2)_n$-,
4) $R_5CO$-O-,
5) $N_3$,
6) 4,5-dihydro-4-($R_6$)-5-($R_7$)-1H-1,2,3-triazol-1-yl,
7) 4-($R_6$)-5-($R_7$)-1H-1,2,3-triazol-1-yl,
8) $R_8CO$-NH-,
9) $R_5CO$-NH-,
10) fluorine,
11) chlorine, or
12) bromine;
provided that $R_2$ is $N_3$ or 4,5-dihydro-4-($R_6$)-5-($R_7$)-1H-1,2,3-triazol-1-yl only when $R_{70}$ and $R_{80}$ taken together are a double bond; and provided that $R_2$ is hydroxy (i.e., $R_2$ is $R_{10}O$ wherein $R_{10}$ is hydrogen) only when $R_{70}$ and $R_{80}$ taken together are a double bond; and $R_2$ is a substituent on $R_1$; and
provided that $R_{32}$ is hydroxy (i.e., $R_{32}$ is $R_{10}O$ wherein $R_{10}$ is hydrogen) only when $R_{70}$ and $R_{80}$ taken together are a double bond;
wherein m is an integer zero, one, two, three or four;
wherein n is an integer zero, one, two, three or four;
wherein $R_3$ is:
1) hydrogen,
2) (C1-C12) alkyl substituted by zero, one or two $R_{22}$,
3) (C2-C12) alkenyl,
4) (C2-C12) alkynyl,
5) (C3-C12) cycloalkyl substituted by zero, one or two $R_{22}$,
6) (C3-C12) cycloalkyl-(C1-C6) alkyl substituted by zero, one or two $R_{22}$,
7) $R_{11}$,
8) $R_{11}$-(C1-C6) alkyl,
9) $R_{11}$-(C2-C6) alkenyl,
10) $R_{11}$-(C2-C6) alkynyl,
11) $R_{13}$,
12) $R_{13}$-(C1-C6) alkyl,
13) $R_{13}$-(C2-C6) alkenyl,
14) $R_{13}$-(C2-C6) alkynyl,
15) $R_{14}O$-,
16) $R_4NH$-,
17) $R_4N(CH_3)$-,
18) an α-amino acid moiety ($N^{\alpha}$-$R_{34}$)-$R_{35}$- wherein $R_{34}$ is (C1-C12) alkanoyl and $R_{35}$ is defined so that $R_{35}$-CO- is an α-amino acid acyl group selecting from the group consisting of glycyl, alanyl, valyl, leucyl, isoleucyl, phenylalanyl, O-$R_{34}$-seryl, O-$R_{34}$-threonyl, $N^{\varepsilon}$-$R_{34}$-lysyl, asparagyl, glutamyl, S-$R_{34}$-cysteinyl, methionyl, O-$R_{34}$-tyrosyl, $N^{in}$-$R_{34}$-tryptophyl, and $N^{im}$-$R_{34}$histidyl; or
19) an α-amino acid moiety $R_{36}$- wherein $R_{36}$ is defined so that $R_{36}$-CO- is prolyl or 4-$R_{34}$ O-prolyl and $R_{34}$ is as defined above; wherein $R_4$ is:
1) (C1-C4) alkyl,
2) phenyl, or
3) (C1-C4) alkyl-$NHSO_2$-;
wherein $R_5$ is:
1) hydrogen,
2) (C1-C12) alkyl substituted by zero, one or two $R_{22}$,
3) (C2-C12) alkenyl,
4) (C2-C12) alkynyl,
5) (C3-C12) cycloalkyl substituted by zero, one or two $R_{22}$,
6) (C3-C12) cycloalkyl-(C1-C6) alkyl substituted by zero, one or two $R_{22}$,
7) $R_{11}$,
8) $R_{11}$-(C1-C6) alkyl,
9) $R_{11}$-(C2-C6) alkenyl,
10) $R_{11}$-(C2-C6) alkynyl,
11) $R_{13}$,
12) $R_{13}$-(C1-C6) alkyl,
13) $R_{13}$-(C2-C6) alkenyl,
14) $R_{13}$-(C2-C6) alkynyl,
15) $R_{14}O$-,

16) $R_4NH$-,
17) $R_4N(CH_3)$-,
18) an α-amino acid moiety ($N^{\alpha}$-$R_{34}$)-$R_{35}$- wherein $R_{34}$ is (C1-C12) alkanoyl and $R_{35}$ is defined so that $R_{35}$-CO- is an α-amino acid acyl group selected from the group consisting of glycyl, alanyl, valyl, leucyl, isoleucyl, phenylalanyl, O-$R_{34}$-seryl, O-$R_{34}$-threonyl, $N^{\epsilon}$-$R_{34}$-lysyl, asparagyl, S-$R_{34}$-cysteinyl, methionyl, O-$R_{34}$-tyrosyl, $N^{in}$-$R_{34}$-tryptophyl, and $N^{im}$-$R_{34}$-histidyl; or
19) an α-amino acid moiety $R_{36}$- wherein $R_{36}$ is defined so that $R_{36}$-CO- is prolyl or 4-$R_{34}$ O-prolyl and $R_{34}$ is as defined above;
wherein $R_6$ and $R_7$ are the same or different and are:
1) hydrogen,
2) (C1-C4) alkyl,
3) (C1-C4) alkyloxy-C(=O)-,
4) $(R_{29})_2NC(=O)$-, or
5) $(R_{24})(R_{25})(R_{26})Si$-;
wherein $R_8$ is:
1) hydrogen,
2) (C1-C12) alkyl substituted by zero, one or two $R_{20}$,
3) (C3-C12) cycloalkyl substituted by zero, one or two $R_{20}$,
4) (C3-C12) cycloalkyl-(C1-C6) alkyl substituted by zero, one or two $R_{20}$,
5) $R_{11}$,
6) $R_{11}$-(C1-C6) alkyl,
7) $R_{13}$,
8) $R_{13}$-(C1-C6) alkyl, or
9) $R_{17}O$-;
wherein $R_9$ is:
1) (C1-C12) alkyl,
2) (C3-C12) cycloalkyl,
3) (C3-C12) cycloalkyl-(C1-C6) alkyl,
4) $R_{11}$, or
5) $R_{11}$-(C1-C6) alkyl;
wherein $R_{10}$ is:
1) hydrogen,
2) (C1-C12) alkyl,
3) (C2-C12) alkenyl,
4) (C3-C12) cycloalkyl,
5) (C3-C12) cycloalkyl-(C1-C6) alkyl,
6) $R_{11}$,
7) $R_{11}$-(C1-C6) alkyl,
8) $R_{11}$-(C2-C6) alkenyl,
9) $R_{13}$,
10) $R_{13}$-(C1-C6) alkyl, or
11) $R_{13}$-(C2-C6) alkenyl;
wherein $R_{11}$ is phenyl or 1- or 2-naphthyl, substituted by zero, one or two (C1-C4) alkyl, F, Cl, Br or (C1-C4) alkyloxy;
wherein $R_{13}$ is:
1) furanyl, substituted by zero, one or two $R_9$,
2) thienyl, substituted by zero, one or two $R_9$,
3) N-((C1-C4) alkyl)-pyrrolyl, substituted by zero, one or two $R_9$,
4) N-((C1-C4) alkyl)-indolyl, substituted by zero, one or two $R_9$, or
5) benzofuranyl, substituted by zero, one or two $R_9$;
wherein $R_{14}$ is:
1) (C1-C12) alkyl,
2) (C2-C12) alkenyl,
3) (C2-C12) alkynyl,
4) (C3-C12) cycloakyl,
5) (C3-C12) cycloalkyl-(C1-C6) alkyl,
6) $R_{11}$,
7) $R_{11}$-(C1-C6) alkyl,
8) $R_{11}$-(C2-C6) alkenyl,
9) $R_{11}$-(C2-C6) alkynyl,
10) $R_{13}$,
11) $R_{13}$-(C1-C6) alkyl,
12) $R_{13}$-(C2-C6) alkenyl, or
13) $R_{13}$-(C2-C6) alkynyl;
wherein $R_{15}$ is phenyl, substituted by zero, one, two or three F, Cl, Br, (C1-C3) alkyloxy or by zero, one or

two $NO_2$ or $NH_2$:
provided that phenyl is substituted by $NO_2$ only when $R_{70}$ and $R_{80}$ taken together are a double bond, and that phenyl is substituted by $NH_2$ only when $R_{70}$ and $R_{80}$ are each hydrogen;
wherein $R_{16}$ is $(R_{24})(R_{25})(R_{26})Si$, benzyl, tetrahydropyran-2-yl or other oxygen protecting group;
wherein $R_{17}$ is:
1) (C1-C12) alkyl,
2) (C3-C12) cycloalkyl,
3) (C3-C12) cycloalkyl-(C1-C6) alkyl,
4) $R_{11}$,
5) $R_{11}$-(C1-C6) alkyl,
6) $R_{13}$, or
7) $R_{13}$-(C1-C6) alkyl;
wherein $R_{18}$ is:
1) (C1-C12) alkyl,
2) (C2-C12) alkenyl,
3) (C3-C12) cycloalkyl,
4) (C3-C12) cycloalkyl-(C1-C6) alkyl,
5) $R_{11}$,
6) $R_{11}$-(C1-C6) alkyl,
7) $R_{11}$-(C2-C6) alkenyl,
8) $R_{13}$,
9) $R_{13}$-(C1-C6) alkyl, or
10) $R_{13}$-(C2-C6) alkenyl;
wherein $R_{19}$ is hydrogen or (C1-C12) alkyl;
wherein $R_{20}$ is:
1) (C1-C12) alkyl,
2) (C3-C12) cycloalkyl,
3) (C3-C12) cycloalkyl-(C1-C6) alkyl,
4) phenyl,
5) phenyl-(C1-C6) alkyl,
6) naphthyl, or
7) naphthyl-(C1-C6) alkyl;
wherein $R_{22}$ is:
1) $R_{16}O$-,
2) $R_{10}O$-,
3) $R_{10}(CH_2)_mS(CH_2)_n$-,
4) F,
5) Cl,
6) Br,
7) I,
8) $R_{23}NH$-,
9) NC-,
10) $NO_2$,
11) NHCHO,
12) (C1-C4) alkyloxy-C(=O)-,
13) phenyl-$CH_2$0C(=O)-,
14) (C1-C4) alkyl-C(=O)-$OCH_2$-,
15) (C1-C4) alkyloxy-N=,
16) CN-,
17) SCN-,
18) (C1-C4) alkyl-C(=O)-,
19) (C1-C4) alkyl-$SO_2$-,
20) phenyl-$SO_2$-,
21) ((C1-C4) alkyl)-phenyl-$SO_2$-,
22) $(R_{33})_3N(+)$ X(-), or
23) $R_{27}$, $R_{28}$-substituted-1-pyridinium(+) X(-);
wherein X(-) is a pharmacologically acceptable anion;
wherein $R_{27}$ and $R_{28}$ are the same or different and are:
1) hydrogen, or
2) (C1-C4) alkyl;
or wherein $R_{27}$ and $R_{28}$ are bonded to adjacent carbon atoms of the pyridine ring in which they occur and taken together are (C3-C5) methylene to form a (C5-C7)-membered carbocyclic ring fused to the pyridine ring;
wherein $R_{23}$ is:
1) hydrogen,

2) t-butyloxycarbonyl,
3) trityl,
4) benzyloxycarbonyl, or
5) benzyl;
wherein $R_{24}$, $R_{25}$ and $R_{26}$ are the same or different and are:
1) (C1-C4) alkyl, or
2) phenyl;
wherein $R_{29}$ is (C1-C4) alkyl;
wherein $R_{32}$ is:
1) $R_{16}O$-,
2) $R_{10}O$-,
3) $R_{10}(CH_2)_mS(CH_2)_n$-,
4) (C1-C4) alkyl, or
5) phenyl substituted by zero, one or two $R_{16}O$-, $R_{10}O$-, $R_{10}$-$(CH_2)_mS(CH_2)_n$-, (C1-C4) alkyl, F, Cl, or Br;
wherein $R_{33}$ is:
1) (C1-C4) alkyl, or
2) benzyl.

2. A compound of claim 1, wherein Q is -$C(R_0)(R_1)(R_{70})$ aand $R_{90}$ is hydrogen and formula I is therefore

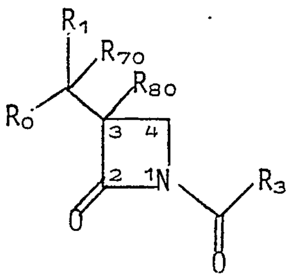

Ia

3. A compound according to claim 2 wherein $R_0$ and $R_1$ are the same or different and are (C1-C12) alkyl substituted by zero, one or two $R_2$, and $R_3$ is (C1-C12) alkyl substituted by zero, one or two $R_{22}$.

4. A compound according to claim 3 wherein $R_0$ and $R_1$ are both methyl and $R_3$ is (C1-C7) alkyl substituted by zero, one or two $R_{22}$.

5. A compound according to claim 4 wherein $R_{22}$ is NC- or Br.

6. A compound according to claim 4 wherein $R_{70}$ and $R_{80}$ are each hydrogen.

7. A compound according to claim 6 wherein $R_3$ is methyl, n-propyl, n-butyl-n-pentyl, or n-heptyl.

8. A compound according to claim 7 wherein $R_3$ is n-pentyl.

9. A compound according to claim 4 wherein $R_{70}$ and $R_{80}$ taken together are a double bond.

10. A compound according to claim 9 wherein $R_3$ is methyl, n-propyl, n-butyl, n-pentyl, or n-heptyl.

11. A compound according to claim 3 wherein $R_{70}$ and $R_{80}$ are each hydrogen; and $R_0$ and $R_1$ are both methyl substituted by zero, one or two $R_2$, and $R_3$ is (C1-C7) alkyl.

12. A compound according to claim 11 wherein $R_2$ is $R_5CO$-O- or F.

13. A compound according to claim 12 wherein $R_2$ is $R_5CO$-O-, $R_5$ is n-propyl, n-pentyl or n-hexyl and $R_3$ is n-propyl, isopropyl, n-pentyl, n-hexyl or n-heptyl.

14 A compound according to claim 12 wherei $R_2$ is F and $R_3$ is n-pentyl.

15. A compound according to claim 2 wherein $R_0$ and $R_1$ are the same or different and are (C1-C12) alkyl substituted by zero, one or two $R_2$, and $R_3$ is -$OR_{14}$.

16 A compound according to claim 15 wherein $R_0$ and $R_1$ are both methyl and $R_{14}$ is n-butyl.

17. A compound according to claim 15 wherein $R_0$ and $R_1$ are both methyl substituted by zero, one or two $R_2$. $R_2$ is $R_5CO$-O-, $R_5$ is n-propyl and $R_{14}$ is n-butyl.

18. A compound according to claim 1 selected from the group consisting of:
1) 2-Azetidinone, 1-acetyl-3-(1-methylethyl)-, (±)-;
2) 2-Azetidinone, 3-(1-methylethyl)-1-(1-oxobutyl)-;
3) 2-Azetidinone, 3-(1-methylethyl)-1-(1-oxopentyl)-, (±);
4) 2-Azetidinone, 3-(1-methylethyl)-1-(1-oxohexyl)-;
5) 2-Azetidinone, 3-(1-methylethyl)-1-(1-oxooctyl)-, (±);
6) 2-Azetidinone, 1-acetyl-3-(1-methylethylidene)-;
7) 2-Azetidinone, 3-(1-methylethylidene)-1-(1-oxobutyl)-;
8) 2-Azetidinone, 3-(1-methylethylidene)-1-(1-oxopentyl)-;,
9) 2-Azetidinone, 3-(1-methylethylidene)-1-(1-oxohexyl)-;
10) 2-Azetidinone -3-(1-methylethylidene)-1-(1-oxooctyl)-;
11) 2-Azetidinone, 3-[1-methyl-2-(1-oxobutoxy)ethyl]-1-(1-oxobutyl)-, (R*,R*)-(±)0;
12) Butanoic acid, 2[1-(2-methyl-1-oxopropyl)-2-oxo-3-azetidinyl]propyl ester, (R*,R*)-(±)-;

13) 2-Azetidinone, 3-[2-(1-oxobutoxy)ethyl]-1-(1-oxohexyl)-, (R,R)-(±)-;
14) Butanoic acid, 2-[2-oxo-1-(1-oxooctyl)-3-azetidinyl] propyl ester, (R*,R*)-(±)-;
15) 2-Azetidinone, 3-[1-methyl-2-[(1-oxohexyl)oxy]ethyl]-1-(1-oxohexyl)- [3(S*)]-;
16) Heptanoic acid, 2-[2-oxo]-(1-oxoheptyl)-3-azetidinyl]-propyl ester, (R*,R*)-(±)-;
17) [3S*(S*)]-(±)-(1-Methyl-2-fluoroethyl)-1-(1-oxohexyl)-2-azetidinone;
18) 1-Azetidinecarboxylic acid, 3-(1-methylethylidene)-2-oxo-, butyl ester;
19) 1-Azetidinecarboxylic acid, 3-(1-methylethyl)-2-oxo-, butyl ester; and
20) 1-Azetidinecarboxylic acid, 3-[1-methyl-2-(1-oxobutoxy)-oxobutoxy)ethyl]-2-oxo, butyl ester, (R*,R*)-(±)-.

19. A compound of claim 1, wherein Q is hydrogen and formula I is therefore

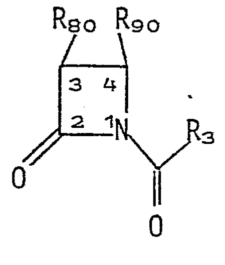

Ib

20. A compound of claim 19, wherein $R_{80}$ and $R_{90}$ taken together are (C4-C8)cycloalkyl or (C4-C8)cycloalkenyl and $R_3$ is (C1-C6)alkyl.

21. A compound of claim 20 selected from the group consisting of:
1) N-Acetyl-cis-3,4-trimethylene-2-azetidinone;
2) N-Hexanoyl-cis-3,4-trimethylene-2-azetidinone; and
3) N-Hexanoyl-cis-3,4-tetramethylene-2-azetidinone.

22. A compound of claim 1, wherein Q is hydrogen and $R_{80}$ is hydrogen and formula I is therefore

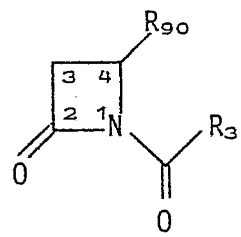

Ic

23. A compound of claim 22, wherein $R_{90}$ is (C1-C6)alkyl and $R_3$ is (C1-C6)alkyl.

24. N-Hexanoyl-4-tert-butyl-2-azetidinone, a compound of claim 23.

25. A compound of claim 1, wherein Q is -C($R_0$)($R_1$)($R_{70}$) and formula I is therefore

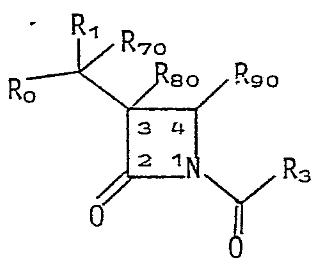

Id

26. A compound of claim 25, wherein $R_1$ and $R_0$ are each hydrogen or (C1-C8)alkyl, $R_3$ is (C1-C6)alkyl, and $R_{90}$ is (C1-C8)alkyl.

27. A compound of the formula II

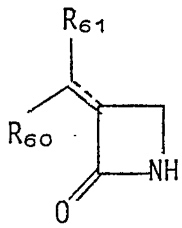

II

wherein $R_6$ and $R_{61}$ are the same or different and are:
1) hydrogen.
2) (C1-C12) alkyl substituted by zero, one or two $R_{12}$,
3) (C2-C12) alkenyl,
4) (C2-C12) alkynyl,
5) (C3-C12) cycloalkyl substituted by zero, one or two $R_{12}$,
6) (C3-C12) cycloalkyl-(C1-C6) alkyl substituted by zero, one or two $R_{12}$,
7) $R_{11}$,
8) $R_{11}$-(C1-C6) alkyl,
9) $R_{11}$-(C2-C6) alkenyl,
10) $R_{11}$-(C2-C6) alkynyl,
11) $R_{13}$,
12) $R_{13}$-(C1-C6) alkyl,
13) $R_{13}$-(C2-C6) alkenyl, or
14) $R_{13}$-(C2-C6) alkynyl;
or wherein $R_{60}$ and $R_{61}$ are taken together with the carbon atom to which they are bonded to form:
1) (C3-C12) cycloalkyl substituted by zero, one or two $R_{12}$, or
2) (C4-C12) cycloalkenyl;
provided that $R_{60}$ or $R_{61}$ contains an alkynyl group only when ═══ is a double bond; and provided that the carbon atom of attachment of alkenyl or alkynyl to oxygen of alkenyl-O- or alkynyl-O-groups is not also part of a carbon-carbon double or triple bond:
wherein $R_{11}$ is phenyl or 1- or 2-naphthyl, substituted by zero, one or two (C1-C4) alkyl, F, Cl, Br or (C1-C4) alkyloxy:
wherein $R_{12}$ is:
1) $(R_{24})(R_{25})(R_{26})SiO-$,
2) $R_{18}O-$,
3) $R_{18}(CH_2)_mS(CH_2)_n-$, or
4) $R_{40}(CH_2)_mS(CH_2)_n-$;
wherein m is an integer zero, one, two, three or four;
wherein n is an integer zero, one, two, three or four;
wherein $R_{13}$ is:
1) furanyl, substituted by zero, one or two $R_9$,
2) thienyl, substituted by zero, one or two $R_9$,
3) N-((C1-C4)alkyl)-pyrrolyl, substituted by zero, one or two $R_9$,
4) N-((C1-C4)alkyl)-indolyl, substituted by zero, one or two $R_9$, or
5) benzofuranyl, substituted by zero, one or two $R_9$;
wherein $R_9$ is:
1) (C1-C12) alkyl,
2) (C3-C12) cycloalkyl,
3) (C3-C12) cycloalkyl-(C1-C6) alkyl,
4) $R_{11}$, or
5) $R_{11}$-(C1-C6) alkyl;
wherein $R_{18}$ is:
1) (C1-C12) alkyl,
2) (C2-C12) alkenyl,
3) (C3-C12) cycloalkyl,
4) (C3-C12) cycloalkyl-(C1-C6) alkyl,
5) $R_{11}$,
6) $R_{11}$-(C1-C6) alkyl,
7) $R_{11}$-(C2-C6) alkenyl,
8) $R_{13}$,
9) $R_{13}$-(C1-C6) alkyl, or
10) $R_{13}$-(C2-C6) alkenyl;
wherein $R_{24}$, $R_{25}$ and $R_{26}$ are the same or different and are:

1) (C1-C4) alkyl, or
2) phenyl;
wherein $R_{40}$ is:
1) a sulfur-protecting group, or
2) hydrogen;
provided that $R_{40}$ is a sulfur-protecting group when m is zero and that $R_{40}$ is hydrogen when m is other than zero.

28. A compound according to claim 18 wherein $R_{60}$ and $R_{61}$ are the same or different and are (C1-C12) alkyl.

29. A process for making a compound of the formula II

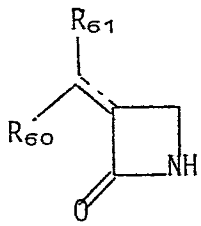

II

wherein $R_{60}$ and $R_{61}$ are the same or different and are:
1) hydrogen,
2) (C1-C12) alkyl substituted by zero, one or two $R_{12}$,
3) (C2-C12) alkenyl,
4) (C2-C12) alkynyl,
5) (C3-C12) cycloalkyl substituted by zero, one or two $R_{12}$,
6) (C3-C12) cycloalkyl-(C1-C6) alkyl substituted by zero, one or two $R_{12}$,
7) $R_{11}$,
8) $R_{11}$-(C1-C6) alkyl,
9) $R_{11}$-(C2-C6) alkenyl,
10) $R_{11}$-(C2-C6) alkynyl,
11) $R_{13}$,
12) $R_{13}$-(C1-C6) alkyl,
13) $R_{13}$-(C2-C6) alkenyl, or
14) $R_{13}$-(C2-C6) alkynyl;
or wherein $R_{60}$ and $R_{61}$ are taken together with the carbon atom to which they are bonded to form:
1) (C3-C12) cycloalkyl substituted by zero. one or two $R_{12}$, or
2) (C4-C12) cycloalkenyl;
provided that $R_{60}$ or $R_{61}$ contains an alkynyl group only when ⩶ is a double bond; and
provided that the carbon atom of attachment of alkenyl or alkynyl to oxygen of alkenyl-O- or alkynyl-O- groups is not also part of a carbon-carbon double or triple bond;
wherein $R_{11}$ is phenyl or 1- or 2-naphthyl, substituted by zero, one or two (C1-C4) alkyl, F, Cl, Br or (C1-C4) alkyloxy:
wherein $R_{12}$ is:
1) $(R_{24})(R_{25})(R_{26})SiO-$,
2) $R_{18}O-$,
3) $R_{18}(CH_2)_mS(CH_2)_n-$, or
4) $R_{40}(CH_2)_mS(CH_2)_n-$;
wherein m is an integer zero, one, two, three or four;
wherein n is an integer zero, one, two, three or four;
wherein $R_{13}$ is:
1) furanyl, substituted by zero, one or two $R_9$,
2) thienyl, substituted by zero, one or two $R_9$,
3) N-((C1-C4)alkyl)-pyrrolyl, substituted by zero, one or two $R_9$,
4) N-((C1-C4)alkyl)-indolyl, substituted by zero, one or two $R_9$, or
5) benzofuranyl, substituted by zero, one or two $R_9$;
wherein $R_9$ is:
1) (C1-C12) alkyl,
2) (C3-C12) cycloalkyl.
3) (C3-C12) cycloalkyl-(C1-C6) alkyl,
4) $R_{11}$, or
5) $R_{11}$-(C1-C6) alkyl:
wherein $R_{18}$ is:
1) (C1-C12) alkyl,

2) (C2-C12) alkenyl,
3) (C3-C12) cycloalkyl.
4) (C3-C12) cycloalkyl-(C1-C6) alkyl,
5) $R_{11}$,
6) $R_{11}$-(C1-C6) alkyl,
7) $R_{11}$-(C2-C6) alkenyl,
8) $R_{13}$,
9) $R_{13}$-(C1-C6) alkyl, or
10) $R_{13}$-(C2-C6) alkenyl;
wherein $R_{24}$, $R_{25}$ and $R_{26}$ are the same or different and are:
1) (C1-C4) alkyl, or
2) phenyl;
wherein $R_{40}$ is:
1) a sulfur-protecting group, or
2) hydrogen;
provided that $R_{40}$ is a sulfur-protecting group when m is zero and that $R_{40}$ is hydrogen when m is other than zero;
which comprises treating a compound of the formula III

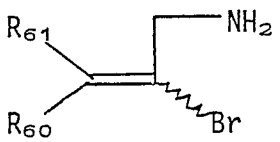

III

with a catalytic amount of palladium(O) tetrakis (triphenylphosphine) in the presence of triphenylphosphine and a base selected from the group consisting of triethylamine, diisopropylethylamine and tri-n-butylamine at 80 to 135° C.

European Patent Office

# EUROPEAN SEARCH REPORT

Application number

EP 87 30 0226

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 038 661 (BEECHAM)<br>* Claims * | 27 | C 07 D 205/08<br>C 07 D 205/10<br>C 07 D 205/12<br>C 07 D 403/06<br>C 07 D 405/06 //<br>A 61 K 31/395 |
| Y | THE JOURNAL OF ORGANIC CHEMISTRY, vol. 49, no. 6, 23rd March 1984, pages 1056-1059, American Chemical Society; T. SHONO et al: "Electroorganic chemistry, 82. beta-amino acid esters from alpha-methoxycarbamates and ketene silyl acetals; cyclization to beta-lactams"<br>* Whole article * | 27 | |
| D,Y | TETRAHEDRON, vol. 41, no. 2, 1985, pages 375-385, Pergamon Press Ltd; M. MORI et al.: "New synthesis of beta-lactams"<br>* Whole article * | 27-29 | **TECHNICAL FIELDS SEARCHED (Int. Cl.4)** |
| A | GB-A- 841 915 (LEPETIT)<br>* Claims * | 27 | C 07 D 205/00<br>C 07 D 403/00<br>C 07 D 405/00<br>A 61 K 31/00 |
| A | EP-A-0 101 598 (HOFFMANN-LA ROCHE)<br>* Claims *<br>--- -/- | 1 | |

The present search report has been drawn up for all claims

| Place of search | Date of completion of the search | Examiner |
|---|---|---|
| THE HAGUE | 27-03-1987 | CHOULY J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

European Patent Office

# EUROPEAN SEARCH REPORT

Application number

EP 87 30 0226



## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | CHEMICAL ABSTRACTS, vol. 105, no. 19, 10th November 1986, page 711, abstract no. 172114t, Columbus, Ohio, US; H.J. BERGMANN et al.:"Synthesis of acyl derivatives of 4-phenyl-2-azetidinone and their reactions with amines", & ARCH. PHARM. (WEINHEIM, GER.) 1986, 319(7), 635-41 * Abstract * | 1 | |
| | ----- | | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 27-03-1987 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03.82